(19) Europäisches Patentamt / European Patent Office / Office européen des brevets

(11) **EP 2 084 296 B1**

(12) **EUROPEAN PATENT SPECIFICATION**

(45) Date of publication and mention
of the grant of the patent:
**05.08.2015 Bulletin 2015/32**

(21) Application number: **07834611.1**

(22) Date of filing: **28.09.2007**

(51) Int Cl.:
***C12Q 1/68*** (2006.01)

(86) International application number:
**PCT/NL2007/050473**

(87) International publication number:
**WO 2008/039071 (03.04.2008 Gazette 2008/14)**

(54) **HIGH-THROUGHPUT DIAGNOSTIC TESTING USING ARRAYS**

DIAGNOSTISCHE TESTVERFAHREN MIT ARRAYS UND HOHEM DURCHSATZ

ESSAI DIAGNOSTIQUE EXTREMEMENT PRODUCTIF AU MOYEN DE JEUX ORDONNES

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **29.09.2006 US 848275 P**

(43) Date of publication of application:
**05.08.2009 Bulletin 2009/32**

(73) Proprietor: **Agendia N.V.**
**1098 XH Amsterdam (NL)**

(72) Inventors:
• **GLAS, Annuska Maria**
**1911 PX Uitgeest (NL)**
• **FLOORE, Arno Nicolaas**
**1531 EV Wormer (NL)**
• **VAN 'T VEER, Laura Johanna**
**1013 HC Amsterdam (NL)**

(74) Representative: **Hatzmann, Martin**
**V.O.**
**Johan de Wittlaan 7**
**2517 JR Den Haag (NL)**

(56) References cited:
GB-A- 2 407 090          US-A1- 2005 158 772
US-A1- 2006 134 668

• POHJANVIRTA ET AL: "Evaluation of various housekeeping genes for their applicability for normalization of mRNA expression in dioxin-treated rats" CHEMICO-BIOLOGICAL INTERACTIONS, ELSEVIER SCIENCE IRLAND, IR, vol. 160, no. 2, 25 March 2006 (2006-03-25), pages 134-149, XP005323987 ISSN: 0009-2797
• AFFYMETRIX: "Affimetrix GeneChip Human Genome U133 Array Set HG-U133A" NCBI GEO, 11 March 2002 (2002-03-11), XP002355386
• GLASS ET AL: "To find a proper baseline for visualization expression signatures" PROCEEDINGS OF THE 11TH WORLD CONGRESS ON MEDICAL INFORMATICS, [Online] 2004, page 1617, XP002473410 Amsterdam IOS Press ISBN: 1 58603 444 8 Retrieved from the Internet: URL:http://cmbi.bjmu.edu.cn/news/report/2004/medinfo2004/pdffiles/papers/119_d0400 05035.pdf> [retrieved on 2008-03-18]
• LEE PETER D ET AL: "Control genes and variability: Absence of ubiquitous reference transcripts in diverse mammalian expression studies" GENOME RESEARCH, vol. 12, no. 2, February 2002 (2002-02), pages 292-297, XP002473411 ISSN: 1088-9051
• ROGLER CHARLES E ET AL: "RNA expression microarrays (REMs), a high-throughput method to measure differences in gene expression in diverse biological samples." NUCLEIC ACIDS RESEARCH 2004, vol. 32, no. 15, 2004, page e120, XP002473412 ISSN: 1362-4962

EP 2 084 296 B1

- HUANG YIFAN ET AL: "Statistical selection of maintenance genes for normalization of gene expressions.", STATISTICAL APPLICATIONS IN GENETICS AND MOLECULAR BIOLOGY 2006 LNKD- PUBMED:16646868, vol. 5, 2006, page Article4, ISSN: 1544-6115
- "THERE IS NO SILVER BULLET - A GUIDE TO LOW-LEVEL DATA TRANSFORMS AND NORMALISATION METHODS FOR MICROARRAY DATA", BRIEFINGS IN BIOINFORMATICS, OXFORD UNIVERSITY PRESS, OXFORD, GB, vol. 6, no. 1, 1 March 2005 (2005-03-01), pages 86-97, XP008064607, ISSN: 1467-5463, DOI: DOI: 10.1093/BIB/6.1.86
- FALK OHL ET AL: "Gene expression studies in prostate cancer tissue: which reference gene should be selected for normalization?", JOURNAL OF MOLECULAR MEDICINE, SPRINGER, BERLIN, DE, vol. 83, no. 12, 1 December 2005 (2005-12-01), pages 1014-1024, XP019320313, ISSN: 1432-1440, DOI: 10.1007/S00109-005-0703-Z
- LAURA J VAN 'T VEER ET AL: "Gene Expression Profiling Predicts Clinical Outcome of Breast Cancer", NATURE: INTERNATIONAL WEEKLY JOURNAL OF SCIENCE, NATURE PUBLISHING GROUP, UNITED KINGDOM, vol. 415, no. 6871, 31 January 2002 (2002-01-31), pages 530-536, XP008138701, ISSN: 0028-0836, DOI: 10.1038/415530A
- WANG Y ET AL: "Gene-expression profiles to predict distant metastasis of lymph-node-negative primary breast cancer", THE LANCET, LANCET LIMITED. LONDON, GB, vol. 365, no. 9460, 19 February 2005 (2005-02-19), pages 671-679, XP027763936, ISSN: 0140-6736 [retrieved on 2005-02-19]
- "Array Design for the GeneChip - HUMAN GENOME U133 SET", INTERNET CITATION, 2001, pages 1-8, XP008116589, Retrieved from the Internet: URL:www.affymetrix.com [retrieved on 2001-12-18]
- LINDBERG ANDERSEN C ET AL: "Normalization of real-time quantitative reverse transcription-PCR data: A model-based variance estimation approach to identify genes suited for normalization, applied to bladder and colon cancer data sets", CANCER RESEARCH, AMERICAN ASSOCIATION FOR CANCER RESEARCH, US, vol. 64, no. 15, 1 August 2004 (2004-08-01), pages 5245-5250, XP008112436, ISSN: 0008-5472
- LI ZIRONG ET AL: "A global transcriptional regulatory role for c-Myc in Burkitt's lymphoma cells", PROCEEDINGS OF THE NATIONAL ACADEMY OF SCIENCES, NATIONAL ACADEMY OF SCIENCES, US, vol. 100, no. 14, 8 July 2003 (2003-07-08), pages 8164-8169, XP002327641, ISSN: 0027-8424, DOI: 10.1073/PNAS.1332764100

**Description**

The invention relates to a method for normalizing data obtained with an array comprising between 10 and 12.000 nucleic acid molecules comprising at least ten nucleic acid molecules that are able to hybridize to at least two of the genes listed in Table 1, and use of the array.

Microarray analysis is a widely used technology for studying gene expression on a global scale. Various studies have shown that microarray analysis results in improved diagnosis and risk stratification in many tumors [1-12]. More specifically, in human breast cancer, molecular profiles have identified subtypes [3,8], and prognostic subgroups that are relevant to patient management [4,6,13,14], and may add to the prediction of therapy response [15-18].

Normalization of microarray data often involves the use of housekeeping genes, also termed maintenance genes, such as beta-actin, GAPDH and transferrin receptor. See, for example, US 2005/158772; US 2006/134668; Falk et al., J Mol Med 83: 1014-1024; Huang et al., 2006. Statistical Applications Gen Mol Biol 5: Article 4; Lee et al., 2002. Genome Res 12: 292-297; and Pohjanvirta et al., 2006. Chemico-biological interactions 160, 134-149. These house keeping genes are often included on commercially available arrays such as, for example, the U133 gene array of Affymetrix (see Array Design for the GeneChip® - HUMAN GENOME U133 SET", pages 1-8, available on www.affymetrix.com).

One study involved the discovery of a profile associated with the risk of early development of distant metastasis in young patients with lymph-node negative breast cancer [6]. The development of distant metastases is the primary cause of death in breast cancer patients; approximately one third of women with lymph node negative breast cancer will develop distant metastasis.

The risk profile was generated using an array comprising approximately 25.000 individual nucleic acid molecules or probes. This high number allowed global normalization of the hybridization data on the assumption that the mean of intensity of hybridization signals obtained from different samples is constant.

The limited number of nucleic acid molecules on an array comprising between ten and about 12,000 nucleic acid molecules does not allow global normalization of the hybridization data.

Therefore, the invention provides a method for normalizing data of a breast cancer sample, the method comprising providing a first and a second array comprising between 10 and 12.000 nucleic acid molecules comprising a first and second set of nucleic acid molecules wherein each nucleic acid molecule of said first set comprises a nucleotide sequence that is able to hybridize to a different gene selected from the genes listed in Table 1, wherein said first and said second array comprise an identical first set of nucleic acid molecules comprising at least 10 molecules comprising a nucleotide sequence that is able to hybridize to a different gene selected from the genes listed in Table 1, dividing a sample in at least two sub-samples and labeling nucleic acid expression products in a first sub-sample with a first label, and labeling a second sub-sample with a second label different from said first label, contacting said first array with said first sub-sample and determining for each of said nucleic acid molecule of said first set the amount of said first label that is associated therewith, contacting said second array with said second sub-sample and determining for each of said nucleic acid molecule of said first set the amount of second label that is associated therewith, determining from the difference in associated first and second label for each of said first set of nucleic acid molecules a systematic error in the measurements of detected label depending on the amount of said first and said second label that is detected, and correcting at least one value for the detected amount of label associated with at least one nucleic acid on said array for the systematic error..

An array as described comprises only a limited number of genetic markers belonging to a second set of molecules. These genetic markers can be used for e.g. diagnosing stages of a disease or the presence or developmental stage of a tumor. These genetic markers are included on the array because of their differential expression between samples. However, their inclusion in normalization protocols will lead to a bias in the data generated because of their differential expression.

Therefore, the first set of molecules on an array comprises nucleotide sequences that are able to hybridize to at least ten of the genes listed in Table 1. The genes listed in Table 1 were selected because their RNA expression level was constant between different samples. The constant level of expression allows their use for normalization of hybridization data obtained with arrays comprising between 10 and 12.000 nucleic acid molecules. Such array furthermore reduces the sample RNA input for labeling and hybridization, and reduces the data processing time. Therefore, this array can be used for clinical practice allowing high throughput processing of many samples on a routine basis.

The first set comprises preferably at least 10 molecules, more preferred about 50 molecules, more preferably about 100 molecules, more preferred about 200 molecules, more preferably a minimum of 300 molecules, more preferred about 400 molecules, more preferably about 500 molecules, more preferred about 600 molecules, more preferably about 700 molecules, more preferred about 800 molecules, more preferably about 900 molecules, or more preferably about 915 molecules.

Therefore, a preferred array comprises between 10 and 12.000 nucleic acid molecules, wherein the first set of molecules comprises at least ten, more preferred at least twenty, more preferred at least fifty nucleic acid molecules that are able to hybridize to different genes listed in Table 1.

The first set of molecules of a further preferred array comprises at least 465 nucleic acid molecules that are able to hybridize to different genes listed in Table 1.

The first set of molecules of yet a further preferred array comprises at least 915 nucleic acid molecules that are able to hybridize to different genes listed in Table 1.

It is further preferred that genes used for normalization are rank-ordered according to the differences observed in level of expression for these genes in different samples. These differences are reflected by the statistical dispersion or spread of the values representing the determined expression level. A factor quantifying the statistical dispersion of the determined levels of expression, such as, for example, the standard deviation, can be used to rank-order the normalization genes. Therefore, the genes numbered 1-465 as listed in Table 1 were rank-ordered whereby the gene with the lowest standard deviation was put at position 1 (rank-ordered positions are provided in column entitled "STD order" of Table 1).

A further preferred array comprises at least ten nucleic acid molecules that are able to hybridize to genes listed in Table 1 which have the lowest standard deviation and are rank-ordered 1-10, more preferred at least fifty nucleic acid molecules that are able to hybridize to genes listed in Table 1 which have the lowest standard deviation and are rank-ordered 1-50; most preferred at least 465 nucleic acid molecules that are able to hybridize to genes listed in Table 1 which have the lowest standard deviation and are rank-ordered 1-465.

The first set of molecules may be present in multiple copies on an array of the invention. The molecules preferably comprise nucleotide sequences that are able to hybridize to at least ten of the genes listed in Table 1, are present in duplicate, in triplicate, in quadruplicate, in quintuplicate, or in sextuplicate on an array.

The genes listed in Table 1 were identified as being non-differentially expressed. The hybridization data obtained with the at least 10 genes from Table 1 can therefore be used for normalization using, for example, the non-linear (LOWESS) method of curve fitting to correct for dye bias. Normalization methods correct for systemic bias such as dye bias by transforming the log10 ratios of the intensities of signals obtained with differentially labeled expression products of a gene in a sample to approximately zero.

The genes listed in Table 1 comprise genes of which the translation products are involved in metabolism and signal transduction, and comprise genes encoding proteins involved in primary and cellular metabolism and control of metabolism, G-protein-couples receptors, and protein-binding and DNA-binding proteins.

The first set of molecules on the array preferably comprises nucleotide sequences that are able to hybridize to expression products of genes listed in Table 1 which are expressed at different levels in the cells or tissues that are being studies.

The genes listed in Table 1 were identified because their expression level was constant when comparing different samples. However, the expression level of the individual genes differs from extremely low to very high. Thus, some of the genes listed in Table 1 are expressed at very low levels in the tissues examined (indicated as intensity group 1), some genes are expressed at low levels (indicated as intensity group 2), some genes are expressed at moderate levels (indicated as intensity group 3), some genes are expressed at high levels in the tissues examined (intensity group 4), while some genes are expressed at very high levels (indicated as intensity group 5).

The first set of molecules on the array preferably comprises nucleotide sequences that are able to hybridize to expression products of the genes listed in Table 1 that differ in expression level from very low to very high in the cells or tissues that are being studied.

Therefore, the first set of molecules of a preferred array comprises nucleic acid molecules selected from at least two different intensity groups as depicted in Table 1.

The first set of molecules on the array more preferably comprises nucleotide sequences that are able to hybridize to expression products of genes listed in Table 1 that cover a range of expression levels between very low and very high, in the cells or tissues that are being studied.

The first set of molecules of a most preferred array comprises at least ten nucleic acid sequences that hybridize to a total of 10 genes that encompass all five intensity groups as depicted in Table 1.

Preferably, said first set of molecules on the array comprises sequences that are able to hybridize to at least 15, more preferably 20, more preferably 25, more preferably 30, more preferably 35, more preferably 40, more preferably 45, more preferably 50, more preferably 60, more preferably 100, more preferably 200, more preferably 300, more preferably at least 400 of the gene products of genes listed in Table 1 encompassing all five intensity groups as depicted in Table 1.

Said first set of molecules on the array preferably comprises nucleotide sequences that are able to hybridize to at least 465 of the gene products of genes selected from each of the five intensity groups as depicted in Table 1, more preferably comprises nucleotide sequences that are able to hybridize to the first 465 genes listed in Table 1, which are numbered 1-465.

It is further preferred that a gene is selected from each of the five intensity groups with the lowest standard deviation as a measure for the spread of values representing the determined expression level of said gene in relevant samples. Therefore, a preferred array comprises nucleic acid molecules that are able to hybridize to genes listed in Table 1 which have the lowest standard deviation in at least two of the five indicated intensity groups.

More preferred is an array wherein the first set of molecules comprises nucleic acid molecules that are able to hybridize to genes selected from all five intensity groups as depicted in Table 1 having the lowest standard deviation in each of said five intensity groups. The number of rank-ordered genes according to the standard deviation is preferably at least one for each intensity group, more preferred at least two for each intensity group, more preferred at least three for each intensity group, more preferred at least four for each intensity group, more preferred at least five for each intensity group, most preferred at least ten for each intensity group.

The number of genes selected differs for each of the intensity groups, resulting, for example, in two genes selected for a first intensity group, and ten genes selected for a second intensity group.

Said array can be used to determine the expression level of a reporter gene in a cell, a collection of cells, or a tissue sample.

For this, an array preferably further comprises a second set of nucleotide sequences capable of hybridizing to nucleic acid molecules that are differentially expressed in human cells and/or in human tissues, such as, for example, genes that are differentially expressed during development, genes that are differentially expressed during treatment with a drug or a hormone, genes that are differentially expressed during development of a disease, or genes that are differentially expressed during tumor development.

In this aspect, an array further comprises a second set of nucleic acid molecules. The second set comprises nucleotide sequences that are able to hybridize to nucleic acid molecules that are expressed in a human cell or tissue.

The second set of molecules preferably comprises nucleotide sequences that are capable of hybridizing to nucleic acid molecules that that are expressed in samples of breast tissue.

The second set of molecules comprises nucleotide sequences that allow diagnosing the presence and/or staging of a tumor. The tumor can be of any origin. Preferred tumors are lymphoma such as Hodgkin's lymphoma or non-Hodgkin's lymphoma, sarcoma such as osteosarcoma or chondrosarcoma, leukemia such as acute myelogenous leukemia, chronic myelogenous leukemia, acute lymphocytic leukemia, or chronic lymphocytic leukemia, myeloma such as plasmacytoma or multiple myeloma, or carcinoma such as adenocarcinoma, colon carcinoma, lung carcinoma, squamous cell carcinoma or breast, carcinoma.

A second set of molecules preferably comprises nucleotide sequences that allow diagnosing the presence and/or staging of a breast carcinoma.

Recently, a method for diagnosing and staging of breast tumor cells has been developed. Expression profiles of a number of genetic markers from tumor samples are compared to profiles of reference breast tumor samples. Differences and similarities in these profiles are then used to evaluate the presence and/or stage of disease [WO 02/103320]. In this study, a total of 231 genetic markers were identified, of which the expression can be correlated with the presence and/or staging of a breast tumor. This number was further reduced by the "leave-one-out" method to a total of 70 genetic markers [6]. A gene expression profile generated by these 70 genetic markers can be used to determine the presence and/or stage of a breast tumor cell in a collection of cells or tissue sample.

Therefore, the second set of nucleotide sequences on an array may comprise at least two sequences that are able to hybridize to any of the 231 genetic markers for breast tumor cells that are listed in Table 2.

In another embodiment, the second set of nucleotide sequences on an array may comprise at least 3 sequences, more preferably at least 4 sequences, more preferably at least 5 sequences, more preferably 10, more preferably 15, more preferably 20, more preferably 25, more preferably 30, more preferably 35, more preferably 40, more preferably 45, more preferably 50, more preferably 55, more preferably 60, more preferably at least 65 sequences that are able to hybridize to any of the 231 genetic markers for breast tumor cells that are listed in Table 2.

Therefore, the second set of nucleic acid molecules preferably comprises at least five nucleotide sequences capable of hybridizing to nucleic acid molecules that are expressed in breast samples and that are selected from Table 2.

The second set of nucleic acid molecules more preferably comprises 70 nucleotide sequences that are able to hybridize to the genetic markers for breast tumor cells numbered 1 through 70 as listed in Table 2.

The second set of nucleotide sequences on the array comprises at least two sequences, preferably at least 3, more preferably at least 4, more preferably at least 5, more preferably at least 10, more preferably at least 15, more preferably at least 20, more preferably at least 25, more preferably at least 30, more preferably at least 35, more preferably at least 40, more preferably at least 45, more preferably at least 50, more preferably at least 60, more preferably at least 100, more preferably at least 200 nucleotide sequences capable of hybridizing to the genetic markers for breast tumor cells that are listed in Table 3.

Therefore, the second set of nucleic acid molecules comprises at least five nucleotide sequences capable of hybridizing to nucleic acid molecules selected from Table 3.

The second set of molecules may be present in multiple copies on an array.

In a preferred embodiment, the second set of molecules is present in duplicate, in triplicate, in quadruplicate, in quintuplicate, or in sextuplicate on an array.

In a further preferred embodiment, the second set of molecules is present in triplicate on an array.

A preferred array comprises a total of 1900 molecules.

A further preferred array comprises a total of about 4000 molecules.

A further preferred array comprises a total of about 8000 molecules.

An array as described can be printed in multiple identical regions on a slide. In this way, multiple samples can be processed at the same time.

Therefore, a preferred array as described is printed in multiple identical regions on a slide.

Described is an array that is printed in two or more identical regions, an array that is printed in three or more identical regions, an array that is printed in four or more identical regions, an array that is printed in five or more identical regions, an array that is printed in six or more identical regions, an array that is printed in seven or more identical regions, an array that is printed in eight or more identical regions, an array that is printed in ten or more identical regions, or an array that is printed in sixteen or more identical regions on a slide. A preferred array is printed in eight identical regions on a slide.

Further described is a method for producing an array comprising a first set of nucleic acid molecules that are able to hybridize to at least two of the genes listed in Table 1, the method comprising immobilization of the molecules on a support.

Methods for producing an array comprise general methods that are or will be known by a person skilled in the art and include, but are not limited to, immobilization of the molecules to a solid support. This support can be porous or non-porous such as a nitrocellulose, silica, acrylamide, or nylon membrane or filter.

The nucleic acid molecules preferably comprise DNA sequences, RNA sequences, or copolymer sequences of DNA and RNA. The molecules may also comprise DNA and/or RNA analogues such as, for example nucleotide analogues or peptide nucleic acid molecules (PNA), or combinations thereof. The molecules may comprise full or partial fragments of genomic DNA. The molecules may also comprise synthesized nucleotide sequences, such as synthetic oligonucleotide sequences. The sequences can be synthesized enzymatically in vivo, or enzymatically in vitro (e.g. by PCR), or non-enzymatically in vitro.

The length of the sequences may be between 20 and 500 nucleotides, such as, for example, 25 or more nucleotides, 30 or more nucleotides, 35 or more nucleotides, 40 or more nucleotides, 45 or more nucleotides, 50 or more nucleotides, 55 or more nucleotides, 60 or more nucleotides, 65 or more nucleotides, 70 or more nucleotides, 75 or more nucleotides, or 80 or more nucleotides, or 100 or more nucleotides

The length of the sequences preferably is about 60 nucleotides.

The molecules on the array may be carefully designed to minimize nonspecific hybridization. The nucleotide sequence can be complementary to a region on the known or predicted mRNA that is known or will be known by a skilled person to be transcribed from the genes listed in Table 1. Therefore, this nucleotide sequence is able to hybridize to a complementary nucleic acid molecule that is derived from this mRNA by methods that are or will be known to a skilled person.

The nucleotide sequence preferably is complementary to a region on the mRNA that is within 1 kilobases from the poly(A) sequence on the mRNA.

An array may comprise a support or surface with an ordered array of molecules. Preferably the arrays are addressable arrays, and more preferably positionally addressable arrays. More specifically, each molecule on the array is preferably located at a known, predetermined position on the solid support such that the identity (i.e., the sequence) of each molecule can be determined from its position on the array. In preferred embodiments, each molecule is covalently attached to the solid support at a single site.

It will be clear for a skilled person that contacting means the hybridization of the labeled sample to the nucleic acid molecules on the array, followed by washing to remove unbound labeled sample. Preferred conditions for hybridization and washing are stringent conditions, as known to a skilled person.

Said first set of nucleic acid molecules preferably comprises at least 10 nucleic acid molecules that are able to hybridize to genes listed in Table 1, whereby it is further preferred that said nucleic acid molecules are selected according to their rank-ordering position based on the determined standard deviation (see Table 1).

In a preferred embodiment, the invention relates to said method for normalizing data, wherein said nucleic acid for which said value is corrected is a member of said second or a third set of nucleic acid molecules.

In a further preferred embodiment, a method according to the invention further comprises determining a transformation function for transforming the log10 ratios of the intensities of the detected hybridization signals to zero or approximately zero for expression products of genes listed in Table 1; using the determined transformation function to transform the intensity of the log10 ratios of hybridization signals obtained from nucleic acid molecules belonging to said second set of molecules.

Methods known in the art comprise quantifying the fluorescence intensities on scanned images. The values can be corrected for background non-specific hybridization, and can be normalized using, for example, Feature Extraction software (Agilent Technologies). Normalization methods include methods that are or will be known to a person of ordinary skill in the art, such as locally weighted polynomial regression [23, 24].

For the locally weighted polynomial regression, a low-degree polynomial is fit to a subset of the data at each point in the data set, with explanatory variable values near the point whose response is being estimated. The polynomial is fit using weighted least squares, giving more weight to points near the point whose response is being estimated and less weight to points further away. The value of the regression function for the point is then obtained by evaluating the local polynomial using the explanatory variable values for that data point. The locally weighted polynomial regression fit is complete after regression function values have been computed for each of the n data points.

Samples can be processed in numerous ways, as is known to a skilled person. For example, they can be freshly prepared from cells or tissues at the moment of harvesting, or they can be prepared from surgical biopsies that are stored at -70°C until processed for sample preparation. Alternatively, tissues or surgical biopsies can be stored under protective conditions that preserve the quality of the RNA. Examples of these preservative conditions are fixation using e.g. formaline, RNase inhibitors such as RNAsin (Pharmingen) or RNasecure (Ambion). Alternatively, specific salts such as a salt comprising ammonium sulfate or cesium sulfate can be added to the sample as an RNA-preserving agent.

The biopsies preferably have a depth of at most 10 millimeter, more preferred at most 5 millimeter, and a diameter of about 2 millimeter, about 3 millimeter, about 4 millimeter, about 5 millimeter, about 6 millimeter, about 7 millimeter, about 8 millimeter, about 9 millimeter, or about 10 millimeter. However, other forms that are equal in size are also possible.

The sample may also be prepared from a needle aspiration biopsy, which is a procedure by which a thin needle is inserted in a tissue to extract cells. A needle aspiration biopsy can be processed and stored under protective conditions that preserve the quality of the RNA. Examples of these preservative conditions are fixation using e.g. formaline, RNase inhibitors such as RNAsin (Pharmingen) or RNasecure (Ambion). Alternatively, a solution of specific salts such as a salt comprising ammonium sulfate or cesium sulfate can be added to the sample as an RNA-preserving agent.

Further described is a sample container comprising a RNA-protecting agent and a human biopsy, the sample container being stored in a plastic envelope, the envelope further comprising written sampling instructions and a punch.

The sample container preferably comprises a solution of a salt comprising ammonium sulfate or cesium sulfate and a biopsy of a human tumor.

The sample container preferably comprises a solution of a salt comprising ammonium sulfate or cesium sulfate and a biopsy of human breast tissue.

For the generation of a hybridization mixture, the sample RNA is preferably extracted and labeled. The RNA can be extracted from the sample by any method known in the art. RNA can be isolated from the whole sample or from a portion of the sample generated by, for example section or laser dissection. The extracted sample RNA is preferably subsequently converted into a labeled sample comprising either complementary DNA (cDNA) or cRNA using a reverse-transcriptase enzyme and labeled nucleotides. A preferred labeling introduces fluorescently-labeled nucleotides such as, but not limited to, Cyanine-3-CTP or cyanine-5-CTP. Examples of labeling methods are known in the art and include Low RNA Input Fluorescent Labeling Kit (Agilent Technologies), MessageAmp Kit (Ambion) and Microarray Labeling Kit (Stratagene).

The labeled sample preferably comprises two dyes that are used in a so-called two-color array. For this, the sample is split in two or more parts, and one of the parts is labeled with a first fluorescent dye, while a second part is labeled with a second fluorescent dye. The labeled first part and the labeled second part are independently hybridized to an array as described. The duplicate hybridizations with the same samples allow compensating for dye bias.

The labeled sample can be hybridized against the molecules that are spotted on the array. A molecule in the labeled sample will bind to its appropriate complementary target sequence on the array. Before hybridization, the arrays are preferably incubated at high temperature with solutions of saline-sodium buffer (SSC), Sodium Dodecyl Sulfate (SDS) and bovine serum albumin (BSA) to reduce background due to nonspecific binding.

The arrays are preferably washed after hybridization to remove labeled cDNA that did not hybridize on the array, and to increase stringency of the experiment to reduce cross hybridization of the sample sequences to partial complementary probe sequence on the array. An increased stringency will substantially reduce a-specific hybridization of the sample, while specific hybridization of the sample is not substantially reduced. Stringent conditions include, for example, washing steps for five minutes at room temperature 0.1x Sodium chloride-Sodium Citrate buffer (SSC)/0.005% Triton X-102. More stringent conditions include washing steps at elevated temperatures, such as 37 degrees Celsius, 45 degrees Celsius, or 65 degrees Celsius, either or not combined with a reduction in ionic strength of the buffer to 0,05x SSC or 0,01x SSC as is known to a skilled person.

Image acquisition and data analysis can subsequently be performed to produce an image of the surface of the hybridised array. For this, the slide can be dried and placed into a laser scanner to determine the amount of labeled sample that is bound to each target spot. Laser excitation of the incorporated targets yields an emission with characteristic spectra.

The interpretation of the data produced can be performed using known clustering analyses based on, for example, hierarchical or partitional clustering.

Data obtained with a labeled sample can be compared to data obtained with reference samples. Reference samples can include a collection of cell lines, representing different tissues, tissue samples with and without a tumor, or samples comprising different stages of a tumor. The expression profile of an unknown sample can be used to compare with the expression profile of a reference sample or multiple reference samples so as to evaluate the presence and/or stage of a disease.

In one aspect, a method is provided for detecting and/or staging of a disease comprising providing an array comprising between 10 and 12.000 nucleic acid molecules comprising a first set of at least 10 nucleic acid molecules wherein each nucleic acid molecule of said first set comprises a nucleotide sequence that is able to hybridize to a different gene selected from the genes listed in Table 1; contacting said array with a sample comprising expression products from cells of a patient; detecting hybridization of said expression products to nucleic acid molecules of said array to provide an expression profile; comparing said hybridization with the hybridization of at least one reference sample to a similar array.

In a further aspect, a method is described for classifying the presence and/or stage of a disease, comprising providing an array comprising between 10 and 12.000 nucleic acid molecules comprising at least 10 molecules of a first set of nucleic acid molecules wherein each nucleic acid molecule of said first set comprises a nucleotide sequence that is able to hybridize to a different gene selected from the genes listed in Table 1; contacting said array with a sample comprising expression products from cells of a patient; detecting hybridization of said expression products to nucleic acid molecules of said array to provide an expression profile; comparing said expression profile with the expression profile of at least one reference sample to a similar array; and classifying the presence and/or stage of a disease on the basis of the comparison of the expression profile.

In yet a further aspect, a method is described for prognosticating the risk of distant metastasis of breast cancer, comprising providing an array comprising between 10 and 12.000 nucleic acid molecules comprising at least 10 molecules of a first set of nucleic acid molecules wherein each nucleic acid molecule of said first set comprises a nucleotide sequence that is able to hybridize to a different gene selected from the genes listed in Table 1; contacting said array with a sample comprising expression products from cells of a patient; detecting hybridization of said expression products to nucleic acid molecules of said array; comparing said hybridization with the hybridization of at least one reference sample to a similar array; classifying said patient as having a first prognosis or a second prognosis on the basis of the comparison with the hybridization of the at least one reference sample.

The result of a comparison of the determined expression profile with the expression profile of at least one reference sample is preferably displayed or outputted to a user interface device, a computer readable storage medium, or a local or remote computer system. The storage medium may include, but is not limited to, a floppy disk, an optical disk, a compact disk read-only memory (CD-ROM), a compact disk rewritable (CD-RW), a memory stick, and a magneto-optical disk.

The result of said comparison preferably is a score indicating a similarity of the determined expression profile in relevant cells of a patient, and the expression profile of at least one reference sample, whereby said reference sample is a sample form a patient with known disease, or with known outcome of a disease. It is further preferred that said score varies between +1, indicating a prefect similarity, and -1, indicating a reverse similarity.

The invention further relates to the use of an array comprising between 10 and 12.000 nucleic acid molecules comprising a first set of nucleic acid molecules wherein each-nucleic acid molecule of said first set comprises a nucleotide sequence that is able to hybridize to a different gene selected from the genes listed in Table 1, wherein the first set of molecules comprises at least ten nucleic acid molecules that are able to hybridize to different genes listed in Table 1, in a method of the invention..

Said at least ten nucleic acid molecules preferably are able to hybridize to genes listed in Table 1 which have the lowest standard deviation and are rank-ordered 1-10.

The first set of molecules preferably comprises nucleic acid molecules selected from at least two different intensity groups as depicted in Table 1, more preferably from all five intensity groups as depicted in Table 1.

An array that is used according to the invention preferably further comprises a second set of nucleic acid molecules comprising nucleic acid molecules capable of hybridizing to nucleic acid molecules that are expressed in samples of breast tissue. The second set of nucleic acid molecules comprises at least five nucleotide sequences capable of hybridizing to nucleic acid molecules that are expressed in breast samples and that are selected from Table 2. Alternatively, the second set of nucleic acid molecules comprises at least five nucleotide sequences capable of hybridizing to nucleic acid molecules selected from Table 3.

The invention further provides the use of an array comprising between 10 and 12.000 nucleic acid molecules comprising a first and second set of nucleic acid molecules wherein said first set of nucleic acid molecules comprises at least 10 molecules, each nucleic acid molecule of said first set comprising a nucleotide sequence that is able to hybridize to a different gene selected from the genes listed in Table 1, said array further comprising a second set of nucleic acid molecules comprising nucleic acid molecules capable of hybridizing to nucleic acid molecules that are expressed in samples of breast tissue in a method for detecting and/or staging of breast cancer, the method comprising

contacting said array with a sample comprising expression products from breast cancer cells of a patient, detecting hybridization of said expression products to nucleic acid molecules of said array to provide an expression profile, normalizing hybridization data obtained with at least one nucleic acid molecule of the second set of nucleic acid molecules with hybridization data obtained with the first set of nucleic acid molecules; and comparing said hybridization with the hybridization of at least one reference sample to a similar array.

The second set of nucleic acid molecules on an array that is used according to the invention comprises at least five nucleotide sequences capable of hybridizing to nucleic acid molecules that are expressed in breast samples and that are selected from Table 2.

Said reference sample preferably is a sample from a patient with known breast cancer, or with known outcome of breast cancer.

Definitions

Array

An array is a collection of molecules comprising nucleotide sequences that are attached to a solid surface, such as glass, plastic or silicon. The molecules are termed probes, and can hybridize to complementary nucleic acid molecules. The complementary molecules can be e.g. fluorescently labeled so that their hybridization to a probe on the array can be monitored with a fluorescent detection device.

Set

A collection of at least two molecules that is present on an array.

Slide

A solid surface, such as glass, plastic or silicon, onto which the molecules comprising nucleotide sequences are attached.

Normalization

The term normalization refers to a method for adjusting or correcting a systematic error in the measurements of detected label. A preferred method is provided by dye-swapping, in which probes are labeled with a first and a second label. Differences in the amount of a first and a second label that is detected are used to correct the raw data.

The correction for systemic bias such as dye bias is preferably performed by transforming the log10 ratios of the intensities of signals obtained with differentially labeled expression products of a normalization gene in a sample to approximately zero by applying an appropriate transformation function. Said transformation function is subsequently used for normalization of intensities of signals obtained from further genes.

Reference sample

A reference sample can include a collection of cell lines, representing different tissues, tissue samples with and without a tumor. A reference sample can be used to compare stages of a tumor, or, for example, to compare tumor samples from patients with different prognoses. A preferred reference sample is a sample form a patient with known disease, or with known outcome of a disease.

Expression profile

An expression profile is a reflection of the expression levels of multiple genes in a sample. It can be obtained by analyzing the hybridization pattern of a sample on an array, or by analyzing the expression levels using, for example, Northern blotting or quantitative polymerase chain reaction.

Legends to the Figures

Figure 1. Expression data matrix of 70 prognostic markers genes from tumors of 78 breast cancer patients hybridized using the custom microarray.

Figure 2. Comparison of current data to published values

Figure 3. Custom array outcome of replicate experiments.

Figure 4. Custom diagnostic microarray outcome of two samples over time.

Figure 5A. Kaplan-Meier analysis of the probability that patients would remain free of distant metastases

Figure 5B. Kaplan-Meier Analysis of the probability of overall survival

Figure 6. The number of normalization genes used plotted against the difference in MPI as determined using up to 100 independent sets of randomly selected normalization genes compared to the MPI as determined using 465 normalization genes. The two straight lines indicate the 95% confidence interval for determined MPI using all normalization genes.

Figure 7. The number of normalization genes used plotted against the difference in MPI as determined using up to 100 independent sets of rank-ordered normalization genes according to the standard deviation of the logratio, compared to the MPI as determined using 465 normalization genes. The two straight lines indicate the 95% confidence interval for determined MPI using all normalization genes.

Figure 8. The number of normalization genes used plotted against the difference in MPI as determined using up to 100 independent sets of randomly selected normalization genes from each of the five intensity intervals, compared to the MPI as determined using 465 normalization genes. The two straight lines indicate the 95% confidence interval for determined MPI using all normalization genes.

Figure 9. The number of normalization genes used plotted against the difference in MPI as determined using up to 100 independent sets of normalization genes from each of the five intensity intervals that were rank-ordered according to the standard deviation of the logratio, compared to the MPI as determined using 465 normalization genes. The two straight lines indicate the 95% confidence interval for determined MPI using all normalization genes.

Examples

Example 1

Recently, using complex microarrays, a 70-gene prognosis profile was identified that is a powerful predictor for the outcome of disease in young breast cancer patients [6]. This profile was generated using 78 tumor samples of patients having lymph node negative disease by hybridization of fluorescent-dye labeled RNA to microarrays containing 25,000 60-mer oligonucleotide probes. To facilitate the use in a diagnostic setting, the 70-gene prognosis profile was translated into a microarray comprising a reduced set of 1,900 probes that include 915 probes that were added for normalization purposes and that are directed towards genes listed in Table 1. To enable the use of this prognostic classifier in a diagnostic setting, custom-made 8-pack mini-arrays were used (Agilent Technologies), which comprise a single 1"x3" slide containing eight identically printed regions or sub-arrays.

Aliquots of total RNA of 149 frozen tumor samples was available for this study, while for 13 samples (8 out of 78 and 5 of the 145 tumor series, see above) new RNA was isolated from available frozen tumor tissue as described previously [6]. Two-hundred nanogram total RNA was amplified using the Low RNA Input Fluorescent Labeling Kit (Agilent Technologies). Cyanine 3-CTP or Cyanine 5-CTP (Perkin Elmer) was directly incorporated into the cRNA during in vitro transcription. A total of 200 ng of Cyanine-labeled RNA was co-hybridized with a standard reference to custom 8-pack microarrays at 600□C for 17 hrs and subsequently washed according to the Agilent standard hybridization protocol (Oligo Microarray Kit, Agilent Technologies). The reference sample consisted of pooled and amplified RNA of 105 primary breast tumors selected from patients of the clinical validation series [6] in such a way that it had a similar proportional distribution between good and poor profile patients. Sufficient reference material was generated for over 30,000 hybridizations. For each tumor two hybridizations were performed by using a reversal fluorescent dye.

The 8 pack microarray contained 1,900 60-mer oligonucleotide probes that comprise the 231 prognosis related genes [6], including the genes of the 70-gene prognosis classifier, spotted in triplicate. Each array additionally comprises 289 probes for hybridization and printing quality control, 915 normalization genes listed in Table 1, and a triplicate probe for detecting the expression level of estrogen receptor 1 (ESR1). After hybridization the slides were washed and subsequently scanned with a dual laser scanner (Agilent Technologies).

For the hybridization, 200 ng each of Cy3- and Cy5-labeled RNA were hybridized to each array in a 45ul total volume of hybridization buffer (Agilent Technologies) for 16 hours at 60C, followed by room temperature disassembly in 6x Sodium chloride-Sodium Citrate buffer (SSC)/0.005% Triton X-102, a ten minute room-temperature wash in 1x SSC/0.005% Triton X-102, and a five minute room temperature wash in 0.1x SSC/0.005% Triton X-102

Dye bias was corrected by multiplying each background-subtracted intensity measurement by an appropriate dye transformation function using the non-linear (LOWESS) method of curve fitting [19, 22]. Odds ratios were calculated based on a two by two contingency table. P-values associated with odds ratios were calculated by Fisher's exact test. Survival periods of patients were analyzed from the calendar date of surgery to the time of the first event or the date on which data were censored, according to the method of Kaplan Meier. The curves were compared using the log rank test.

Fluorescence intensities on scanned images were quantified, values corrected for background non-specific hybridization, and normalized using Feature Extraction software (Agilent Technologies). Data was further analyzed using custom algorithms in Matlab (The Mathworks). To obtain an overall expression value for each of the signature genes on the array, an error-weighted mean value was calculated for the three identical probes belonging to the same gene as log10 ratios. To establish appropriate relative weights, the Rosetta error model was used, which corrects for the uncertainties in individual probe measurements [21]. Probes were excluded from further calculations if their background corrected intensities were below zero and/or if spots were flagged as non-uniformity outliers as determined by the image analysis software.

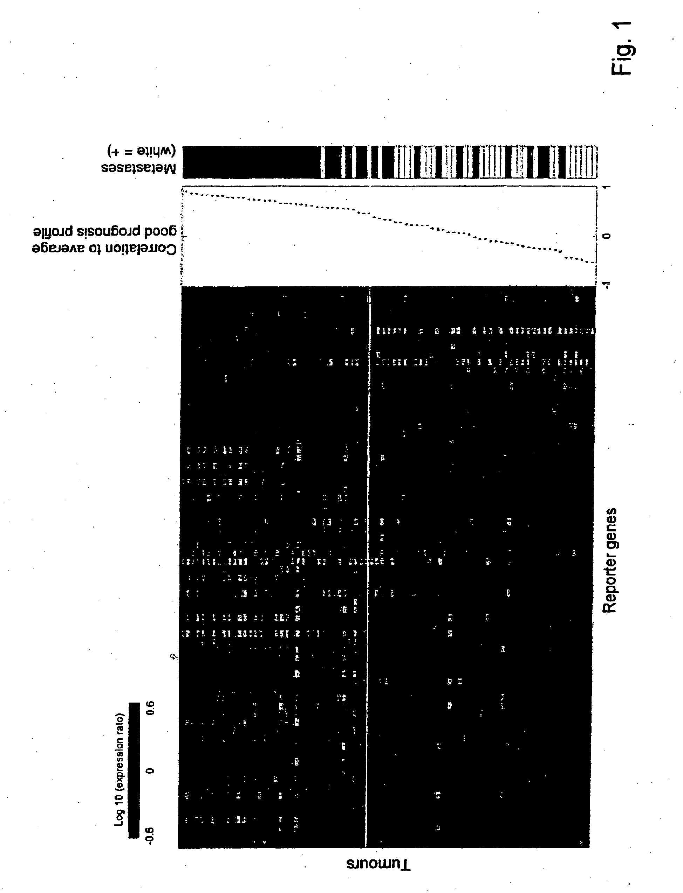

The expression intensities of the 70 signature genes for the 78 original samples hybridized to the customized array are shown in Figure 1. The tumors are rank-ordered according to their correlation coefficients with the re-established 'good prognosis template' (Figure 1 middle panel). Genes are ordered according to their correlation coefficient with the two prognostic groups as previously described. Tumors with correlation values above or below the previously determined threshold (indicated by the yellow line in Figure 1) were assigned to the good or poor prognosis profile group, respectively. The right panel in Figure 1 shows the distant metastasis status of the patients and confirms the strong correlation of prediction and high accuracy between the profile predicted and actual outcome of disease of the patients, as observed in the original studies.

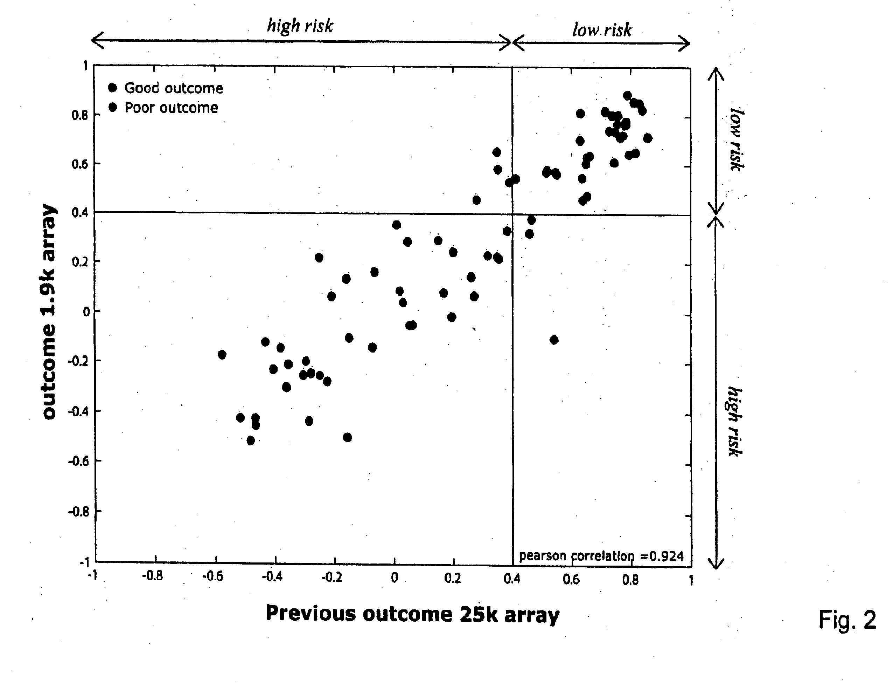

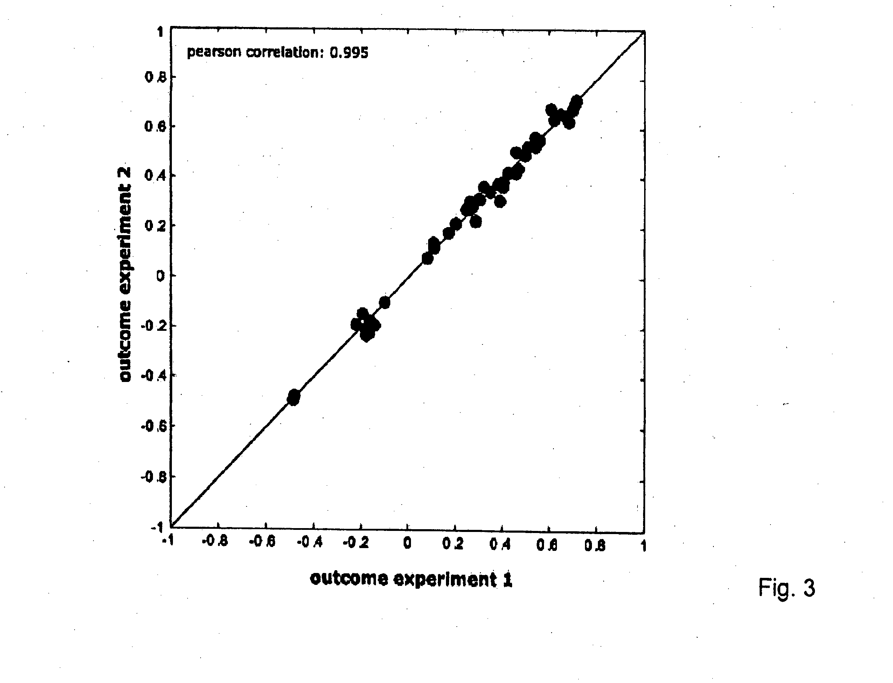

Outcome prediction for the 78 tumor samples used in Figure 2 and 3 was performed as described by Van 't Veer et al [6]. In brief, the 'good prognosis template' was (re-)constructed using the average expression for each of the 70 genes in tumors from the 44 'good outcome' patients as determined on the customized mini-array. Subsequently, the expression of the 70 profile genes for each patient was correlated in a leave-one-out cross validation procedure to the 'good prognosis template'. A patient with a cosine correlation to the good prognosis template higher than 0.4 was assigned to the good-profile group. Patients with a correlation lower than this threshold were assigned to the poor-profile group.

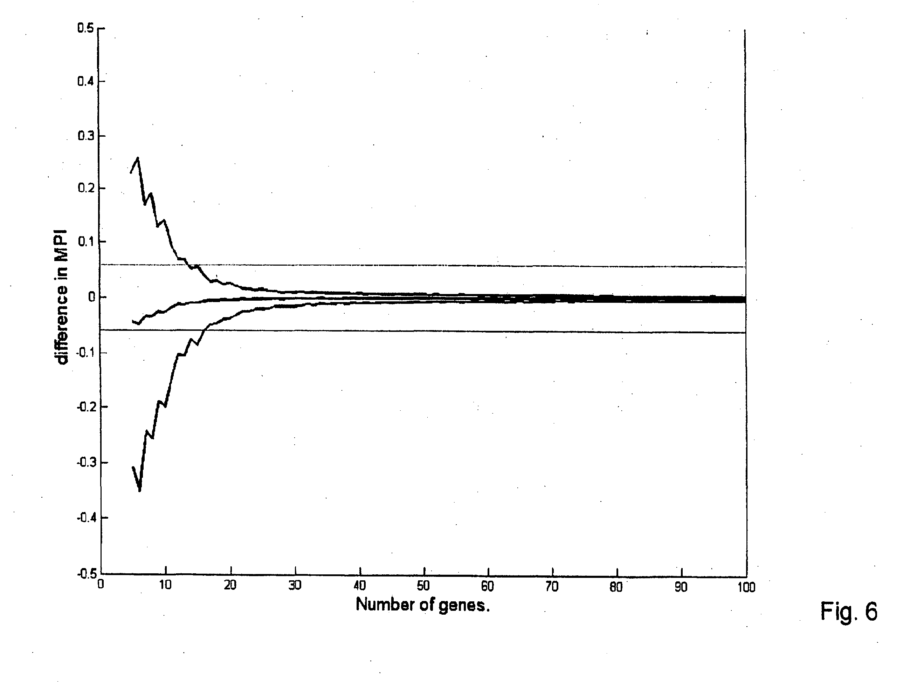

Outcome prediction for the 145 tumor samples used in Figure 6 was performed as described [6]. For each of the 84 tumors from patients that were not included in the original study [6], a correlation coefficient of the 70-gene expression with the template was calculated as described above. For the 61 patients who were included in the original study [6], correlation coefficients were calculated according to the cross-validated classification method using all 231 genes. This approach was originally employed to minimize the overestimation of the value of the prognosis profile. The only deviation is that 231 instead of a varying number of prognosis correlated genes (range $238 \pm 23$) were used in the cross-validation procedure since only these 231 genes are present on the mini array.

To perform a comprehensive evaluation of the microarray results, we compared the current classification to the good and poor prognostic profiles with that of the originally published classification for each sample (see Figure 2). Results from the original study are shown (X-axis) plotted against those obtained from the customized mini-array (Y axis). The data generated using the diagnostic test is highly similar (Pearson correlation of 0.92, p<0.0001) to the original published data. The overall accuracy of the diagnostic test was determined by calculating the odds ratio for the development of distant metastases within five years. The odds ratio calculated based on the current results (OR =13. 95%CI 3.9 to 44) was highly comparable to the original data (OR=15, 95%CI 2.1 to 19) using the methods described in the supplementary information of reference [6].

A more detailed evaluation revealed seven discordant cases between risk assessment using an 8-pack mini-array and the published data. These cases included two patients that did not develop distant metastases, who were classified as having poor prognosis in the published data. However, the present diagnostic test correctly classified them into the good prognosis group. Furthermore, one patient who did develop metastases was originally classified as good prognosis, whereas in the current results this patient was classified correctly as having a poor prognosis. On the other hand, however, there were two good outcome patients classified as poor prognosis using the diagnostic test, while in the original data these samples were classified correctly, as well as two poor outcome patients classified as good prognosis by the current test who where correctly classified by original analysis as poor prognosis.

These results indicate that the genes listed in Table 1 can be used for normalizing hybridization data from arrays comprising a reduced set of probes.

Example 2 Reproducibility

To further investigate if the differences seen were due to technical variation of the current test or could be otherwise explained, 49 samples were amplified and hybridized a second time to an 8-pack mini-array (Figure 3). The Pearson correlation for the replicate experiments was 0.995, indicating a very high degree of reproducibility of classification for individual tumor samples using the customized 8-pack array. Also an ANOVA analysis performed on the 70-gene expression values obtained in the duplicate experiments showed no significant differences, independent of variation between individual samples and profile genes (p=0.960).

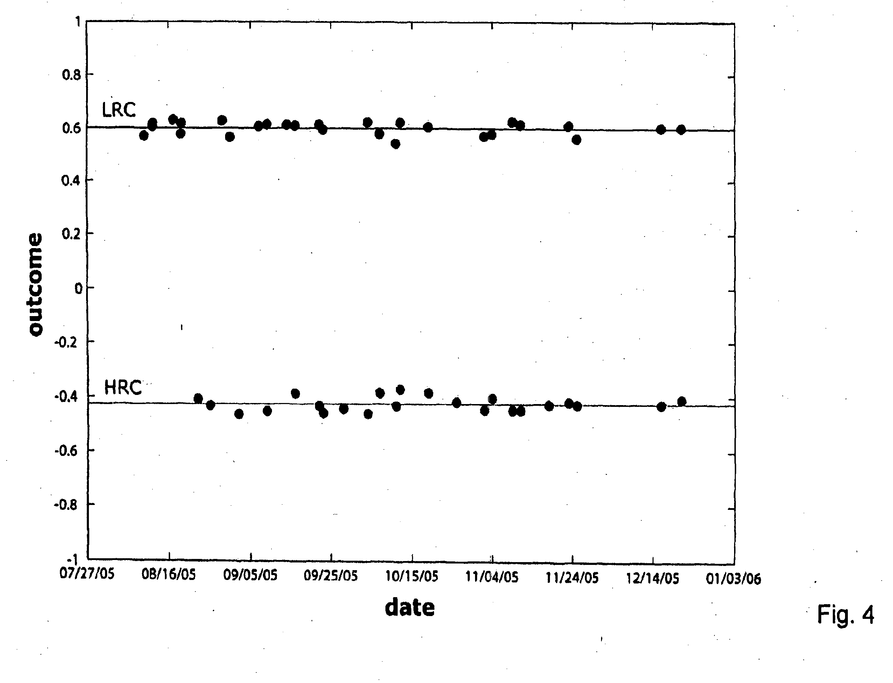

To ensure that the outcome of the test does not change over time, two samples were amplified and labeled repeatedly over a period of 5 months (Figure 4). One sample (HRC) was classified as poor prognosis with an average cosine correlation to the good prognosis template of -0.43. The other sample (LRC) was classified as good prognosis (average correlation to the good prognosis template of 0.60). Both samples were stable over time as shown and the diagnostic test result was observed to have a very low standard deviation (LRC stdev 0.026, HRC stdev 0.023), indicating the robustness of the diagnostic test.

Example 3 Clinical validation

To more accurately estimate the risk of metastases associated with the 70-gene prognostic profile, a validation study was performed using a cohort of 295 young breast cancer patients. For the current study we selected 151 patients from a cohort without lymph node involvement at diagnosis [13] of which 145 RNAs were available.

We calculated the probability of a patient remaining free of distant metastases and overall survival according to the prognosis profile and compared this to the published data [13].

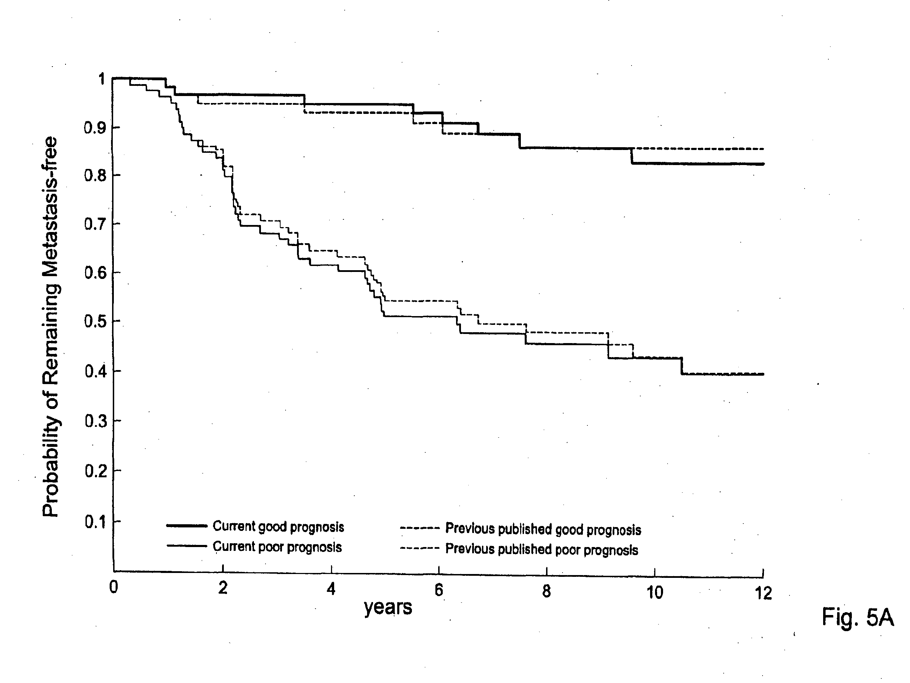

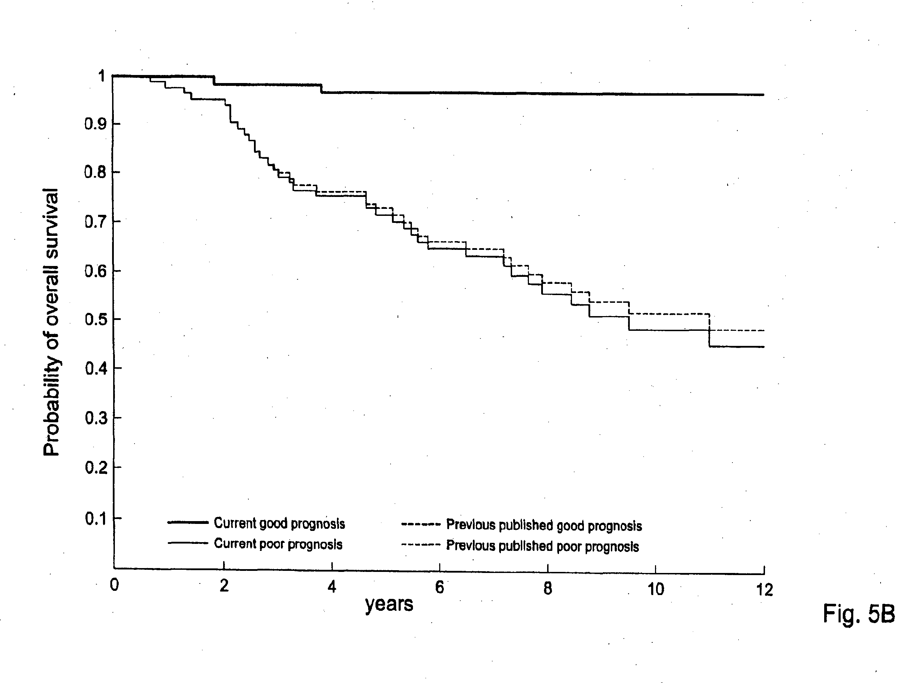

The data generated using the customized array was found to be highly similar (Pearson correlation of 0.88, p<0.0001) to the original data. As was seen before, Kaplan-Meier curves showed a significant difference in the probability that patients would remain free of distant metastases when classified in the good or poor prognosis profile group (Figure 5A, LogRank p< 0.001). The difference in prediction of probability of overall survival (Figure 5B) between the groups with good or poor prognosis profiles was also highly significant (p<0.001). The estimated hazard ratio for distant metastases as a first event in the group with a poor prognosis signature versus the group with a good-prognosis signature, over the entire follow up period, was 5.6 (95% CI 2.4 to 7.3, P < 0.0001). This confirms the published data [13] (HR=5.5, 95%CI 2.5 to12.2, P<0.001).

When the probability of a patient remaining free of distant metastases was compared between the current result (Figure 5 blue line) and the original analysis (Figure 5 dashed green line) in the good prognosis profile groups, no significant difference was found (logRank p=0.890). Similarly for those patients in the poor prognosis profile groups (logRank p=0.794) (Figure 5 red and dashed magenta lines). Equally, there is no significant difference in overall survival of patients grouped by either the current result or the original published result for either prognosis profile group (two results of good prognosis profile group: logRank p=0.747, two results of poor prognosis profile group logRank p=0.760, respectively). All results taken together indicate that there is not only a strong correlation between good prognosis and the absence of distant metastasis or death but the findings generated using the more complex microarray platform were nearly perfectly reproduced using the customized mini-arrays, and demonstrate the robustness of the 8-pack mini-array test.

Example 4

A total of 329 breast tumor samples were hybridized to customized 8 pack microarrays. For each sample, data of duplicate hybridizations were analyzed (reverse color).

For every single hybridization (n=658), background (BG) subtracted raw intensity columns from the Feature Extraction (FE) txt file were read into Matlab using XPrint code. The intensities were used to calculate the

$$\text{logRatio (M) as } M = \log 10(R/G; \text{ and}$$

the average intensities A = log10(sqrt(R.G);
whereby R defines the raw intensities for the red channels and G defines the raw intensities for the green channel.

Data for each slide was normalized using increasing number of normalization genes. The raw intensities were normalized with different sets of normalization genes for each number of normalization genes, using Lowess normalization within Matlab which is comparable to the normalization used in Feature extraction. After each normalization, the duplicate hybridizations were combined and a correlation to the average good prognosis profile (MPI) was calculated using XPrint code as reported (WO 2002-103320). The calculated MPI was compared to the MPI calculated using Feature extraction normalized log ratios.

Using different numbers of normalization genes, and up to hundred (100) different sets of normalization genes for each number, a Lowess curve was calculated. This is the most optimal curve through the normalization genes and represents the dye-bias of the experiment. The calculated Lowess curve was interpolated to the rest of the genes spotted on the array. At each intensity, the difference of the Lowess curve and the log10 = 0 line was subtracted from the genes over the complete intensity range.

MPI was calculated using the normalized logratios (M - lowess-value) and compared to the MPI using all normalization genes.

All calculations were performed in Matlab 7.4.0. The average intensity per gene over the 658 hybridizations was calculated for 465 normalization genes (genes labeled 1-465 in Table 1). Subsequently, the normalization genes were divided in five groups of equal size, i.e. 93 genes per intensity group as determined by the average intensity per gene.

Random sets with increasing number of normalization genes were chosen to define the minimum number of genes that can be used for normalizing the data. The number of normalization genes varied between 5 genes and 100 genes. Data derived from 100 independent sets of randomly selected normalization genes were determined for each number of normalization genes, wherever possible.

For all samples, the MPI determined using all normalization genes was subtracted from the new MPI calculated with different numbers comprising different sets of normalization genes. The min, max and mean were plotted using a confidence interval of 95%.

The total of normalizations performed is: 658 * 96 * 100 = 6,316,800.

Figure 6 shows the mean difference of the MPI calculated for all sets comprising increasing numbers of randomly selected normalization genes, as well as the largest differences observed. The red lines indicate the upper and lower limits of +0.06 and -0.06, being two times the determined technical variation (2xTechVar) of MPI using the normalization genes.

These results indicate that a minimal number of randomly selected normalization genes is between 5 and 10. From a total of 18 randomly selected normalization genes onwards, a MPI within the 2xTechVar is obtained.

In a further experiment, the standard deviation of the 658 hybridizations was calculated for all 465 normalization genes (gene numbers 1-465 in Table 1) and the genes were sorted according to the standard deviation.

Starting with the top 5 ranked genes with the lowest standard deviation, the number of normalization genes was increased from 5 onwards to normalize all 658 hybridizations.

The difference between the calculated MPI, normalized with a number of rank-ordered normalization genes and the MPI as determined with all normalization genes was calculated and for every subset of normalization genes the mean, min and max was after removing 5% of the outliers.

The total number of normalizations done for this test is: 658 * 465 = 305,970 normalizations.

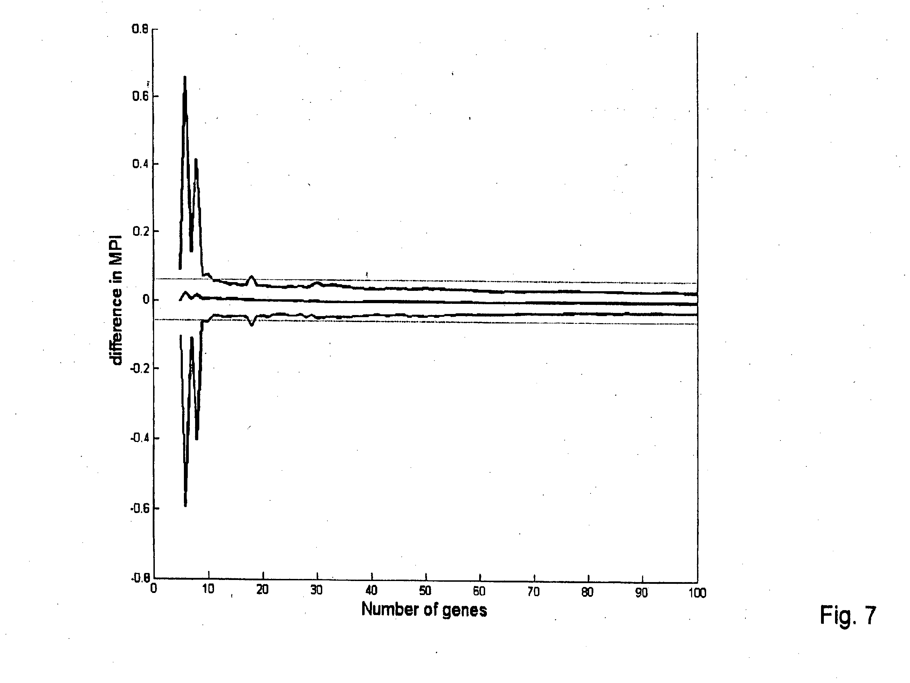

Figure 7 shows the results from the normalization experiments starting with the top 5 rank-ordered genes with the lowest standard deviation to normalize the microarray data. These results indicate that a minimal number of randomly selected normalization genes is about 9. From a total of 10 rank-ordered normalization genes onwards, a MPI within within the 2xTechVar is obtained.

In yet a further experiment, random selection of normalization genes from each of five different intensity intervals was used for MPI determination. Starting with one gene selected from each of five equally divided intensity intervals, from five up to 93 normalization genes were selected. Whenever possible, hundred independent sets of genes were chosen and MPI was determined for all sets for every number of normalization genes.

The difference between the calculated MPI, normalized with a set of normalization genes, and the original MPI was calculated and for every set of normalization genes the mean, min and max was calculated after removing outliers.

The total of normalizations done for this test is: 93 * 100 * 658 = 6,119,400 normalizations.

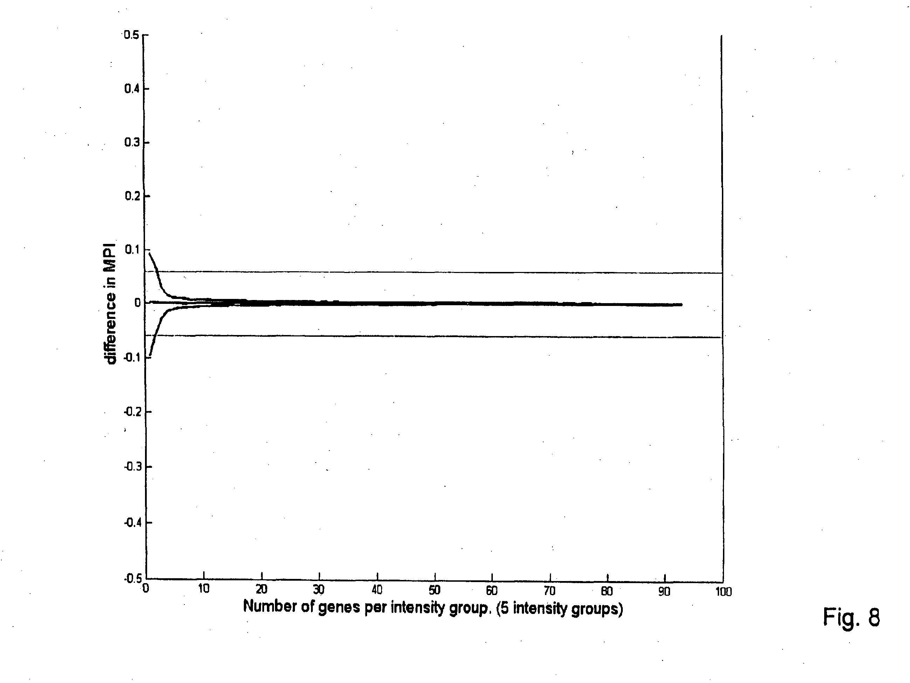

Figure 8 shows the results from the normalization experiments starting with 5 normalization genes, one from every intensity interval, to normalize the microarray data. These results indicate that a minimal number of randomly selected normalization genes from each intensity interval is about 1, resulting in a total of 5 normalization genes. From a total of 2 normalization genes per intensity interval onwards, a MPI within the 2xTechVar is obtained.

In an even further experiment, normalization genes within the five equally divided intensity intervals were selected starting with the genes with the lowest standard deviation. Starting with 5 genes, one gene with the lowest standard deviation from each intensity range, a gene with the next lowest standard deviation in each intensity range was added to the set of normalization genes to normalize the samples.

The difference between the calculated MPI, normalized with a set of normalization genes, and the original MPI was calculated and for every subset of normalization genes the mean, min and max is calculated after removing outliers.

The total of normalizations done for this test is: 93 * 658 = 61,194 normalizations.

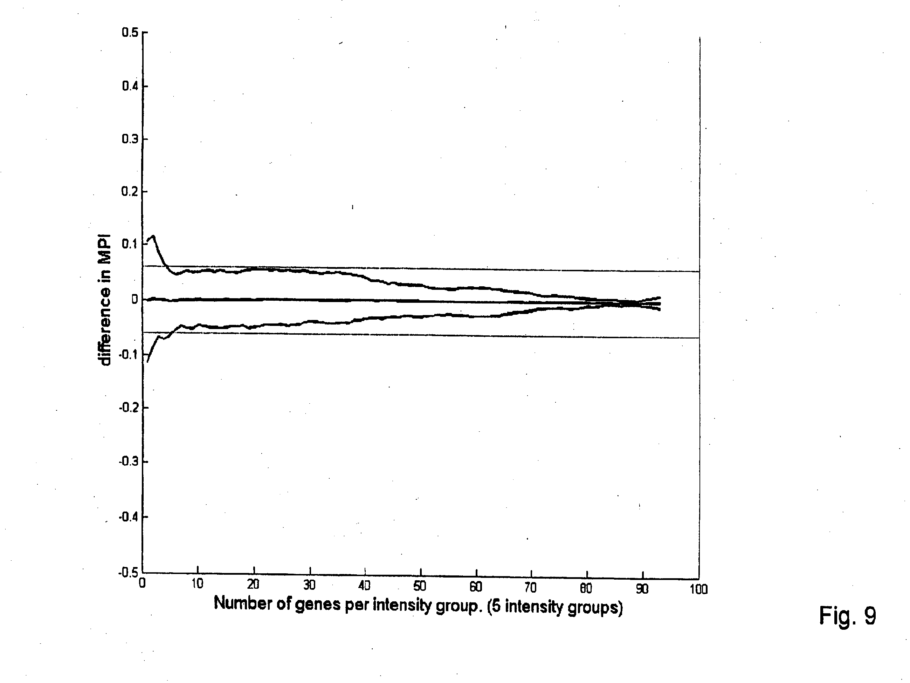

Figure 9 shows the results from the normalization experiments starting with 5 rank-ordered normalization genes, one from every intensity interval, to normalize the microarray data. These results indicate that a minimal number of rank-ordered normalization genes from each intensity interval is about 3, resulting in a total of 15 normalization genes. From a total of 10 rank-ordered normalization genes per intensity interval onwards, a MPI within the 2xTechVar is obtained.

Conclusion:

Using a microarray test as described in a clinical setting will provide accurate information on recurrence risk as compared to conventional clinical criteria and may improve the guidance for the requirement of adjuvant therapy for women diagnosed with breast cancer. As a result, many patients may be spared the side effects and risks of such treatment, improving quality of life and reducing healthcare costs.

References

1. Golub et al., Science, 286:531 (1999).
2. Alizadeh et al., Nature, 403:503 (2000).
3. Perou et al., Nature, 406:747 (2000).
4. Sorlie et al., Proc Natl Acad Sci U S A, 98:10869 (2001).
5. Pomeroy et al., Nature, 415:436 (2002).
6. van 't Veer et al., Nature, 415:530 (2002).
7. Bittner et al., Nature, 406:536 (2000).
8. Hedenfalk et al., N Engl J Med, 344:539 (2001).
9. Ramaswamy et al., Proc Natl Acad Sci U S A, 98:15149 (2001).
10. Glas et al., Blood, 105:301 (2005).
11. Khan et al., Nat Med, 7:673 (2001).
12. Huang et al., Lancet, 361:1590 (2003).
13. van de Vijver et al., N Engl J Med, 347:1999 (2002).
14. Wang et al., Lancet, 365:671 (2005).
15. Jansen et al., J. Clin. Oncol. 23:732 (2005).
16. Chang et al., The Lancet, 362:362 (2003).
17. Ayers et al., J. Clin. Oncol. 22:2284 (2004).
18. Hannemann et al., J. Clin. Oncol., 23:3331 (2005).
19. Yang et al., Nucl. Acids Res., 30: 15 (2002).
20. Schmittgen and Zakrajsek, J. Biochem. Biophys. Methods, 46: 69 (2000).
21. Weng et al., Bioinformatics, 22:1111 (2006).
22. Quackenbush, Nat Genet 32 Suppl: 496 (2002).
23. Cleveland, J. Am. Stat. Assoc. 74: 829 (1979).
24. Cleveland and Devlin, J Am Stat Assoc 83: 596 (1988).

Table 1

| | Genbank accession number | Gene Symbol | Probe Sequence | Intensity group | STD order |
|---|---|---|---|---|---|
| 1 | NM_002611 | PDK2 | GGCTTTCTCCCTAGGACCTTCTGTGTATATAGTTAGTTTTATAACCCTGAATGCCCCCAC | 4 | 120 |
| 2 | NM_000871 | HTR6 | TGCCATATGCTTCACCTACTGCAGGATCCTGCTAGCTGCCCGCAAGCAGGCCGTGCAGGT | 1 | 162 |
| 3 | NM_018520 | | GAGGCGCAGTGTCTTTTTAACCACTTTGAAGACCAACAGCCAAGACATCTTGAGCTAGTT | 1 | 442 |
| 4 | NM_006671 | SLC1A7 | CCATCACCTTCAAGTGCCTGCTGGAGAACAACCACATCGACCGGCGCATCGCTCGCTTCG | 4 | 44 |
| 5 | NM_013403 | STRN4 | ATTATCACTGTGTGATGGTCTCAGTCAGTCTCCTCCCTGTCTCCACTCTTTCCCTCTATT | 5 | 81 |
| 6 | NM_016347 | CML2 | CACAGACATGTCTGACATCACCAAATCCTACCTGAGTGAGTGTGGCTCCTGCTTCTGGGT | 4 | 45 |
| 7 | NM_002230 | JUP | CTCACCAACTCCCTCTTCAAGCATGACCCGGCTGCCTGGGAGGCTGCCCAGAGCATGATT | 4 | 218 |
| 8 | NM_007264 | ADMR | CATGCTGCACTGTGTCATCAACCCCATCCTTTACAACTTTCTCAGCCCACACTTCCGGGG | 3 | 54 |
| 9 | NM_016621 | PHF21A | GCAAGGAGAACAGAACACTGAAGACTCTAGAAAAGCAAAGCCGGATTTCTGGAAAGTGCA | 4 | 219 |
| 10 | NM_001738 | CA1 | TTTGATGATTCTGAGAAGAAACTTGTCCTTCCTCAAGAACACAGCCCTGCTTCTGACATA | 2 | 5 |
| 11 | NM_006439 | MAB21L2 | AAAGTTTTACTGATGTTAAACGTTCTCAGTGCCAATGTCAGACTGTGCTCCTCCCTCTCC | 2 | 27 |
| 12 | NM_004343 | CALR | AGCAGAAGGGGGTGGTGTCTCCAACCCCCCAGCACTGAGGAAGAACGGGGCTCTTCTCAT | 5 | 397 |
| 13 | NM_017701 | PRR5 | TGTATTTCTGTCTTGGTTGGAAATACCATCAGCCTTCCTTGCTCGGCCCAGGTCTGTTTC | 4 | 87 |
| 14 | NM_005839 | SRRM1 | TCTTGTATTTGGAAGGCTGGAAGGGCCCAGACTTTGGAATAGTGTCTTGGTTTCACTGTT | 5 | 334 |
| 15 | NM_004142 | MMP25 | CTGTGTGTTCTCTGGATCTTTTCAGCCCTGTGGTCCAGTGTCCATCACAGCCATGCTGAC | 3 | 67 |
| 16 | NM_016174 | CEECAM1 | TGGGGTCTCCGTCCACGTGTGCAATGAGCACCGTTATGGGTACATGAATGTGCCGGTGAA | 2 | 108 |
| 17 | NM_004770 | KCNB2 | AGCACATCAGTACCATCCTCTTAGAAGAAACCCCCTCCCAGGGAGACAGACCCTTGCTGG | 4 | 153 |
| 18 | NM_006605 | RFPL2 | AAATTACTTGGGTGGGTAGACTTAGGAACGCTCTACTTCGTAAAAGCATTATACAAAGTC | 1 | 450 |
| 19 | NM_003021 | SGTA | CTCCCTGGCTCCTGGCCGTCTGTGAGGTAGGCGCAGTACCGTGTATCGTAGGTAGCAGTA | 5 | 112 |
| 20 | NM_003305 | TRPC3 | GACAGGGACCATGTCTTATTCATCTTTGTGTCTCCAGCATCTAGTACAGTGCCTGGTATA | 2 | 250 |
| 21 | NM_005056 | JARID1A | CTACAAGGCAAGGAGCCGTTTTTTGGCTTTGAAAAGTCTTGCTGTCTTGGGTCTACATTT | 5 | 446 |
| 22 | NM_004533 | MYBPC2 | CCAAGACAATTGGTGGTGGAGTCCTGACCCCAATCCCCAACCTCCCAGGACTGTGTTCTT | 3 | 114 |
| 23 | NM_014390 | SND1 | GTCCAACTGTTGATTATGTGATTTTTCTGATACGTCCATTCTCAAATGCCAGTGTGTTCA | 5 | 190 |
| 24 | NM_012345 | NUFIP1 | TATGGCTTATGGGTTAATAAATGAATTCATGGACTCCTGGACTACTTTCATTGATGACCA | 1 | 209 |

| | Genbank accession number | Gene Symbol | Probe Sequence | Intensity group | STD order |
|---|---|---|---|---|---|
| 25 | NM_004047 | ATP6V0B | TCACTTTTATTTATTGCTGGTTTTCCTGGGACAGCTGGAGCTGTGTCCCTTAGCCTTTCA | 5 | 340 |
| 26 | NM_020213 | | ATCTATGCTGAAGCCAGCTGTCTGTACTCGTGAACTATGCGTTTTCTCCTTCTACACACT | 1 | 244 |
| 27 | NM_006298 | ZNF192 | AAAGCAATCAAAGTCTTACGTCATAGAGGACCACCTTTTGGATCATAAATTCTTTCCCTA | 2 | 447 |
| 28 | NM_001042 | SLC2A4 | GCAGCCATGGCTGTGGCTGGTTTCTCCAACTGGACGAGCAACTTCATCATTGGCATGGGT | 1 | 260 |
| 29 | NM_003245 | TGM3 | AACGAGGCTCGTGTGCGGAAGCCTGTGAACGTGCAGATGCTCTTCTCCAATCCACTGGAT | 1 | 167 |
| 30 | NM_004314 | ART1 | CTGAGGATGTTGGCCATGTGTGCTTCAGTGTAACCAAGATTCCTGTCAATCCCATCTGCA | 1 | 140 |
| 31 | NM_007102 | GUCA2B | GAGGCTTCCAGCATCTTCAAGACCCTGAGGACCATCGCTAACGACGACTGTGAGCTGTGT | 3 | 405 |
| 32 | NM_012164 | FBXW2 | TGAGATAGCAAACTTGGCCTTGCTTGGCTTTGGAGATATCTTTGCCCTGCTGTTTGACAA | 3 | 210 |
| 33 | NM_017700 | FLJ20184 | TTTTTTCCCCCTGCGAGAATGACTAAAAATAACATGGAAGAAGATTTAGAGCTCTGCAGC | 2 | 82 |
| 34 | NM_016494 | LOC51255 | GAGACTGCCATTGAGATGCCTTGCCATCACCTTTTCCATTCCAGCTGCATTCTGCCCTGG | 5 | 174 |
| 35 | NM_006462 | RBCK1 | TGCTGAAAACCGCAGTGCCTTCAGCTACCATTGCAAGACCCCAGATTGCAAGGGATGGTG | 2 | 361 |
| 36 | NM_004122 | GHSR | TGTGGGTGTCCAGCATCTTCTTCTTCCTTCCTGTCTTCTGTCTCACGGTCCTCTACAGTC | 5 | 188 |
| 37 | NM_018959 | DAZAP1 | TTTTTT GGAAATTATTTTCCTGAGCCTTTTGTTTTACGGTATATTGTAAACTTTTATGTT | 4 | 200 |
| 38 | NM_004256 | SLC22A13 | CCCCCAATACTCTGTCTGGGTTAGGATCTTGGGTATGTCTTGGAATTAACTTGTCCTCTA | 1 | 24 |
| 39 | NM_002777 | PRTN3 | ATGGCATCATCCAAGGAATAGACTCCTTCGTGATCTGGGGATGTGCCACCCGCCTTTTCC | 1 | 142 |
| 40 | NM_003926 | MBD3 | GCCAGGGCAGCCGAGGGAAGCTCCAGGTGGGGACCACGTCTTCCTGAGGTTGGTGCCCAC | 5 | 337 |
| 41 | NM_004760 | STK17A | CACCTCCCCCCATGCTTGTCATTTAATTTTGGCCACTTGTAGGTATCAGTGTGATCTGAT | 5 | 410 |
| 42 | NM_003301 | TRHR | TTGGTTTTTGCTCTTTTGCAGAATTTGCATTTATCTCAACAGTGCCATCAACCCGGTGAT | 1 | 356 |
| 43 | NM_005968 | HNRPM | TTGGGGTAATTTGAATTACIIIIIAATGACTGGGGTTCCATTTGACTGTTTGCATTGAG | 5 | 369 |
| 44 | NM_013286 | RBM15B | TATCTCCTGGGTTATTTTGGTTCATTTGGGTGGGGATCAAAGTCCTGTCCACCACCAAAA | 4 | 134 |
| 45 | NM_002843 | PTPRJ | GCACCCACAGCGAAGGCACATGCCCCGATGTCGACATGTTTTTATATGTCTAATATCTTA | 2 | 148 |
| 46 | NM_017612 | ZCCHC8 | TGTGACTATCCGTCGAGAGTGATGGTTTTTATCTGTCTTTTGTACATTGTTTTCCCTTTC | 3 | 413 |
| 47 | NM_007017 | SOX30 | TATAACAGTCATAGCCACAGTGGGGAAGAAAACTTAAACCCTGTGCCTCAGCTGGACATT | 1 | 213 |
| 48 | NM_017420 | SIX4 | CCCAGCAGTACATCAGGATTTTGTCCAAGAACATCGTTTGGTTCTGCAATCGGTAGCTAA | 1 | 445 |

(continued)

| | Genbank accession number | Gene Symbol | Probe Sequence | Intensity group | STD order |
|---|---|---|---|---|---|
| 49 | NM_017703 | FBXL12 | CTCACGGTTACATTGCAAAGCCTTACTCTAAAAGCTCCCAGCCTCCAGAGGCTCTCAATG | 3 | 160 |
| 50 | NM_015726 | WDR42A | TCTTACCTTTAGCTGCTTGATCATTAAGCCATCCAACTTCATGCCAGTTCCCTTCTTTAT | 3 | 217 |
| 51 | NM_016498 | SEC14L2 | AGCTCCTCCTGATCATACTCTGGTACCTGGCCTGTGCATCGGCCTCCTGCTTCATGTCAA | 4 | 38 |
| 52 | NM_016407 | C20orf43 | GCTTTAAAAAGGATGGATTTCAAATACACTGTGCCCACTAGAAGCTTCGAAGGGCCTCGT | 5 | 257 |
| 53 | NM_017841 | FLJ20487 | TGGTCCTGCCTCAAGTGATGGACATAACCCTCTCCTAGGACATACATGTAAATGCACAAT | 4 | 96 |
| 54 | NM_014807 | TMEM24 | GGGACAAGTGCAGGTTTCTTACATGCATATATTGCATGCATAGAGGTGAAGTCTGGGCTT | 1 | 314 |
| 55 | NM_002632 | PGF | AATGTCACCATGCAGCTCCTAAAGATCCGTTCTGGGGACCGGCCCTCCTACGTGGAGCTG | 3 | 293 |
| 56 | NM_014717 | ZNF536 | TACAGTTCTGATGGCTTAGCAGCCTTTAACGGACTTGCAAGTAGCACAGCAAATTCTGGA | 3 | 464 |
| 57 | NM_016046 | EXOSC1 | AGTACAACCCGAATTCTTGCAGACCTAAGAAGCCACTTTTACCCTATGGAAGGGGGTAA | 4 | 228 |
| 58 | NM_004724 | ZW10 | TATGTGGATTCTTCGCTTGGCATAATTACTCCCTTAAAGACTTCTTTGAATCGCCCATTG | 3 | 310 |
| 59 | NM_003169 | SUPT5H | ACACAAGATCCTCCTGCGAGGGCTAGGCGGATTGTTCTCGGATTTCCTTTTGTTTTTCCTTT | 5 | 191 |
| 60 | NM_017610 | RNF111 | ACTGTTGCTATGGTTATATACTCCCACTTCATACATTACCAAGAGTCGATCACTGATTTA | 3 | 359 |
| 61 | NM_004676 | PRY | ACCTCCTTCTCTTCTGGACATGTCCAGGAGTGGCCGTTGCTACAAGTCACCTGGTGCTAC | 2 | 64 |
| 62 | NM_016158 | | CAAAGAAGAACCCCTTCATGTTCATTAATATTCTCCGCTCTTCTATACCCATTGATAT | 2 | 298 |
| 63 | NM_001148 | ANK2 | CCCCATCCTCTTTAAGTATAAAGCTAATTTGTGACCAAAGATGGCATCCTTCATACTGGA | 3 | 179 |
| 64 | NM_017630 | | CTTGATTCCAGTTGTAAGCCATTTCAGTTACTATAACTTTAAAAATAGGCTTTTGACTGGG | 1 | 454 |
| 65 | NM_004121 | GGTLA1 | TCATCATCTCTGCTGTGGCCCCAGCCCATCATGAGCAAGAGCAGTGTGGCTTGGACCTGA | 2 | 404 |
| 66 | NM_002939 | RNH1 | TCACCCTGCATATCCTAGGTTTGAAGAGAAAACGCTCAGATCCGCTTATTTCTGCCAGTAT | 5 | 280 |
| 67 | NM_004258 | IGSF2 | ACAGACCCCGGCAACTTCTAGATGAACCCAAGTGAACTTTCCTCATTACCATCCTGAAGT | 3 | 65 |
| 68 | NM_015582 | | TCTCTCATTTTCTCTTGTGGACCAGTTAGTTTTGCCCATAAACGCAGTATTCTGAGTTTGC | 5 | 323 |
| 69 | NM_018275 | FLJ10925 | CAAGATTGCCAAGCGCGAGTGCAAGGTCCTGGTGGTGGAACCCGTCAAGTAGCACCGTGC | 1 | 109 |
| 70 | NM_016372 | GPR175 | ACATCATCGAGGGGCTCTGCTGTGTAGATGCCACAACCTTCCTGTACTTCAGCTTCTTCG | 3 | 58 |
| 71 | NM_012475 | USP21 | ACAAAGCCGGAAGTCCTGTATACCAGCTGTATGCCCTTTGCAACCACTCAGGCAGCGGTCC | 4 | 198 |
| 72 | NM_014745 | FAM38A | TGGAGGAGGAGTTGTACGCCAAGCTCATCTTCCTCTACCGCTCACCGGAGACCATGATCA | 4 | 411 |

| | Genbank accession number | Gene Symbol | Probe Sequence | Intensity group | STD order |
|---|---|---|---|---|---|
| 73 | NM_014841 | SNAP91 | TGCTGAGTTTCAAAGGGAGCCACCAGTACCAAACCCAATACTTACTCATAACTTCTCTTC | 2 | 296 |
| 74 | NM_002103 | GYS1 | TAGATCTGGAACCTTACCACGTTACTGCATACTGATCCCTTTCCCATGATCCAGAACTGA | 3 | 159 |
| 75 | NM_015725 | PPAN | AGTGTCATGGGCCTGCAGGGTGTCATCTTCAACGATGTCTATGCAGCTTCCAAGTTCGCC | 1 | 94 |
| 76 | NM_019109 | ALG1 | AGGTGTCCCCTTTCTGCCGTGTTCCTAACATTTTGATTCCTGTCTTGAAAAAAGCACCTG | 5 | 20 |
| 77 | NM_004625 | WNT7A | AGAACATGAAGCTGGAATGTAAGTGCCACGGCGTGTCAGGCTCGTGCACCACCAAGACGT | 1 | 398 |
| 78 | NM_006632 | SLC17A3 | ATCAAGAGGATTTTCGAGCATAGCACCTGTCATTGTACCCACTGTCAGCGGATTTCTTCT | 2 | 420 |
| 79 | NM_007284 | PTK9L | ATGAGGGCGACCCCCTTGAGTCTGTAGTGTTCATCTACTCCATGCCGGGGTACAAGTGCA | 4 | 239 |
| 80 | NM_000339 | SLC12A3 | GGCTGAGCACACCAAGAGGTTTGAGGACATGATTGCACCCTTCCGTCTGAATGATGGCTT | 4 | 83 |
| 81 | NM_013266 | CTNNA3 | GTGCCTATATGACAAAATCCTGCCTAACCACACTGCTTTATTTTACACTTAAGAAGTTCT | 2 | 21 |
| 82 | NM_017530 | LOC55565 | CTTCTAGGACTGTGGGGCCCCTGTGTGGCCCATGAAGTTGTGAAGTCAAATAAATTAATT | 4 | 358 |
| 83 | NM_006932 | SMTN | TGATGATCATGGGCAAGAAGCCTGACCCCAAGTGTGTCTTCACCTATGTGCAGTCGCTCT | 3 | 278 |
| 84 | NM_001686 | ATP5B | TCTGTACTTGTCTCTCTCCTTGCCCCTAACCCAAAAAGCTTCATTTTTCTATATAGGCTG | 5 | 245 |
| 85 | NM_014623 | MEA1 | CCAGAACCAGCACAGGACTGAACACATCCCTGGTTGTAATGTCCATTTCCATCTTCCCCG | 5 | 248 |
| 86 | NM_001051 | SSTR3 | ACCCCATCCTTTATGGCTTCCTCTCCTACCGCTTCAAGCAGGGCTTCCGCAGGGTCCTGC | 5 | 15 |
| 87 | NM_015911 | ZNF691 | CTGGCCTTTGAGGAAGTACTTATGAGATGGGTGTCACTGTCTGAAGGTTCTCCAAATTGT | 2 | 336 |
| 88 | NM_016258 | YTHDF2 | CAAGGTTGTGTCTTTAAGGGTGGTTCATTTTCTCTGACCTTTTGTTACTCAAAGTAAAGT | 5 | 342 |
| 89 | NM_013279 | C11orf9 | GTGTGCATAGAGCGCCCCCTACTTCCCAGTTAACTCCCAGTTCTTCTCCCTGAGCTTGGT | 5 | 99 |
| 90 | NM_012222 | MUTYH | CAGTGACACCTCTGAAAGCCCCCATTCCCTGAGAATCCTGTTGTTAGTAAAGTGCTTATT | 5 | 121 |
| 91 | NM_017595 | NKIRAS2 | CCCAGACAGGAAGCAGAGTCACCACGCAGCAGTGTCCCTTCTTGGGTCTGAGTTCCTATT | 3 | 256 |
| 92 | NM_018678 | | GATAATGCAGAGAGGTACCATCTTGATTTTCCCCAGTTACATTCACCTGGTCTGGTCTCT | 3 | 269 |
| 93 | NM_002408 | MGAT2 | TGTTTGTTAAACACCCTGTCAGAACAGTCATTTTCAGTATTAGATTCCTGTACTATTGTG | 4 | 419 |
| 94 | NM_003252 | TIAL1 | AAGGGCTATTCATTTGTCAGATTTTCAACCCATGAAAGTGCAGCCCATGCCATTGTTTCG | 3 | 363 |
| 95 | NM_000030 | AGXT | TGGCCAACTTCTGGGGCTGTGACGACCAGCCCAGGATGTACCATCACACAATCCCCGTCA | 1 | 221 |
| 96 | NM_017834 | | AAAGTTCCCTGGACACGAAGCCAGGAGATCTGTGTTCTAAGCCCAGACTCACTGTCACCT | 2 | 286 |

(continued)

| | Genbank accession number | Gene Symbol | Probe Sequence | Intensity group | STD order |
|---|---|---|---|---|---|
| 97 | NM_019609 | CPXM | TCACGCCCACACCAGATGATGCTGTGTTTCGCTGGCTCAGCACTGTCTATGCTGGCAGTA | 3 | 352 |
| 98 | NM_003928 | CXX1 | GTGCCTTTGTTCAACACAGTAAGCCCTGCTCCCTTCCCTGCTCTAATACACTACCTGTA | 5 | 2 |
| 99 | NM_002896 | RBM4 | TTACGGGCATGAGAGTGAGTTGTCCCAAGCTTCAGCAGCCGCGCGGAATTCTCTGTACGA | 4 | 157 |
| 100 | NM_001181 | ASGR2 | CTCTGGCTAACCCATACCCCACACCTGCCCCAGCTCTCGGCTTCTCTGTTGAGGATTTTGAG | 5 | 10 |
| 101 | NM_006715 | MAN2C1 | TACCCACTACAATACCTCTTGGGACTGGGCTCGATTTGAGGTGTGGGCCCATCGCTGGAT | 3 | 327 |
| 102 | NM_001771 | CD22 | GGAAAGCCCAGAAAAGGACAGAAAACGAAGTAGAAAGGGGCCCAGTCCTGGCCTGGCTTCT | 2 | 246 |
| 103 | NM_007259 | VPS45A | AGGTTTTCCCTACTAAACAAAGGTGTTGGAGAGCAGCTTTGGGGTTCTGTGCTGGTTGTTA | 3 | 375 |
| 104 | NM_004356 | CD81 | GTTCGAGAGCCGAGTCTGTGGGCACTCTCTGCCTTCATGCACCTGTCCTTTCTAACACGT | 5 | 312 |
| 105 | NM_004205 | USP2 | AGGCAGAAAACGGTGTATAAAGAAGTTCTCCATCCAGAGGTTCCCAAAGATCTTGGTGCT | 2 | 306 |
| 106 | NM_004112 | FGF11 | AGTTAAGAAGACCAAGGCAGCTGCCCACTTTCTGCCCAAGCTCCTGGAGGTGGCCATGTA | 1 | 77 |
| 107 | NM_019045 | WDR44 | ATTTCAGTGGCTCTTTTGGCCTCTTACTAGGGGGGATAGTCTTGTTTCTAGCTTAAACAA | 2 | 422 |
| 108 | NM_018655 | LENEP | GCTTGGCACAGTGAGCCCACCAGCTCAGATGGTTGATCTTACCATACCCTCATAGTACCA | 1 | 90 |
| 109 | NM_003422 | MZF1 | GTGTGGCAAGGCCTTCCGCCAGCGGCCCACGCGTCACGCAGCATCTGCGCACCCACCGACG | 4 | 304 |
| 110 | NM_000124 | PGBD3 | GCTAAACAACATTGCTTCCTAAACTTTCAAGTCCCTTTTCTAACGGCATTTCTGATTA | 2 | 344 |
| 111 | NM_005929 | MFI2 | CGACACCAACATGTTCACCGTGTATGGACTGCTGGACAAGGCCCAGGACCTGTTTGGAGA | 2 | 283 |
| 112 | NM_007008 | RTN4 | GCAGTGTTGATGTGGTATTTACCTATGTTGGTGCCTTGTTTAATGGTCTGACACTACTG | 5 | 432 |
| 113 | NM_017738 | C9orf39 | CAGGGAGCAACAGATTTGTAGTAGTATGAGCAAAATAAAATGAAGCATCATAAACTTGAGCAT | 2 | 459 |
| 114 | NM_003590 | CUL3 | CAACTAGGATGTCTTGAAACCCGTGCATTTAATTTTAGAAAATGGCAAATTTGTAAGCGTG | 2 | 455 |
| 115 | NM_006634 | VAMP5 | GGTGGTGGTTGGTGTCCTGCTCATCATCCTGATTGTGCTGCTGGTCGTCTTTCTCCCTCA | 5 | 230 |
| 116 | NM_014425 | INVS | TGTGTTCGGGGGAGCTGGCATAGCTAGTGCAGAGTTCAGATTTTCTGCTGATAATCTTTT | 3 | 208 |
| 117 | NM_001278 | CHUK | ACTTCAGCAGAACATGATCATTCTGTCATGTGTGTAACTCCTCAAGATGGGGAGACT | 1 | 390 |
| 118 | NM_016486 | MEM69 | TTACCACATTATCCCAACTGGTTTAAAGCCCTGAGGATAGTCACTTATTGGCCACT | 2 | 371 |
| 119 | NM_005883 | APC2 | TAAATAGTGGTAAATAGTGAAAGCCATCTGTCCTAAATGTAAAGCCATCTGTCCGGCG | 3 | 49 |
| 120 | NM_007024 | TMEM115 | TGCTGGGGCTTTCCATGGCCTTCTGCGTGTTTCTGCCCAACACTACCCAGGACTCTTGCTA | 5 | 72 |

(continued)

| | Genbank accession number | Gene Symbol | Probe Sequence | Intensity group | STD order |
|---|---|---|---|---|---|
| 121 | NM_001118 | ADCYAP1R1 | GATCAAAGGCCCTGTGGTTGGCTCTATCATGGTTAACTTTGTGCTTTTTATTGGCATTAT | 4 | 122 |
| 122 | NM_004285 | H6PD | GCTGGGCATGGGTGCCGACGGGCACACAGCCTCCCTCTTCCCACAGTCACCCACTGGCCT | 4 | 18 |
| 123 | NM_017547 | FOXRED1 | CCACCCGCTAGTTGTCAACATGTACTTTGCTACTGGCTTCAGTGGTCACGGGCTCCAGCA | 5 | 307 |
| 124 | NM_004720 | EDG4 | ATGGTTTAGGCTGTGAGTCCTGCAATG'fCCTGGCTGTAGAAAAGTACTTCCTACTGTTGG | 3 | 338 |
| 125 | NM_014470 | RND1 | CAGTCGCTCTGAACTCATCTCTTCTACCTTCAAGAAGGAAAAGGCCAAAAGCTGTTCCAT | 2 | 325 |
| 126 | NM_020168 | PAK6 | GCAGGACTTGCCTGCCTCCTCCTCTCAGTATTCTCTCCAAAGATTGAAATGTGAAGCCCC | 5 | 7 |
| 127 | NM_014963 | KIAA0963 | CCCAAGACACAGGGACCGTTTCTCCCCTAGGAGCAGCGGTGGGGAGCAGGGCCAAGGTCC | 2 | 328 |
| 128 | NM_017880 | FLJ20558 | CCCTTGACAACTTTAAATGCTAGTTAGGCACTTAGATGGCCCTGTTCCTTGGTAAACTGC | 3 | 30 |
| 129 | NM_000267 | NF1 | TTTCCCCCCATGTTGTAATGCTGCACTTCCTGTTTTATAATGAACCCATCCGGTTTGCCA | 4 | 59 |
| 130 | NM_002217 | ITIH3 | GACACACCAGCTCCTGTTGGGATGGATGGCCCGGATTTTATGGCATCTGGAACATGGGCA | 2 | 407 |
| 131 | NM_006574 | CSPG5 | AGTGTGCGACCTCTTCCCAAGTTACTGTCACAATGGCGGCCAGTGCTACCTGGTGGAGAA | 4 | 6 |
| 132 | NM_000733 | CD3E | TATCCTGGATCTGAAATACTATGGCAACACAATGATAAAAACATAGGCGGTGATGAGGAT | 3 | 463 |
| 133 | NM_020232 | TNFSF5IP1 | GTGCCAGAGTCATTGTTCTTTCAAGCAGTCATTCATATCAGCGTAATGATCTGCAGCTTC | 4 | 178 |
| 134 | NM_020421 | ADCK1 | GCTGAGCACAAGAAGAAGAATACCTGTTCATTCTTCAGAAGGACCCAGATCTCTTTCAGC | 1 | 387 |
| 135 | NM_004643 | PABPN1 | GGGTTTGCGTATATAGAGTTCTCAGACAAAGAGTCAGTGAGGACTTCCTTGGCCTTAGAT | 5 | 241 |
| 136 | NM_003280 | TNNC1 | CAGCGGCACGGTGGACTTTGATGAGTTCCTGGTCATGATGGTTCGGTGCATGAAGGACGA | 2 | 297 |
| 137 | NM_001382 | DPAGT1 | ACATGACCCTCATCAACTTGCTACTTAAAGTCCTTGGGCCCATACATGAGAGAAACCTCA | 3 | 265 |
| 138 | NM_013259 | TAGLN3 | ATCTCAGAGTCAAAGATGGCTTTTAAGCAGATGGAGCAAATCTCCCAGTTCCTAAAAGCT | 3 | 322 |
| 139 | NM_005177 | ATP6V0A1 | CCCAAAGCCCTTTCATCTTCCCCGTGCATTGTAGATGGAAGGAGCACCCATGCCATTCAC | 2 | 13 |
| 140 | NM_021191 | NEUROD4 | CCTGGATAACCTGAGGCGAGTCATGCCATGCTACTCTAAAACCCAAAAACTTTCCAAGAT | 2 | 118 |
| 141 | NM_000078 | CETP | AGCTCTTCTTAAGCCTCTTGGATTTCCAGATTACACCAAAGACTGTTTCCAACTTGACTG | 1 | 103 |
| 142 | NM_002831 | PTPN6 | AGTACAAGTTCATCTACGTGGCCATCGCCCAGTTCATTGAAACCACTAAGAAGAAGCTGG | 3 | 249 |
| 143 | NM_000369 | TSHR | AGAGTATATGCAAACGGTTTTGTAAGTTAACACTACACTACTCACAATGGTAGGGGAACT | 1 | 462 |
| 144 | NM_016525 | UBAP1 | TGAAAGATTCTTCCAGGGTTTTATTTTTTCCCCTCCTAACAAAGTCTCATAGTGTTAACA | 3 | 243 |

EP 2 084 296 B1

| | Genbank accession number | Gene Symbol | Probe Sequence | Intensity group | STD order |
|---|---|---|---|---|---|
| 145 | NM_014027 | | TATTTTAACTCTTTGCCCCTACAAACAAACAGCAGTACTTGCCAGAACCATTCTTGGGAT | 3 | 277 |
| 146 | NM_000082 | ERCC8 | ACTATGCTTAAGGGACATTATAAAACTGTTGACTGCTGTGTATTTCAGTCAAATTTCCAG | 1 | 417 |
| 147 | NM_020806 | GPHN | GTAAGAAAAATAATCTTTGCACTACCTGGGAATCCTGTATCGGCTGTGGTCACCTGCAAT | 1 | 164 |
| 148 | NM_018485 | GPR77 | GTGGCGGATTTGCTGTGCTGTTTGTCTCTGCCCATCCTGGCAGTGCCCATTGCCCGTGGA | 3 | 42 |
| 149 | NM_018319 | TDP1 | CATTGAGCCACAAACATGGAATCTCTTCTTTGTACTGGATGTCCACTTCCCTTAAAGTCT | 4 | 332 |
| 150 | NM_016202 | ZNF580 | TGAACCGGTGGTTGTCTGCGGGGAAGAGATGATAAAGAGCACGGGCACGGTCTGGTTCAT | 4 | 426 |
| 151 | NM_015590 | GPATC4 | GGATGATTATAACGGGTGGTCTCCTTAGAAAGGCTCCTTATCTGTACTCCATCCTGTAGA | 3 | 34 |
| 152 | NM_017685 | | TGAGTTTCTGCCTTGCCATCCAATTAGCTGTGTGATAAACTTCAGTTTTCTCATTTTTTA | 1 | 48 |
| 153 | NM_001288 | CLIC1 | ACTCCTGAAAGCCCTGAAGGTTTTAGACAATTACTTAACATCCCCCCTCCCAGAAGAAGT | 5 | 149 |
| 154 | NM_014516 | CNOT3 | GCAGGGCACCTACATCTACTTTGACTACGAGAAGTGGGGCCAGCGGAAGAAGGAAGGCTT | 2 | 317 |
| 155 | NM_001922 | DCT | CCCTATTGGTCACAATCGGATGTACAACATGGTTCCTTTCTTCCCTCCAGTGACTAATGA | 3 | 88 |
| 156 | NM_004401 | DFFA | GATATAGCTGCACCAACAATATCCCGCCTCCTCTAATTACATATGATGTTCTCTGTTCAA | 5 | 252 |
| 157 | NM_018052 | VAC14 | CCACCCAGTGGGGGGCTATAGCCTCAGAGACCACTCATCCTCTGGAATCAACCTCTTTCT | 4 | 403 |
| 158 | NM_017950 | | ATGCTATATAGCCTTTTTTATATTGCCTATCAAGCCCGGAATGTCTGGGTCTAGCGGGTA | 2 | 341 |
| 159 | NM_014757 | LTC4S | ATGGCTTTATTTATGAACCTGGTTTTCGGGAGTCAGGGGAGGAGATGACTTTGCTTCTGT | 3 | 169 |
| 160 | NM_002212 | ITGB4BP | TCACCTTCCAAGTTGTTCCATGGGCTCCTGGCTCTGGACTGTGGCCAACCTTCTCCACAT | 5 | 201 |
| 161 | NM_015909 | NAG | AGGATTGGGGAATGAAGTTTTGAAAATGTGTCGCTCTTTGTATAACACCAAGCAGATGCT | 5 | 377 |
| 162 | NM_006269 | RP1 | ACCTCTAAGAATTTTCCACTTCTTCAAAATGAACTTACTCTAGAAAGCTTACCCTTGGAT | 2 | 434 |
| 163 | NM_019108 | FLJ12886 | CTACCCCCACCTAGTCTTCTTGCAGAACAAAGCTCGCCGAGAGGACTTCTGTCCTCGGAA | 4 | 181 |
| 164 | NM_014649 | SNRPE | CAGGGTTCCCTCGAACTTGGGGGATCTTTTTAAAAGCAAAGTAAATCCTGCCACCATGTT | 5 | 225 |
| 165 | NM_303830 | SIGLEC5 | AAAGAAAACAGATGATGGAATTAGAGAGGTGGGCTCAAATCTAGGCCCTGGCACTGTCAT | 3 | 425 |
| 166 | NM_001988 | EVPL | AGCCTCATCCCAGGCAGTGGGTCTTCCCTCTGTCCAACCACTGTTTTATTATTTTACTAA | 4 | 128 |
| 167 | NM_001842 | CNTFR | CCCAGCCTCCTGTCTATCCCAGGGTCTCTGTTGCCACCATCAGATTATAAGCTCCTGATG | 5 | 9 |
| 168 | NM_006225 | PLCD1 | CAGCCTCTTGCTCAGAGCTAGGCCCCCAAATTGCC'fTCAGCCCTAACATAGTGTCTGCTG | 4 | 234 |

(continued)

| | Genbank accession number | Gene Symbol | Probe Sequence | Intensity group | STD order |
|---|---|---|---|---|---|
| 169 | NM_019888 | MC3R | CTGGAGACCATCATGATCGCCATCGTCCACAGCGACTACCTGACCTTCGAGGACCAGTTT | 2 | 104 |
| 170 | NM_004257 | TGFBRAP1 | AAATCCCTTTTGTGAGCCTGTGTTTGTTAGATACCCAAATGGTGGTCTTGTGCACACCCA | 4 | 92 |
| 171 | NM_001514 | GTF2B | TGTTACAATCAGACAGTCCTATAGACTGATCTATCCTCGAGCCCCAGATCTGTTTCCTAC | 2 | 439 |
| 172 | NM_020410 | ATP13A1 | CTCACCGTCATGCTCCAGTTCTTTGTGCACTTCCTGAGCCTTGTCTACCTGTACCGTGAG | 3 | 74 |
| 173 | NM_016615 | SLC6A13 | CCATCTTCGAGTCCCTCTGTGTGGCTTGGGTTTACGGAGCCAAGCGCTTCTACGACAACA | 2 | 37 |
| 174 | NM_005453 | ZBTB22 | CCCGGGTGATCTCCCACCACACTTACTGTCTTCCTTTATCTCTGTGGACTTGTATATATT | 3 | 102 |
| 175 | NM_016154 | RAB4B | GCGTGGAGTTTGGATCCCGGGTGGTCAACGTGGGTGGGAAGACTGTGAAGCTACAGATTT | 3 | 258 |
| 176 | NM_006653 | FRS3 | AAGGCATGGAGGTGGGACCAGATGCTTCCCTGTGCTGGCTGGAGTCCCCAGAGATATCAG | 5 | 68 |
| 177 | NM_003427 | TNF76 | CTACTTGGCACCAGGGACTTCCTGACACCACAGTCAATTAATTCCTCAGGGGCCTGTGGC | 4 | 173 |
| 178 | NM_007185 | TNRC4 | TTTTGGCTTTGTGAGTTTCGACAATCCGGCCAGTGCCCAGGCTGCCATCCAGGCCATGAA | 2 | 43 |
| 179 | NM_006246 | PPP2R5E | CCCTGTTTTTAGCCGGAAAGGATTCAGGATAAACATTATTATGCATTCTGAATTGGATGC | 5 | 409 |
| 180 | NM_001088 | AANAT | CCTATGTCCAGAGCTGTCCCTGGGCTGGTTCGAGGAGGGCTGCCTTGTGGCCTTCATCAT | 4 | 19 |
| 181 | NM_000200 | STATH | GAAGAAAATTCCATGAAAAGCATCATTCACATCGAGAATTTCCATTTTATGGGGACTATG | 2 | 458 |
| 182 | NM_005489 | SH2D3C | GCACAGTGGCACACCACGGAGGCCTGTACCACACCAATGCTGAAGTCAAGCTGCAGGGGT | 5 | 343 |
| 183 | NM_003664 | AP3B1 | TTTACTGTCAGTTGTCTCAACTCTTGAATCCATGTGGCGTTTTCTCTGTCCTGCTGCTTC | 4 | 350 |
| 184 | NM_005290 | GPR15 | CCCTTCAATACTTTCAAGTTCCTGGCCATTGTCTCTGGGTTGCGGCAAGAACACTATTTA | 2 | 47 |
| 185 | NM_006400 | DCTN2 | TGTTAACAGCTTACATAGGGTTTCCCCTTTACTATAACTCTAGCATCCCCATCCCATTTG | 5 | 158 |
| 186 | NM_003923 | FOXH1 | GCTGGCTGCTCTCCTGGTGCAGCCTGTGAGGCTCTTAAGACAGGGGCCGCTCCTCCCTCC | 4 | 321 |
| 187 | NM_003749 | IRS2 | GGTCTCAAGTACATCGCCATCGACGTGAGGGAGGAGCCCGGGCTGCCACCCCAGCCGCAG | 1 | 141 |
| 188 | NM_014299 | BRD4 | TCCATTGTTCCGTGCATTTCCAAAGCTTAAGTTGCTGGTGGGCATTTCCCCAGTTTCTAT | 1 | 66 |
| 189 | NM_004182 | UXT | CAGTGGTCCCAGATACTTCACGCATCTATGTGGCCCTGGGATATGGTTTTTTCCTGGAGT | 5 | 240 |
| 190 | NM_013370 | OKL38 | TGGTGTTCAACCAGCTGCCCAAGATGCTGTACCCCGAGTACCACAAGGTGCACCAGATGA | 2 | 267 |
| 191 | NM_017840 | MRPL16 | ATATAGGCTACTGAAAGAAGGATTCTGCATTTCTATTCCCCTCAGCCTACCCACTGAAGT | 5 | 311 |
| 192 | NM_003581 | NCK2 | CAGCTACAACGGGCAGATCGGCTGGTTCCCCTCCAACTACGTCTTGGAGGAGGTGGACGA | 4 | 275 |

EP 2 084 296 B1

| | Genbank accession number | Gene Symbol | Probe Sequence | Intensity group | STD order |
|---|---|---|---|---|---|
| 193 | NM_000482 | APOA4 | ACGTGGAAGGCCACTTGAGCTTCCTGGAGAAGGACCTGAGGGACAAGGTCAACTCCTTCT | 1 | 368 |
| 194 | NM_016362 | GHRL | ACAAGCCTTACTCACCTCTCTCTAAGTTTAGAAGCGCTCATCTGGCTTTTCGCTTGCTTC | 2 | 272 |
| 195 | NM_014387 | LAT | TACCCCCAGAACCAGCCTGTGAGGATGCAGATGAGGATGAGGACGACTATCACAACCCAG | 2 | 127 |
| 196 | NM_002248 | KGNN1 | GTTCCTCCAAGCCATCCATCAGGCTCAGAAGCTCCGGAGTGTGAAGATCGAGCAAGGGAA | 2 | 46 |
| 197 | NM_007375 | TARDBP | CCCATAAGAATGCTGTTTGCTGCAGTTCTGTGTCCTGTGCTTGGATGCTTTTTATAAGAG | 5 | 412 |
| 198 | NM_002991 | CCL24 | GCTCTGTGGTCATCCCCTCTCCCTGCTGCATGTTCTTTGTTTCCAAGAGAATTCCTGAGA | 5 | 36 |
| 199 | NM_004832 | GSTO1 | CTTCTTTGGTGGCAATTCTATCTCTATGATTGATTACCTCATCTGGCCCTGGTTTGAACG | 5 | 355 |
| 200 | NM_006191 | PA2G4 | TGAATTTGTTGCCCAGTTTAAATTTACAGTTCTGCTCATGCCCAATGGCCCCATGCGGAT | 5 | 289 |
| 201 | NM_005201 | CCR8 | CTACTGCTGCCTAGTTACCATGAACACGTTTTTTCACTATTAATGGTGCGTCATATTTTT | 1 | 187 |
| 202 | NM_005474 | HDAC5 | CATGACTTGACCGCCATCTGTGATGCCTCTGAGGCTTGTGTCTCGGCTCTGCTCAGTGTA | 4 | 313 |
| 203 | NM_020129 | LGALS14 | TACACTGGGATGGATGAGGACTCAGATATTGCTTTCCAATTCCGACTGCACTTTGGTCAT | 1 | 349 |
| 204 | NM_001619 | ADRBK1 | GTGCCTGATTCGGCTGTCTCAGACTCTTTTTGTACCTGGTGACCCCTTTTCAGCTTCTGC | 2 | 17 |
| 205 | NM_006686 | ACTL7B | CTGGGGGTGACCTCACCAACTACCTGATGCAGCTGCTCAATGAGGCGGGCCACGCATTCA | 1 | 175 |
| 206 | NM_017883 | WDR13 | CCGGGATCCCTCACTGCTCATCAATGCTTGCCTCAACAAGTTGCTGCTCTACAGGGTGGT | 5 | 270 |
| 207 | NM_003040 | KCNH2 | CTCCGCATGGTGGTGCTCACCCGTATCTTCACCGACCGAGAGATGAAATGTCTGGATGCT | 4 | 144 |
| 208 | NM_007351 | MMRN1 | GCTTGCATTTGAGTCTGAAAATATTAACAGTGAAATACACTGTGATAGGGTTTTAACTGG | 1 | 460 |
| 209 | NM_002191 | INHA | CACCATCATCAGCTGGGAGGAAAGGCAGAGTTGGGAAATAGATGGCTCCCACTCCTCCCT | 2 | 357 |
| 210 | NM_005912 | MC4R | TTCTATGCTCTCCAGTACCATAACATTATGACAGTTAAGCGGGTTGGGATCATCATAAGT | 1 | 51 |
| 211 | NM_001503 | GPLD1 | CACCATCTCTTGCCAGGACATCTACTGTAACTTGGGCTGGACTCTCTTGGCTGCAGATGT | 2 | 4 |
| 212 | NM_015936 | | TGGACGAGAAGGATTGGATTCTGCTAAAAGGTGTACACAAGCCCTTTATCACAGTAGCAT | 4 | 401 |
| 213 | NM_019612 | IRGC | CATCCCTGTGTTTGGGACGCTGGTGGCTGGCGGCATCAGCTTTGGCGCTGTCTACACCAT | 3 | 137 |
| 214 | NM_001643 | APOA2 | CCACTGGCCAGTCCTAGAGCTCCTGTCCCTACCCACTCTTTGCTACAATAAATGCTGAAT | 5 | 255 |
| 215 | NM_006858 | TMED1 | TACTGTAAATGTGCCTTAGCCTAAGCCTCCCATCCTGTGTTAGCGTTGCCTGGTGCGGGG | 5 | 84 |
| 216 | NM_018687 | LOC55908 | CTGAATCTGCCTGGATGGAACTGAGGACCAATCATGGTGCAAGGAACACTTCCACGCCCC | 1 | 370 |

EP 2 084 296 B1

| | Genbank accession number | Gene Symbol | Probe Sequence | Intensity group | STD order |
|---|---|---|---|---|---|
| 217 | NM_021049 | MAGEA10 | CTCCCCTGACCCAGAGTCTGTGTTCCGAGCAGCACTCAGTAAGAAGGTGGCTGACTTGAT | 4 | 89 |
| 218 | NM_005029 | PITX3 | GACCAGCTCCCCGGGGGCCAACTCACCCTTGGCCCATCCCGCCTTCTCCAGGCTTCCCCT | 5 | 279 |
| 219 | NM_005759 | ABI2 | TTGGTATGAGGGAGTTATGAATGGAGTGACTGGGCTTTTTCCTGGGAATTACGTTGAGTC | 3 | 282 |
| 220 | NM_003198 | TCEB3 | TGGCCAAGACAATTAAAGCTTTCAAGAACAGATTCTCCCGACGATAAACTGAGGACTTGC | 3 | 333 |
| 221 | NM_004979 | KCND1 | GCAAGAGCTGGGCTGTATTTGGAGATCATGGGCTGATfCCATGTTCTTGGGCAACAGTCC | 4 | 130 |
| 222 | NM_000245 | MET | AAAGCAACAGTCCACACTTTGTCCAATGGTTTTTTTCACTGCCTGACCTTTAAAAGGCCAT | 1 | 22 |
| 223 | NM_018381 | PPAN | GCATATTCTCTCAGGAAGGCCGCCATGCTCATTCGGCCCCTGAAGCTGTGACTCTGTGTT | 1 | 69 |
| 224 | NM_000040 | APOC3 | TTAAGGACAAGTTCTCTGAGTTCTGGGATTTGGACCCTGAGGTCAGACCAACTTCAGCCG | 1 | 309 |
| 225 | NM_003631 | PARG | TGGTGCTTGAGGCATATTCATATAACCAAAGTTTGAGAACTGGGAACTTCATGCTGATTT | 3 | 440 |
| 226 | NM_001487 | BLOC1S1 | AAGGAAATTGGGGATGTGGAGAACTGGGCTCGGAGCATCGAGCTGGACATGCGCACCATT | 4 | 324 |
| 227 | NM_017820 | FLJ20433 | ATCCTTTCTGTGCTGCTTTAGGCATCTGCCCTTACGTGGTTCGTGTCCAGCTCTGTCAAC | 2 | 98 |
| 228 | NM_014593 | CXXC1 | CTGCTACGCCAAGTATGAGAGCCAGACGTCCTTTGGGTCCATGTACCCCACACGCATTGA | 3 | 301 |
| 229 | NM_014977 | ACIN1 | TACTTACAGCCTTCTCTTGGGAACAGCCGGGGCCAGGACTGGGTCACCTATGAGCTGAAT | 4 | 291 |
| 230 | NM_014847 | UBAP2L | GTGTATAAATTTGCACTGAAGTCTTGTTTCAGAAACCAGACCACTGAGGAGAGCCTGCTG | 3 | 395 |
| 231 | NM_005011 | NRF1 | GGAATTGCATTTTTTAAAGCACCACTCTTGATTTTCTGGGATTGGTGAAGAAACTGCATT | 1 | 290 |
| 232 | NM_018111 | FLJ10490 | CTGTTCTCAGACTCTGGAAAAGGCTTCTCAACGGCTTGGCCTGACCTCAGCTCCTCGCAT | 2 | 185 |
| 233 | NM 007278 | GABARAP | TCAGCTGTACCAGGAACACCATGAAGAAGACTTCTTTCTCTACATTGCCTACAGTGACGA | 5 | 214 |
| 234 | NM_000606 | C8G | TACTTCCCCAAGTACGGCTTCTGCGAGGCTGCAGACCAGTTCCACGTCCTGGACGAAGTG | 2 | 438 |
| 235 | NM_006481 | TCF2 | GGGTGCATGTGAGGATGAAAGGAGTGAATGTATAAAGACACCTTTCCCGATAACCCATAC | 2 | 415 |
| 236 | NM_004554 | NFATC4 | CTGCTTGCGAAACTCCTTACCTATCAGAAGGCTTCGGCTATGGCATGCCCCCTCTGTACC | 2 | 237 |
| 237 | NM_006463 | STAMBP | TGTTTATATTTACCTCTGGGCTCAATAAGGGCATCTGTGCAGAAATTTGGAAGCCATTTA | 4 | 362 |
| 238 | NM_003952 | RPS6KB2 | AAACTCTACCTCATCCTTGAGTGCCTCAGTGGTGGCGAGCTCTTCACGCATCTGGAGCGA | 2 | 253 |
| 239 | NM_005164 | ABCD2 | TGTCAGCATTGATGTCGAAGGAAAGATATTTCAGGCTGCAAAAGGGGCTGGAATTTCCTT | 1 | 366 |
| 240 | NM_017749 | RPS10 | GGCTTCGGCAACAACATCATCGTCAGCCACCGCATTCACCGCAGCTCTCAGACGGGCACT | 2 | 75 |

EP 2 084 296 B1

(continued)

| | Genbank accession number | Gene Symbol | Probe Sequence | Intensity group | STD order |
|---|---|---|---|---|---|
| 241 | NM_014482 | BMP10 | TTGGTGTCTGGGGAGATATATGGAACCAACAGTGAGTGGGAGACTTTTGATGTCACAGAT | 1 | 264 |
| 242 | NM_006543 | | TCTGCAGGTTGCTCTGCACTTGCAACATAAGCCCAACCACATCAATTGCTGCAAAACAAA | 1 | 444 |
| 243 | NM_001169 | AQP8 | CGAACGGTTTGTGCAGCCATGTCTGGTCGAACTGCTGGGCTCTGCTCTCTTCATCTTCAT | 2 | 180 |
| 244 | NM_017986 | GPR172B | ACTGCAGGGGTGGTCCTTGTGGTGCTGTCGTGGGTGCTGTGTCTGTGTGTGTTCTCATAT | 5 | 93 |
| 245 | NM_005439 | MLF2 | TCCTACTCCTGCCATGCATTGAAGGGTCAATGCATTTTGGGGTGAGCTCTGGGTTTAGGG | 4 | 197 |
| 246 | NM_003227 | TFR2 | ACAGCAGTGCCTATTCCTTCACGGCCTTTGTGGGAGTCCCTGCCGTCGAGTTCTCCTTTA | 2 | 396 |
| 247 | NM_012320 | LYPLA3 | CAGGACTGAAGCTGCCTCCCTTCACCCTGGGACTGTGGTTCCAAGGATGAGAGCAGGGGT | 5 | 12 |
| 248 | NM_015879 | ST8SIA3 | TGAAGCCACATGGTTTAGACTTGATTGATAAAGGGAATGTTGCATTTGGGACTATGCTGC | 3 | 427 |
| 249 | NM_016151 | TAOK2 | GTCACTCTGTGTTCTCCTGGCGCTCCTCCCCTAAGTTATTGCTGTTCGCCCGCTGTGTGT | 5 | 119 |
| 250 | NM_004193 | GBF1 | TTCCTCTTTTACTAATTAGTTGGTCAGTTTGGAGAGTTGACTGGCACCATGGAGGGTAGG | 4 | 171 |
| 251 | NM_000721 | CACNA1E | CCCCCCTCCGATGCATGCTCTTCTCTCACATGGAGAAAACCAAGACAGAATTGGGAAGCC | 5 | 424 |
| 252 | NM_003322 | TULP1 | ACCGTCATCATTCCTGGCATGAGTGCGGAGAACGAGAGGGTCCCCATCCGGCCCCGAAAT | 3 | 374 |
| 253 | NM_016327 | UPB1 | AAGTGTGGCAGGCTTAACATGTCCAGGTTCTCCCCAATAACATTGTCCAGGTTGGTTTTA | 3 | 50 |
| 254 | NM_000678 | ADRA1D | TGCTGGTTCCCTTTCTTCTTTGTCCTGCCGCTCGGCTCCTTGTTCCCGCAGCTGAAGCCA | 5 | 117 |
| 255 | NM_016284 | CNOT1 | ATTAATTAATTGTTCTCCCCCATTACCCCACTGAATGAATGGCCATACAGGCTAAGCTGA | 4 | 33 |
| 256 | NM_000564 | IL5RA | AGGATTCTGTGTTTTGACTGTCACTTTGGCATCCTCTGATGAACTCACACATGCCTCAGT | 1 | 150 |
| 257 | NM_016451 | COPB1 | AAGCTTTACAGTTAATTTAGGTATGGGCTTACTGGACTCCAACATCTTTTGTACTCTTTC | 5 | 456 |
| 258 | NM_005171 | ATF1 | ATCAGGAGATATGCAAACATATCAGATCCGAACTACACCTTCAGCTACTTCTCTGCCACA | 2 | 281 |
| 259 | NM_004687 | MTMR4 | GTGAATTCTGGTTGGCCAAACGAAGACACCATTGCAGAAATTGTGGGAATGTATTTTGTG | 1 | 443 |
| 260 | NM_003420 | ZNF35 | GGGGTAGCAGTTCAACAATTCACTTACGAATGTTTATAAGCTTTCCATTTCCTAGGTAAT | 2 | 299 |
| 261 | NM_014341 | MTCH1 | ACTACTCAGAATGTGTCCTCCTCATCTAATGCTCATCTGTTTAATGGTGATGCCTCGCGT | 5 | 394 |
| 262 | NM_021232 | PRODH2 | TGCCAGGATGCCGAAGGATACCCCACTAGCACCCCTGAGGGGGTCATGTGGTCAATAAAA | 1 | 364 |
| 263 | NM_000915 | OXT | GCTGCGCGGAAGAGCTGGGCTGCTTCGTGGGCACCGCCGAAGCGCTGCGCTGCCAGGAGG | 1 | 101 |
| 264 | NM_003921 | BCL10 | TGAGAATAGACCCTTACTAGGAAGAACGTTTTTTCCTCAGTGCATTTGTGCTAGAAATTT | 4 | 360 |

| | Genbank accession number | Gene Symbol | Probe Sequence | Intensity group | STD order |
|---|---|---|---|---|---|
| 265 | N_000511 | FUT2 | GATGTGGTGTTTGCTGGCGATGGCATTGAGGGCTCACCTGCCAAAGATTTTGCTCTACTC | 2 | 85 |
| 266 | NM_002044 | GALK2 | AAAGTTTGTTTGCTACCAAACCTGGAGGTGGGGCTTTGGTTTTGCTTGAGGCCTGAAAAA | 4 | 320 |
| 267 | NM_007254 | PNKP | GGGGCGGAAGAAGAAAGACTTCTCCTGCGCCGATCGCCTGTTTGCCCTCAACCTTGGCCT | 3 | 215 |
| 268 | NM_002802 | PSMC1 | GGAGTTGCCCAGAGGAATCCCTGTTCCCACTGATTTTTATTAGCAAAACATCCTGTGTCT | 5 | 373 |
| 269 | NM_015607 | C1orf77 | GCGTGTTGGGTTGAATTGCACTTTCTACCTTTGTATGAGATTTACAGACTTTCCTTCTGG | 4 | 316 |
| 270 | NM_016464 | TMEM138 | GCAAGGAGTTCATGCAAGTTCGAAGGTGACCTCTTGTCACACTGATGGATACTTTTCCTT | 3 | 423 |
| 271 | NM_002602 | ARL16 | TGGAATGGAAGGCCTGGGAACAGACATCACAGTCATCTGCCCTTGGGAGGCCTTCAACCA | 4 | 28 |
| 272 | NM_018316 | KLHL26 | TCCTTAGGGGCTTGGGACCTTCCATTTGGCACTGAGCATCTTGTGGGGCCTTAACTGGCT | 2 | 16 |
| 273 | NM_021176 | G6PC2 | AAATAACTACACACTGAGCTTCCGGTTGCTCTGTGCCTTGACCTCATTGACAATACTGCA | 1 | 29 |
| 274 | NM_018113 | LMBR1L | AGAGTTTGGGACCAGGACCTCCTGCTTTTCCATACTTAACTGTGGCCTCAGCATGGGGTA | 3 | 216 |
| 275 | NM_013289 | KIR3DL1 | GCCACAAATCTGGTGCCTCTCTCTTGCTTACAAATGTCTAGGTCCCCACTGCCTGCTGGA | 5 | 3 |
| 276 | NM_018081 | WDR79 | TGTGTTTCCTGAGCCCACAGAGAGTGGGGACGAAGGAGAGGAGCTGGGCCTTCCCTTGCT | 3 | 251 |
| 277 | NM_506663 | PPP1R13L | AGTCACTGCTGACACCATCTCTCCCAGCAGTCTTGGGGTCTGGGTGGGAAACATTGGTCT | 4 | 302 |
| 278 | NM_020131 | UBQLN4 | TGAGTAAAAGGCTTGGAAGTTGGAATTAGCAGTGGGGAGCAGAAGCACTCATAGCTCTTT | 2 | 365 |
| 279 | NM_012278 | ITGB1BP2 | ATTGAGCAGCAGGAGGCTGAAGGAGGGGAGAACAAAATTGTCCAAACCATGCTGTTTTTT | 2 | 262 |
| 280 | NM_013365 | GGA1 | TCTGTCCATCCATCTGTCCGTGGTCAGAAGTGGGGTCAGTGTGTGAGTGAGAGCAGGAGT | 5 | 238 |
| 281 | NM_003461 | ZYX | GAAGCCCCTGTCGATTGAGGCAGATGACAATGGCTGCTTCCCCCTGGACGGTCACGTGCT | 4 | 206 |
| 282 | NM_003347 | UBE2L3 | GCACCGGGCTGGCGTTTCCACATCTGTCTTCATTAGCAGAAAAGTGATGATGGATTTTAT | 5 | 254 |
| 283 | NM_001923 | DDB1 | GTGTATGTGTATCTCACACTCATGCATTGTCCTCTTTTTATTTAGATTGGCAGTGTAGGG | 5 | 205 |
| 284 | NM_018654 | GPRC5D | TGAGAACTGGAAGCAGCATGGAAGGCTCATCTTTATCACTGTGCTCTTCTCCATCATCAT | 1 | 11 |
| 285 | NM_014671 | UBE3C | ACTTGATTCATTGTCCTATTTCTATCTCCACTTTGTGCCTGGAGAGCTTTCAGGGGAGGT | 4 | 151 |
| 286 | NM_002365 | MAGEB3 | GTGATCTGTTGCAAGATAACTTGGAATTAGAATAAGCATTTCCTTGAAAATGTTTAAAAA | 1 | 465 |
| 287 | NM_016465 | | TGCCTGCATTGTATCTCCATCTGGTCACTGCAGGTGCCAACCCTTCATCCCCCATGTTTT | 3 | 56 |
| 288 | NM_004718 | COX7A2L | TAGGAAGATATGAAGATGATGTTTTGGTTTGTTTATGAAATGCATATGGCTTGTCAGAGC | 4 | 392 |

EP 2 084 296 B1

(continued)

| | Genbank accession number | Gene Symbol | Probe Sequence | Intensity group | STD order |
|---|---|---|---|---|---|
| 289 | NM_005095 | ZMYM4 | TAGTCCCCACTATACGAATTTATGGTTTGTATAAACACTAACATTTTCCCCTTCTGTAG | 2 | 437 |
| 290 | NM_004604 | STX4 | ACACTGGCCCCTATGCAGAAGGGCAGACAGTTCTTCTGGGGTTGGCAGCTGCTCATTCAT | 5 | 211 |
| 291 | NM_006012 | CLPP | TTGCTGGGCTTGGAGGGGCCTCTTGAGGAACTTTAATTTGCAGGGGTGCCCGCTATGGA | 5 | 315 |
| 292 | NM_001704 | BAI3 | ACAGTGTGACTATCTTATGTCAGGACCTTCATGTGCCAAACGTCAGTGGTGTTTTCATAT | 1 | 451 |
| 293 | NM_004157 | PRKAR2A | CTGCATGGACATCATGAAGAGGAACATCTCACACTATGAGGAACAGCTGGTGAAGATGTT | 1 | 346 |
| 294 | NM_017925 | DENND4C | ATATTGAAGATTTTCAACCCCTGAACTGCTTTTCTGCCTCTGTGGAAAACTACTTTGGGA | 4 | 418 |
| 295 | NM_004868 | | GATCGGTTTCGCCATCATGACGCAGTGTCTCCCAGTGGCCCTGTTCTCCCTGGTGGGCTT | 5 | 271 |
| 296 | NM_004966 | HNRPF | ATGGGTGGCTATGACTAGTTTTGTTAGGAACATTTGAGTTACTTCAATCATTTCACAGG | 5 | 202 |
| 297 | NM_016239 | MYO15A | GAAAAGCCTTCTTGGAAAATGGGACATTAGCATTGAGTTTTGAAAGATGAGTAGGAGTTT | 1 | 457 |
| 298 | NM_005373 | MPL | GCTTTATGCAACTAACTGTTTACATATCTGTCCCCTGCTACTAGATTGTGAGCTCCTTGA | 4 | 105 |
| 299 | NM_000253 | MTTP | AGCTCCATTCAGGCAATTTGAGAAAAAGTACGAAAGGCTGTCCACAGGCAGAGGTTATGT | 1 | 145 |
| 300 | NM_020394 | ZNF695 | AGAAAAACCTTCCGATGTGAAGAATGTGGAAAGGCCTTAACCAGAGCTCACATCTGACTG | 1 | 416 |
| 301 | NM_001296 | CCBP2 | TCGGGAACTGTGAGGTCAGCCAGCATCTAGACTACGCCACTCCAGGTAACAGAGAGCATCG | 1 | 193 |
| 302 | NM_017624 | | AGGAAGAGACTATGCTGTTCACTCTGATGACCCCAGGACGCAGGAGTGTTCTCCCTGCGT | 2 | 124 |
| 303 | NM_014801 | PCNXL2 | TTAGAACCTGACCTCAAGGATATGGCAGCGCTAGCCTTTAGCTCCCACAGCACGGATGGG | 1 | 448 |
| 304 | NM_016501 | | TGTGATGCTGTGTCTGTATATTCTATACAAAGGTACTTGTCCTTTCCCTTTGTAAACTAC | 5 | 165 |
| 305 | NM_018035 | FLJ10241 | AATGGATTTCCAGTCAATTCAGAAGCI4TTTACCAGTGAAGCCCTCATTATTCCAGTTCA | 5 | 223 |
| 306 | NM_000185 | SERPIND1 | CCCTCATCTGAATACCAAGCACAGAAATGAGTGGTGTGACTAATTCCTTACCTCTCCCAA | 1 | 247 |
| 307 | NM_004489 | GPS2 | TGCAGTGCAGTACCTATCTCAGCCACAGCCTATGCTGTGCATGGCCACTTTCA | 3 | 166 |
| 308 | NM_006445 | PRPF8 | CCAGAACACAGACAAGGGCAACAACCCCAAGGGCTACCTGCCTTCACACTATGAGAGGGT | 5 | 329 |
| 309 | NM_005477 | HCN4 | GAGTGGCCAGTGTTGGCGGTTCTTAGAGCAGATGTGTCATTGTGTTCATTTAGAGAAACA | 2 | 31 |
| 310 | NM_005858 | AKAP8 | CTTCTTGCCCAGGTTTCAAAGTCGAAGTACATTGTCCTTAGCGGCTGTAACATGTCTCTT | 4 | 353 |
| 311 | NM_006196 | PCBP1 | CCCTTTCTGCTGTTCTCCCATGATCCAACTGTGTAATTTCTGGTCAGTGATTCCAGGTTT | 5 | 126 |
| 312 | NM_000119 | EPB42 | ATAATAACCATCGGCCTGTTCTTCTCCAATTTTGAGCGAAACCCACCCGAGAACACCTTC | 2 | 25 |

| | Genbank accession number | Gene Symbol | Probe Sequence | Intensity group | STD order |
|---|---|---|---|---|---|
| 313 | NM_014139 | SCN11A | AAGAGAAGTTCATGGAAGCCAATCCTCTCAAGAAGTTGTATGAACCCATAGTCACCACCA | 1 | 287 |
| 314 | NM_006093 | PRG3 | CTCAGGGGCTGGTTCCTGTGGAAGCGGTTTTGCTGGACTGATGGGAGCCACTGGAATTTT | 2 | 110 |
| 315 | NM_003476 | CSRP3 | TATGTGTTTCTCCTCAGAAGTGATCAGGTCTTTACTGAATGTTAGAAGAGGCCTTTGGAA | 1 | 429 |
| 316 | NM_018356 | C5orf22 | CTTCCTGAGTCACTCTTAATCATGAAACTTGATTTTCTCAATTGGCCAGTCTTCTGATCT | 1 | 266 |
| 317 | NM_005379 | MYO1A | CTCCTGTGTGGGAGGATCTCTAACCCCTCTGATCGTGGCGCATGGCTTGGGGATTAAACT | 3 | 23 |
| 318 | NM_019055 | ROBO4 | AGTGGCTGTGGATAGCTTTGGTTTCGGTCTAGAGCCCAGGGAGGCAGACTGCGTCTTCAT | 3 | 203 |
| 319 | NM_005199 | CHRNG | CTTTGACAATGGGAATGAGGAGTGGTTCCTGGTGGGCCGAGTGCTGGACCGCGTCTGCTT | 5 | 441 |
| 320 | NM_002005 | FES | GTGTCCTCTCTGTGTCCCTGCTGCTGCCAGGGCTTCCTCTTCCGGGCAGAAACAATAAAA | 5 | 52 |
| 321 | NM_002967 | SAFB | GGGGCATGATGGACAGGGATCACAAGAGGTGGCAAGGTGGCGAGAGAAGCATGTCCGGTC | 5 | 176 |
| 322 | NM_001868 | CPA1 | GGAAGCACTATTGACTGGACCTACAGCCAGGGCATCAAGTACTCCTTCACCTTCGAGCTC | 2 | 106 |
| 323 | NM_015340 | LARS2 | CATGTGGTCCCCCTGCAGTTCAGCAGTTAACAGATGACTTTTTTAATAAAATGTT | 5 | 71 |
| 324 | NM_000229 | LCAT | CCCTGCACGGGATACAGCATCTCAACATGGTCTTCAGCAACCTGACCCTGGAGCACATCA | 2 | 263 |
| 325 | NM_002779 | PSD | CGGAAGCCCTGAGATGAGGTTTAGGGTGGGGAGTGCCTGCTGGGCACCTGAAGGATGACA | 3 | 155 |
| 326 | NM_005285 | NPBWR1 | ACCACCTGAGCACCGTGGTGGCGCTCACCACCGACCTCCCGCAGACGCCGCTGGTCATCG | 5 | 433 |
| 327 | NM_006105 | RAPGEF3 | AGACATGACCTTCATTCATGAGGGAAACCACACACTAGTGGAGAATCTCATCAACTTTGA | 1 | 449 |
| 328 | NM_007242 | DDX19B | AAACGTTATCAAACTGAAGCGTGAGGAAGAGACCCTGGACACCATCAAGCAGTACTATGT | 3 | 224 |
| 329 | NM_012418 | FSCN2 | ATGAGCTCTTTGATCTGGAGGAGAGTCACCCACAGGTGGTGCTGGTGGCTGCCAACCACC | 1 | 186 |
| 330 | NM_001291 | CLK2 | ACCTGTGAATATGTGAAATAGTGTAAATATGAAAGAACTTGTACCTATCACTTCAACCCC | 4 | 381 |
| 331 | NM_013359 | ZNF221 | AGTGGAGAAAGCCATTGAAATCTGGAGTGTGGGAAGAGATCTACTCAGAATTCACAGCT | 2 | 435 |
| 332 | NM_014706 | SART3 | TTTAGACAGAAAGGGGAAGGGGTTCTAAGTCAAGAGCCTTTCAGTGCTCCCTCATATTGA | 3 | 111 |
| 333 | NM_006789 | APOBEC2 | ACAGCCTCAGACCCGAGGTTTAGATTTCTGAAATATGCATTTTATGTTAAGTTGGGTATT | 1 | 139 |
| 334 | NM_000718 | CACNA1B | CACCTACAAGACGGCCAACTCCTCACCCATCCACTTCGCCGGGGGCTCAGACCAGCCTCCC | 4 | 61 |
| 335 | NM_016630 | SPG21 | AGGCGGCATCTCCACTAAGCCTGTGTAACTGTTCCCTCTTTGGTTTTCTTAGCTTTTGAA | 5 | 147 |
| 336 | NM_013344 | | GTGGTAAATGTGATTTGGAAAACCAAAGAGACATGATGGTGGAGAATAAGGAGAGTTGTC | 1 | 461 |

(continued)

| | Genbank accession number | Gene Symbol | Probe Sequence | Intensity group | STD order |
|---|---|---|---|---|---|
| 337 | NM_003833 | MATN4 | TTGAGCTGAGTTGGCCTGCGCCCGGACCATTAGGCGGACTGCGGCGGTCAGGGGGGATAGCGG | 3 | 372 |
| 338 | NM_018263 | ASXL2 | TAGGAATACTCCTAAAACAGATATTGCCAACTAGATACACTGCCTCAATCTTGACCAGAC | 4 | 391 |
| 339 | NM_000513 | OPN1MW | GGTCTCTGGTCTCTGGCCATCATTTCCTGGGAGAGATGGATGGTGTCTGCAAGCCCTTT | 3 | 73 |
| 340 | NM_015848 | KRT8 | AAGATCTCCAGACTGCTGGTTCCCAGGGAACCCTCCCTACATCTGGGCTTCAGATCCTGA | 2 | 79 |
| 341 | NM_005626 | SFRS4 | GAATTGATGCCCTTCGATGTATGCCATTTAGTGAAAGTGCTAAGTCTTAAGTTTCCTACC | 4 | 226 |
| 342 | NM_002696 | POLR2G | TGTGGACAAGAAATGACTTTTGCTATTGGCTCCCTGATGGACGATTACTTGGGGCTTGT | 5 | 345 |
| 343 | NM_004767 | GPR37L1 | CCTCTGCTGCCAATGGGTCGGGACAACAAGCTCAAGACCGAGGTGTCCTCTTCCATCTACT | 4 | 385 |
| 344 | NM_005934 | MLLT1 | TACACCGGAGGCTGATGGCCGCTGCGGGAAGATGATGGTTCAGCCACTGGTACTACGAGAATCTGA | 1 | 194 |
| 345 | NM_006308 | HSPB3 | TTCCTGTTCAGATGACATGGGGAAGATGATGGTTCAGCCACTGGTACTACGAGAATGTTT | 1 | 172 |
| 346 | NM_005886 | KATNB1 | AGCCCTGAACTCTTGAGACAACTCTCTCCAGCAATAGCTGCCCAGCTTTGCCCAACTGTT | 4 | 288 |
| 347 | NM_014481 | APEX2 | TTACACCACTTTCCACCTTCCTGTCCGAAGTACACGGACACTAGCTGCCCCAGGAAGTTG | 4 | 115 |
| 348 | NM_001538 | HSF4 | CTTTAACCATTTATAGCACTCCTGAGAGCCGGACTGCCTCCTACTTGGGCCCGGAAGCCA | 2 | 192 |
| 349 | NM_004173 | SLC7A4 | TCTGCCTCATGCTGAAACTTAGCTATCTGACCTGGGTGCGTTCTCCATCTGGCTGCTGA | 3 | 199 |
| 350 | NM_003585 | DOC2B | GGCCGAAAGTCTGCCAGAGTTCCCGGAGGCTCCTGATGATGGGTAAATTGGCACATGCTT | 1 | 384 |
| 351 | NM_006687 | ACTL7A | TGGGTCCACCGCTTTGAGTACGGAGGAACACGGGCCTTTCTTCCTACAGAAGGTGCTTC | 3 | 428 |
| 352 | NM_001976 | ENO3 | CTCGGAGCGTCTGGCCAAATACAACCAACTCATGAGGATCGAGGAGGCTCTTGGGGACAA | 3 | 386 |
| 353 | NM_000212 | ITGB3 | TGGCCTGTTCTTCTCATGGGTTGGACAACCTCATTTTAACTCAGTCTTTAATCTGAGAGGC | 2 | 406 |
| 354 | NM_018060 | MED31 | ATTCTAAGGCTAGCTGTTCCTGACATATAGTAGGGCAAGAACCATCTCCTGGAACCTTACT | 1 | 135 |
| 355 | NM_004409 | DMPK | TTTTGGATGCACTGAGACCCGACATTCCTCGGTATTTATTGTCTGTCCCCACCTAGGAC | 4 | 146 |
| 356 | NM_004707 | ATG12 | TGAAAACAAAGAAGAGTGGGCAGTAGAGCGAACCATCCAAGGACTCATTGACTTCA | 3 | 383 |
| 357 | NM_000069 | CACNA1S | TGGAGTCCTCCATGCCTGAGGACAGAGAAAGAGCTCCACACCAGGGTCTCTTCATGAGGAGA | 1 | 41 |
| 358 | NM_006442 | DRAP1 | AGAAGATTACGACTCCTAGCCGCCTTCTGCCCCCCAGACCCATAGCCCCTTTTAGTTGGTTT | 5 | 78 |
| 359 | NM_003984 | SLC13A2 | AGCACAGGCTGCTGGGCCCCATGACCTTTGCAGAAAAGGCCATCAGCATCCTATTCGTCA | 3 | 236 |
| 360 | NM_014404 | CACNG5 | CTTCCTCATCCCAGAATGTGTCACTGTTCACTTTCTGGGTCTCCTCCGGCCAGGATGT | 2 | 1 |

29

| | Genbank accession number | Gene Symbol | Probe Sequence | Intensity group | STD order |
|---|---|---|---|---|---|
| 361 | NM_015910 | LOC51057 | CCTAGCTGTTGACGTTGGTGCTCGTGACCTCTTTATGGATATTCATTACCTTGCACTAGA | 2 | 57 |
| 362 | NM_003963 | TM4SF5 | CTCCTGCCTGGAGATAGTACTGTGTGGGATCCAGCTGGTGAACGCGACCATTGGTGTCTT | 2 | 143 |
| 363 | NM_000512 | GALNS | AACTGGGCGGTCATGAACTGGGCACCTCCGGGCTGTGAAAAGTTAGGGAAGTGTCTGACA | 3 | 184 |
| 364 | NM_000162 | GCK | CTGGGCGGTGGCCTGTAAGAAGGCCTGTATGCTGGGCCAGTGAGAGCAGTGGCCGCAAGC | 1 | 318 |
| 365 | NM_016539 | SIRT6 | TGTGGATTCTTTTTCTCTCGTGGTCTCACTTTGTTACTTGTTTCTGTCCCCGGGAGCCTC | 3 | 8 |
| 366 | NM_000749 | CHRNB3 | ACCGAATCTTCCTGTGGCTCTTTCTGATAGTGTCAGTAACAGGCTCGGTTCTGATTTTTA | 3 | 382 |
| 367 | NM_000116 | TAZ | TTCTGGATTCTTGGCCCGCACAGAGCTGGGGCTGAGGGATGGACTGATGCTTTTAGCTCA | 2 | 204 |
| 368 | NM_018606 | | AAAGACCTCAAGCTCATTCTGGCCAGGATTAAATCCAAGAGCCTTCTTAGCCCAGTTTCT | 2 | 97 |
| 369 | NM_018627 | | ATTAGTGTTGAAGCCGAATGTTATGGTTTTTATTGCGGAGCTTTTTTGGTGGTTCGAGAA | 1 | 399 |
| 370 | NM_002070 | GNAI2 | CTCCCTGTTTGAAGCCTGCCCTTGTCTGAGATGCTGGTAATGGCCATGGTACCCCCTTCT | 5 | 107 |
| 371 | NM_017438 | SETD4 | TTTTTCAATATTCAGTATAATGCAGTGTATTTCATCATATGCTGTATGGAGAGTGGGCAG | 1 | 453 |
| 372 | NM_017735 | TTC27 | AGCTGTACAAATGCTTTCTTCTGTTCGACTCAATTTACGGGGCTTGTTATCTAAAGCAAA | 4 | 408 |
| 373 | NM_003635 | NDST2 | GTATCCTTTTCCCACAGTTCTGGGACAAATAAAGGGGCTTCCTTTGGTACCCCACATAAT | 3 | 207 |
| 374 | NM_000461 | THRB | CGTGTGAAGGCTGCAAGGGTTTCTTTAGAAGAACCATTCAGAAAAATCTCCATCCATCCT | 1 | 273 |
| 375 | NM_002098 | GUCA1B | GAGCCTTCGACAAGAATGGGGACAACACCATCGACTTCCTGGAGTACGTGGCAGCTCTGA | 1 | 233 |
| 376 | NM_003718 | CDC2L5 | AGAGGCAGAGGCAGAGGGTTACCATACTGAGTATCTGTTTTTCCTCAGGCACATCATTTT | 4 | 351 |
| 377 | NM_015343 | GABARAP | CACTTGGAGTCTGGATGGACACATGGGCCAGGGGCTCTGAAGCAGCCTCACTCTTAACTT | 4 | 161 |
| 378 | NM_018484 | SLC22A11 | GGGATAAGCCTAACCTGCCTCACCATCTACAAGGCTGAACTCTTTCCAACGCCAGTGCGG | 2 | 63 |
| 379 | NM_018174 | MAP1S | CCCTGTCCAGAAAGTCCTCAACCCCCAAGACTGCCACTCGAGGCCCGTCGGGGTCAGCCA | 4 | 129 |
| 380 | NM_017637 | BNC2 | TCCCTTCACTTCAGTAGATTAGTCTCAGAATGGACACTACAAATGCCAGCTCTCACCAGA | 3 | 436 |
| 381 | NM_016172 | UBADC1 | TTTAGCATCTGACAGGTGTTTACAAAAAAGTGGTTGTCGCACTGGGAAGTGGAGTGATGG | 4 | 195 |
| 382 | NM_017854 | TMEM160 | AGCCTGCTCTGGGCGTGCGCCGTGGGCCTCTACATGGGGCAGCTGGAGCTGGACGTGGAG | 4 | 354 |
| 383 | NM_016320 | NUP98 | GGAGGGATTGATCTTCAGGGCTGTTTTTGTTCCTGCCTTTAGAGTTCCATGAACACCATA | 3 | 261 |
| 384 | NM_006028 | HTR3B | CTGAAGGAAGTCTGGTCGCAGCTTCAATCTATCAGCAACTACCTCCAAACTCAGGACCAG | 1 | 421 |

| | Genbank accession number | Gene Symbol | Probe Sequence | Intensity group | STD order |
|---|---|---|---|---|---|
| 385 | NM_006161 | NEUROG1 | TGCCAGCCCGCCTTGAGACCTGCATCTCCGACCTCGACTGCGCCAGCAGCAGCGGCAGTG | 5 | 308 |
| 386 | NM_002134 | HMOX2 | CTAGCTGCTGGACTCTTGGCCTGGTACTACATGTGAAGCACCCATCATGCCACACCGGTA | 5 | 113 |
| 387 | NM_018068 | PIWIL2 | GTGGTAGATCATACAATAACAAGCTGTGAGTGGGTGGATTTCTATCTTCTTGCCCATCAT | 3 | 274 |
| 388 | NM_012340 | NFATC2 | ACAGACAGCTGCCTGGTCTATGGCGGCCAGCAAATGATCCTCACGGGGCAGAACTTTACA | 1 | 231 |
| 389 | NM_017532 | | GCACCAGGGGGAGGATTTTCATCAAGCACCCACACCTCTTTAAGTTTGCAGCAGATCCTC | 4 | 295 |
| 390 | NM_000691 | ALDH3A1 | CGTGGGGAACAGCGGCATGGGATCCTACCATGGCAAGAAGAGCTTCGAGACTTTCTCTCA | 1 | 388 |
| 391 | NM_002938 | RNF4 | AGCTCTCATCATTGTGATGTGTAGCATGTCTGCCCTCTGACTGGACATCATTGCCATTAA | 4 | 86 |
| 392 | NM_002442 | MSI1 | GAACCATCCCGTCCTGTATCATATGTAAATACTGTGAGGTGATGTGCCCACCCCTCTCTA | 4 | 163 |
| 393 | NM_018158 | SLC4A1AP | AGGCAAACTTCCACCAACACTTTGTTCCAAATATCCTGAAGATGACCCAGACTACTGTGT | 4 | 393 |
| 394 | NM_018463 | ITFG2 | TCCACCCCATCTTAAGCTCTGTCTTCCGTGGCACAATTCCAAGTTCTTGACGTTAGTAAT | 3 | 156 |
| 395 | NM_018143 | KLHL11 | AATGCTAGGTTCTCTCAAGCGTGCCGATTAAAACTGTTACACCCGTTTCGTGAAGCTGAA | 1 | 227 |
| 396 | NM_007160 | OR2H2 | CAACCTCTCCTTCTTGGACCTCTGTTTCACCACGAGTTGTGTTCCCCAAATGCTGGCCAA | 4 | 26 |
| 397 | NM_004623 | TTC4 | ACCCCTAAAAAGATTGCCAATTTTCTTCATCTTTGCCATATGGAGGACTGTGACAGACTT | 4 | 276 |
| 398 | NM_017649 | CNNM2 | CCTTCCCACTAGAACATACTTTAACAGAAAACGAGTCGGACCTTCTAGCTGCACTCTGTA | 2 | 80 |
| 399 | NM_006656 | NEU3 | TGGGAGACTTTGTAGATGTTGGGCTATATGTTGGGGTGATGGTAGCTCCTGATGTAATTT | 2 | 177 |
| 400 | NM_006604 | RFPL3 | GAGTCGTAAGTATTAATTATTGCCACCATCCAACTCATTGAGTCTTATGGTTCACATCTT | 1 | 376 |
| 401 | NM_003028 | SHB | ACGTTCTGGGTCAGAACAGCCCTCCGTTCGACAGTGTCCCGGAAGTCATCCACTACTACA | 2 | 182 |
| 402 | NM_014830 | ZBTB39 | TGATCTGTGAGTACCTGTTTGTCTCCAGGCCAAACCTTTGGGCTTAAATATCTTTTTCCT | 3 | 91 |
| 403 | NM_002075 | GNB3 | GACTTCAACTGCAATGTCTGGGACTCCATGAAGTCTGAGCGTGTGGGCATCCTCTCTGGC | 2 | 133 |
| 404 | NM_001528 | HGFAC | GTACACCCTGTACTCGGTGTTCAACCCCAGCGACCACGACCTCGTCCTGATCCGGCTGAA | 2 | 294 |
| 405 | MN_005332 | HBZ | AAGTTCCTATCGGTCGTATCCTCTGTCCTGACCGAGAAGTACCGCTGAGCGCCGCCTCCG | 3 | 222 |
| 406 | NM_002082 | GRK6 | AGCTACTCCGAGCGCCGTTTACAGTTTTGCACAGTGATCTTCCCCATTGTCCACTCAAGT | 3 | 300 |
| 407 | NM_017797 | BTBD2 | GACCTTGCGTTCCTTTTGTTCCTGTCCGTTTATCAGGACACGGGCCCCACCTGTCACGTG | 3 | 95 |
| 408 | NM_000787 | DBH | ATTCCTGAGTAAACAGATATTTTCGCCCACCTAAAGGGAAGCCCTGACAACAACTATCAC | 1 | 168 |

| | Genbank accession number | Gene Symbol | Probe Sequence | Intensity group | STD order |
|---|---|---|---|---|---|
| 409 | NM_016605 | FAM53C | CACTTTAGCTGTTTTGACTATTTGAATTTTCACATATTGGGCTTACCCAGAGTGGAGCAC | 3 | 235 |
| 410 | NM_014071 | NCOA6 | TTTCATAAAGTGCAATGGGGAAAGCAGGACTGTTGAGCCCTTTTGGTGTTGCGAGTTGAA | 4 | 319 |
| 411 | NM_002453 | MTIF2 | GGGTGATATAAGTGCAAATGATGTTAACCTTGCTGAAACATTTGATGGTGTTATATATGG | 3 | 452 |
| 412 | NM_002215 | ITIH1 | ACTCATCGATGGTGCCTACACTGATTATATCGTCCCCGACATCTTCTGAGCCCTCTGGCC | 1 | 53 |
| 413 | NM_001273 | CHD4 | TGCTGAGTGACATGAAAGCTGATGTGACTCGACTCCCAGCTACCATTGCCCGAATTCCCC | 4 | 292 |
| 414 | NM_005155 | PPT2 | TTGGGGAATATCTGTGGCCTATGAGGCCCATCTCAGGTTTGGGGATCCCCCAGTCCCTAT | 2 | 402 |
| 415 | NM_013393 | FTSJ2 | CAGTGGGTCTGCTTTGGTTT TTTGCTGGAAATTTATATCAGTGTCTGGGCTCCCAAGAACA | 5 | 183 |
| 416 | NM_018053 | XKR8 | TGCTCTCAAGGGGCTGCTTTTCAACCAAGAGCCTTGTGAGCCTGGTCTGAGCCTTGCACA | 3 | 125 |
| 417 | NM_006340 | BAIAP2 | GCCTCTTGAGGGTACACGCCTCTGGTCACATGGCCATGGAGCCTTGGGTACCCCTGAGTT | 5 | 414 |
| 418 | NM_004263 | SEMA4F | GGTGGTTCTGCTTATTCTTCAAGTTTATCTGAATCTGTGGGGAGTGCATGATCCCCATGT | 2 | 152 |
| 419 | NM_004756 | NUMBL | AGGATGGAATTCAGGGACGGACCCAGCCTGGCTAAGGGAACCATTTCACTGCCGGACTTA | 1 | 196 |
| 420 | NM_015985 | ANGPT4 | GTCTACTACCACGCTCCCGACAACAAGTACAAGATGGACGGCATCCGCTGGCACTACTTC | 4 | 400 |
| 421 | NM_005120 | MED12 | AGCAACAGACAGCAGCTTTGGTCCGGCAACTTCAACAACAGCTCTCTAATACCCAGCCAC | 3 | 154 |
| 422 | NM_016166 | PIAS1 | AAACAGGAGCAGCACGGACACGGCATCCATCTTTGGCATCATACCAGACATTATTTCATT | 4 | 330 |
| 423 | NM_006289 | TLN1 | AACTGCTGGACCATGTACTGCTGACCCTGCAGAAGCCAAGCCCAGAACTGAAGCAGCAGT | 1 | 70 |
| 424 | NM_016247 | IMPG2 | CGTGATCATAGGCATCACTATTGCCTCCGTGGTTGGACTTCTTGTCATCTTTTCTGCTAT | 1 | 40 |
| 425 | NM_018403 | DCP1A | CATTCTCAGTGATAATGGAGGTTTCACTGTGAAGCATCACCAGCAGATCCTTGTTTTGAC | 5 | 379 |
| 426 | NM_014206 | C11orf10 | CTTAGTGGCCTCACTCTTCATGGGCTTTGGAGTCCTCTTCCTGCTGCTCTGGGTTGGCAT | 5 | 242 |
| 427 | NM_016329 | SFMBT1 | TGCTGGAAAGCACCCTTTTGGTTGTTTTCATTCTGTTCCCTCTCATTGTAGATTGAACTT | 3 | 132 |
| 428 | NM_014293 | NPTXR | AAATTGCTGCTTCTCCAACTCTTGCTTGGGGCCAAGCCCTGCACCAGTTGCTTCCCAGCT | 2 | 367 |
| 429 | NM_001524 | HCRT | TCGTTCTTCGGGCCCTGTCCTGGCCCAGGCCTCTGCCCTCTGCCCACCCAGCGTCAGCCC | 5 | 39 |
| 430 | NM_017727 | FLJ20254 | AGTTCTTCCATCTTTGGTGGGGACAGGGCCCAGCAGCATCTCAGCCTCCTACCCACAATT | 3 | 55 |
| 431 | NM_014339 | IL17RA | GATTTTAATCCCAGGCATCCCTCCTAACTTTTCTTTGTGCAGCGGTCTGGTTATCGTCTA | 4 | 123 |
| 432 | NM_014625 | NPHS2 | TCCACCCTGAATTCCTCACACCTAACCCTGATAGTTACCTAAAGTGACACTTAAATGTTT | 3 | 326 |

| | Genbank accession number | Gene Symbol | Probe Sequence | Intensity group | STD order |
|---|---|---|---|---|---|
| 433 | NM_019064 | SDK2 | CCCACAACTGACCCCTGCCTCCCTGTGCTGGAATTGACTTTCCTGAGCATGGACAAGGAA | 5 | 116 |
| 434 | NM_001057 | TACR2 | AAGGAAGATAAGCTCGAGCTGACTCCCACGACCTCCCTCTCCACGAGAGTCAACAGGTGT | 4 | 305 |
| 435 | NM_307126 | VCP | TGCGCCCACAGCCTGCTCCATTCTCCAGTCTGAACAGTTCAGCTACAGTCTGACTCTGGA | 5 | 138 |
| 436 | NM_016499 | MGC13379 | ACATGCTCCCTGTACGAGCTGTGCTATACCTGTCCCACATGAGCACGGAGAGCCTCATGT | 4 | 303 |
| 437 | NM_003593 | FOXN1 | ATCTTCATCCCAACAGCCCAGCAAGAAGGAGGAGACAGAGAGCTCCTCCCTGGGTTGTCT | 2 | 62 |
| 438 | NM_002712 | PPP1R7 | ATCTAACCGCATCCGGGCAATCGAAAATATCGACACCTTAACCAACCTGGAGAGTTTGTT | 5 | 232 |
| 439 | NM_015877 | | AAACCCTACATGTGTGAAAAGTGTAGGAAATGTTTTTCTTCCTTTACATCCCTGAAAAGA | 3 | 212 |
| 440 | NM_000595 | LTA | TCCCCACCAGTGGCATCTACTTCGTCTACTCCCAGGTGGTCTTCTCTGGGAAAGCCTACT | 2 | 14 |
| 441 | NM_003120 | SPI1 | AACGCCAAACGCACGAGTATTACCCCTATCTCAGCAGTGATGGGGAGAGCCATAGCGACC | 1 | 378 |
| 442 | NM_017659 | QPCTL | CATTCTCGCTGTGTTCCTGGCTGAATACCTGGGGCTCTAGCGTGCTTGGCCAATGACTGT | 3 | 35 |
| 443 | NM_007263 | COPE | AGGCCAAGGAGAACGACTTTGACAGGCTGGTGCTACAGTACGCTCCCAGCGCCTGAGGCT | 5 | 331 |
| 444 | NM_004890 | SPAG7 | CAAGAAAAAGTTTCAGCCAATGAACAAGATCGAGAGGAGCATACTACATGATGTGGTGGA | 3 | 389 |
| 445 | NM_001313 | CRMP1 | GTACTAGTGTGGTGTGTTTGCTTGGAAATTCCTTGCCCCACAGTTGTGTTCATGCTGAAT | 2 | 284 |
| 446 | NM_007110 | TEP1 | TCGGGAATAATGATACCCCTTGTGCTAGAGATGCAAAGCCTGAAGACACTGGTAGCTTTT | 3 | 431 |
| 447 | NM_000757 | CSF1 | TGCCCCGTTTTAACTCCGTTCCTTTGACTGACACAGGCCATGAGAGGCAGTCCGAGGGAT | 2 | 285 |
| 448 | NM_004738 | VAPB | ACAACTGTCATAGGCAGGGAAATTCTCAGTAGTGACAGTCAACTCTAGGTTACCTTTTTT | 4 | 348 |
| 449 | NM_003844 | TNFRSF10A | GATGGAAGAGAGACATGCAAAAGAGAAGATTCAGGACCTCTTGGTGGACTCTGGAAAGTT | 3 | 430 |
| 450 | NM_000294 | PHKG2 | AGCAGAACTCTCCAGAAGAAGGGTTTTGATCATTCCAGCTCCTCTGGGCTCTGGCCTCAG | 4 | 189 |
| 451 | NM_004213 | SLC28A1 | ACAGGAAGCAGTGGATCTCCGTCAGAGCTGAAGTCCTCACGACGTTTGCCCTCTGTGGAT | 2 | 76 |
| 452 | NM_019016 | KRT24 | AGTTGGTGGATGGCAAGGTTGTCTCGTCTCAAGTCAGCAGTATTTCTGAGGTGAAAGTTA | 2 | 60 |
| 453 | NM_015610 | WIPI2 | TTTTGTTTCTGATCTTGTTTTCCCTGTGGATATTGGAGGACAGCGAGGTTCTTTCTGATA | 4 | 170 |
| 454 | NM_014113 | | AGGAAAATCCCTGACCCCCTAAGAATGATCTCTCTCACTGTTCCAGACAGTCCATTTTAT | 4 | 268 |
| 455 | NM_000638 | VTN | TGACTACAGGATGGACTGGCTTGTGCCTGCCACCTGTGAACCCATCCAGAGTGTCTTCTT | 3 | 380 |
| 456 | NM_004062 | CDH16 | GGGAGTGCTCCAAATGTCAGGGTGTTTGCCCAATAATAAAGCCCCAGAGAACTGGGCTGG | 1 | 335 |

EP 2 084 296 B1

| | Genbank accession number | Gene Symbol | Probe Sequence | Intensity group | STD order |
|---|---|---|---|---|---|
| 4571 | NM_004921 | CLCA3 | AGCCCTAGGCCCGAAATGACTAGCAAATATCATTTTCTGTATGAATTGTGGAACATCACA | 3 | 339 |
| 4581 | NM_006262 | PRPH | CCTGACACTTGATGTGACCTATGTGCTTCCCTTTTCATGTCCCGATAAGAAGCCAATGAT | 3 | 259 |
| 459 | NM_016566 | | CTGCTGGTGGCTATGCTCGTCACTTGAACAAGGAAGGAGACTACACTCTCTGCAGTCTTG | 1 | 136 |
| 460 | NM_001258 | CDK3 | CATGAGGTGGTGACACTGTGGTATCGCGCCCCCGAGATTCTCTTGGGCAGCAAGTTCTAT | 3 | 229 |
| 461 | NM_001940 | ATN1 | CTGCCCCGTTGGTGTGATTATTTCATCTGTTAGATGTGGCTGTTTTGCGTAGCATCGTGT | 4 | 100 |
| 462 | NM_004070 | CLCNKA | CTTCAGCGGCGTCCTGTTCAGCATCGAGGTCATGTCTTCCCACTTCTCTGTCCGGGATTA | 4 | 131 |
| 463 | NM_007188 | KCNH2 | TCATCAGCCAGGAGCCCGTCCTGTTTGGGACGACCATCATGGAAAACATCCGCTTTGGGA | 1 | 220 |
| 464 | NM_002723 | PRB4 | CCACCCAGACCTGCCCAGGGACAACAGCCTCCCCAGTAATCTAGGATTCAATGACAGGAA | 5 | 32 |
| 465 | NM_002002 | FCER2 | GAGCTGTCCTGGAACCTGAACGGGCTTCAAGCAGATCTGAGCAGCTTCAAGTCCCAGGAA | 1 | 347 |
| 466 | NM_004930 | CAPZB | GCTGAACGAGATCTACTTTGGAAAAACAAAGGATATCGTCAATGGGCTGAGGTCTGTGCA | 4 | ND |
| 467 | NM_017777 | MKS1 | TCAGATGGGCATCACAGCAAGGCAGGCTTTTTGATCTGAAGCTGTTTGGGGTGACCCCAT | 2 | ND |
| 468 | NM_014921 | LPHN1 | TCGGCTATCTGGGGAGCAGATTTTGGGTCTGGATCTCCCTGGGGAGTGGGTCCTGGGCTT | 5 | ND |
| 469 | NM_016131 | RAB10 | CGGCATTGTACATAGAAAGGATATGGCTACCTTTTGTTAAATCTGCACTTTCTAAATATC | 5 | ND |
| 470 | NM_000259 | MYO5A | TGCTGTGTTATCTTTATAGCAACACTCATCCTTAACCAACTAGGTACCGTGAGTTTACAT | 2 | ND |
| 471 | NM_017730 | QRICH1 | GAAAGGGCTTGGACTGTGAAAAGAAATGTGGCCCCTTTCCATCTTCAAGAGAGATGGAAT | 4 | ND |
| 472 | NM_014713 | LAPTM4A | TGCAGAGGCAAGAAAAATATTTGACATTGTGACTTGACTGTGGAAGATGATGGTTGCATG | 5 | ND |
| 473 | NM_016511 | CLEC1A | CATAGGAATTTCTCCCAGGAAAGAAATATATCCCCATCTCCGTTTCATATCAGAACTACC | 4 | ND |
| 474 | NM_020530 | OSM | TGGTGGTGGATCCTGGAATTTTCTCACGCAGGAGCCATTGCTCTCCTAGAGGGGGTCTCA | 2 | ND |
| 475 | NM_001736 | C5AR1 | TCTCAAAAGTTCTTTGGGACAAAACAGAAGTCCATGGAGTTATCTAAGCTCTTGTAAGTG | 1 | ND |
| 476 | NM_005242 | F2RL1 | ATGAGTTTTCTGCATCTGTCCTCACTGGAAAACTGACCACGGTCTTCCTTCCAATTGTCT | 2 | ND |
| 477 | NM_000130 | F5 | TCCGTGTCATTCCTAAAACATGGAATCAAAGTATTGCACTTCGCCTGGAACTCTTTGGCT | 3 | ND |
| 478 | NM_018939 | PCDHB6 | CTAAATCTTACTTATGTTTGGAGATCTCTTTTAACTTAAAGTTACATGGTCTGTTTCTTG | 1 | ND |
| 479 | NM_018195 | C11orf57 | AAATCTTTGTTGGAAGGAGCTTTGGCCATATATTTTTTAGCATGCATTGTTTCTGTGCCC | 1 | ND |
| 480 | NM_020682 | AS3MT | GGAAGGTGAAATTGTTGAAGTGGATGAAGAAACAGCAGCTATCTTGAAGAATTCAAGATT | 3 | ND |

(continued)

| | Genbank accession number | Gene Symbol | Probe Sequence | Intensity group | STD order |
|---|---|---|---|---|---|
| 481 | NM_000025 | ADRB3 | TGCAATCAGATAAATAAATATCACTGAATGCAGTTCATCCTCGGCCCACTTTCCCTCCGT | 1 | ND |
| 482 | NM_000434 | NEU1 | ATATTTGGTTTCAGAGTAAGGACTAGGTGCACCACCATGACTGACTATCAATCAAAATGT | 5 | ND |
| 483 | NM_000523 | HOXD13 | AAGGACAAGAGAAAATTGTCTCCAAGCTCAAAGATACTGTCTCCTGATGTGGTCCAGGTTGG | 1 | ND |
| 484 | NM_000986 | RPL24 | CTGCTAAGGCACCTACAAAGGCAGCACCTAAGCAAAAGATTGTGAAGCCTGTGAAAGTTT | 5 | ND |
| 485 | NM_005787 | ALG3 | CCTTACACCCAACCAGATCGTTTCTACCCTCTTCACCTCCAACTTCATTGGCATCTGCTT | 4 | ND |
| 486 | NM_001866 | COX7B | CAAATACGGTAATGCTGTATTAGCTAGTGGAGCCACTTTCTGTATTGTTACATGGACATA | 5 | ND |
| 487 | NM_014784 | ARHGEF11 | AGTGGAAGGCGGAACAAAGGCTACGGGGAACTGCTTTTATGTCAGCATGCCATCAGGACC | 3 | ND |
| 488 | NM_005383 | NEU2 | TCTGTACGGAAGCCAATGATTACGAGGAGATTGTCTTTCTCATGTTCACCCTGAAGCAAGC | 2 | ND |
| 489 | NM_018651 | ZNF167 | CTGTATACTTTTATACCAACTTATTGTAGGCTCTTTGAGGTCAGGTATGTATTTCTTTCC | 5 | ND |
| 490 | NM_001040 | SHBG | TCCTTGCTCTTGGGACACCAGAGAACCCATCTTGGCTCAGTCTCCACCTCCAAGATCAAA | 3 | ND |
| 491 | NM_005750 | C4orf6 | TTGGGAAGGAAGGAACACGCAAAAATTTTTACCTTCTTCTTTCAATTGGACACTATGGAC | 1 | ND |
| 492 | NM_006434 | SORBS1 | TTGGTACTTCAAGAAGGACAAAGCAGTTTGGTACTTTTCCAGGCAACTATGTAAAACCTT | 2 | ND |
| 493 | NM_014423 | AFF4 | ATTCTCGTTGCCTCTGATTTTCTCCACAACACTGTGTCACATCACGAAGGAAAACTGCCA | 2 | ND |
| 494 | NM_003967 | TAAR5 | ACTTCCCTTGTTCTTTGTCCCCTGCCTCATTATGATCAGCTTGTATGTGAAGATCTTTG | 5 | ND |
| 495 | NM_001833 | CLTA | CTGTGGAAACACTACATCTGCAATATCTTAATCCTACTCAGTGAAGCTCTTCACAGTCAT | 5 | ND |
| 496 | NM_021257 | NGB | CTCATTCAGCACTTCTGCTGGGAACTCCCTGACTATTCCGCTGCTGCAGGAACCCAGCTA | 2 | ND |
| 497 | NM_014473 | HSA9761 | CAAGTTCAGGAGCCGCAGAGACGCACAACTTACACTTATCATTTCTAACAGTTTATTGT | 4 | ND |
| 498 | NM_020187 | C3orf37 | CAACGTCACCCAAAAAGGAAGACTCAAAAACACCTCAAAAGGAAGAGTCAGATGTTCCCC | 4 | ND |
| 499 | NM_000102 | CYP17A1 | CCTGGTGGACCTAGTCCCCTGGTTGAAGATTTTCCCCAACAAAACCCTGGAAAAATTAAA | 1 | ND |
| 500 | NM_003457 | ZNF207 | CTTGGTGAGGGCTGTAACTGTTTCCAAGTACTTGTACATTGGAAGTCTGAATGTGTAACA | 5 | ND |
| 501 | NM_012433 | SF3B1 | AATAACCTGTCTTTGTTTTGATGTTAAACAGTAAATGCCAGTAGTGACCAAGAACACAG | 5 | ND |
| 502 | NM_004037 | AMPD2 | TATGTTGTTGTTGCTGACTGAGGCCTTTGCTGTGAACTGCAGTGTTTCATACGAACCATCT | 5 | ND |
| 503 | NM_000739 | CHRM2 | TGTTGGCTTTCATCATCACTTGGGCCCCATACAAATGTCATGGTGCTCATTAACACCTTTT | 1 | ND |
| 504 | NM_001087 | AAMP | GGACCTTGGCCATCTATGACCTGGCTACGCGAGACTCTTAGGCATCAGTGTCAGCACCAGT | 5 | ND |

(continued)

| | Genbank accession number | Gene Symbol | Probe Sequence | Intensity group | STD order |
|---|---|---|---|---|---|
| 505 | NM_014891 | PDAP1 | GAAGAGATTGAGAAGCAGAAGGCAAAAGAGCGTTACATGAAAATGCACTTGGCCGGGAAG | 4 | ND |
| 506 | NM_006601 | PTGES3 | TTTTTTGATTCAGCTTATACCCGGGCTGAAAACCTCAATTTATGTTCATGACAGTGGGGA | 5 | ND |
| 507 | NM_007016 | | TTCAAGGCAGGAAAAAAGGGTATTTAATGCTCTGTCAAGCACATCAGCAATCATAGATTA | 1 | ND |
| 508 | NM_003145 | SSR2 | ATGAAAGCTATGGGACTCTTGGAGGATACCCAGTGTCTATTCTGGGTTAGAGAAGTGCTT | 5 | ND |
| 509 | NM_001104 | ACTN3 | CAGCTCAACGAGTTCCGAGCATCCTTCAACCACTTTGACAGGAAGCGGAATGGGATGATG | 3 | ND |
| 510 | NM_005284 | GPR6 | TTGTGTTCCAGTACTTGGTGCCCTCGGAGACTGTGAGTCTGCTCACGGTGGGCTTCCTCG | 2 | ND |
| 511 | NM_016335 | PRODH | CCCACCTCTGTGACCCCCATGTCCTTGGACCTAGAGGATTGTCCACCTTCTGCCAAGGCC | 5 | ND |
| 512 | NM_007167 | ZMYM6 | TGTGATGGTTGTAAACGACAGGGTAAACTAAGCGAGTCCATAAAGTGGCGAGGCAACATT | 1 | ND |
| 513 | NM_017752 | TBC1D8B | CTTGTATGTAATGATTGCCAAGGTAACAGAATTGCTGACATCTGTCTAAAAAGTTTTGAC | 2 | ND |
| 514 | NM_014582 | OBP2A | TTTAAGAAATTGGTGCAGCACAAGGGACTCTCGGAGGAGGACATTTTCATGCCCCTGCAG | 1 | ND |
| 515 | NM_018015 | CXorf57 | GTTGAGCAGCCTATGAACCTAAAGACATACTGCAGTTTGTTCATAAATGTATTCAGTCTT | 1 | ND |
| 516 | NM_001048 | SST | GGATGAAATGAGGCTTGAGCTGCAGAGATCTGCTAACTCAAACCCGGCTATGGCACCCCG | 1 | ND |
| 517 | NM_021131 | PPP2R4 | GAACTGTCCATTGCTTTTATAGGGTGAGGTAAGTGACAGCCTCCCAAGCCCAGGCTTTGG | 4 | ND |
| 518 | NM_003159 | CDKL5 | ACCATTCTGGACCCCAAGATAGACGCTTCATGTTAAGGACGACAGAACAACAAGGAGAAT | 2 | ND |
| 519 | NM_002833 | PTPN9 | TAGAGATGAACTCACTGAGCAGCCAGAGCCAAATGCAATCGGTACGAATCTTAGAAGGAA | 3 | ND |
| 520 | NM_004540 | NCAM2 | ATCCAACTGGTCTTTGACAGATTTGACTGTCCATATTTAGTTTATGTTTGTCTGATCATC | 2 | ND |
| 521 | NM_002114 | HIVEP1 | GACCAATAATTGTTGTTTTGTGTCAGCTCCAGCCATTTTTGTACATGTTGTATAGACAAT | 3 | ND |
| 522 | NM_017952 | FLJ20758 | AGTTTAGGCTGCACAACTGGTAAAATGACTGTAGATAAATGTTGTAATTAGTGTACACGT | 4 | ND |
| 523 | NM_001994 | F13B | GAATGCAATGTGACAGAGGGCAGTTAAAATATCCAAGATGTATTCCAAGACAAAGCACTC | 1 | ND |
| 524 | NM_003669 | UBE1 | GGCAGCATGACAAAACCCTGTCTCTACCAAAAATACAAAAATTAGCCACGCATGGTGGCA | 5 | ND |
| 525 | NM_001061 | TBXAS1 | CAACCCTGACTGCCAAGAGAAGCTTCTGAGAGAGGTAGACGTTTTTAAGGAGAAACACAT | 2 | ND |
| 526 | NM_005293 | GPR20 | GCCCGAAGCTCCCGCCGCCTGCCGCCAGCCTGCCTGTGCCAGGGCCGTGTGCGCCTTCGT | 5 | ND |
| 527 | NM_002071 | GNAL | TCATTTACGTCGCAGCCTGCAGTAGCTACAACATGGTGATTCGAGAAGATAACAACACCA | 1 | ND |
| 528 | NM_012326 | MAPRE3 | ACTTCTACTTCAGCAAACTTCGTGACATCGAGCTCATCTGCCAGGAGCATGAAAGTGAAA | 1 | ND |

36

EP 2 084 296 B1

| | Genbank accession number | Gene Symbol | Probe Sequence | Intensity group | STD order |
|---|---|---|---|---|---|
| 529 | NM_001824 | CKM | TCCGCCGCTTCTGCGTAGGGCTGGAGAAGATTGAGGAGATCTTTAAGAAAGCTGGCCACC | 1 | ND |
| 530 | NM_000681 | ADRA2A | ATCATGTCATTGATGAACTGCCAAAGTCAGGGGAGGAGGGCAGAGACTTTGTGTTTACAT | 5 | ND |
| 531 | NM_005469 | ACOT8 | CTATATTGGCGAGGGCGACATGAAGATGCACTGCTGCGTGGCCGCCTATATCTCCGACTA | 3 | ND |
| 532 | NM_018486 | HDAC8 | GGGTCATCCTAGGGAAAACACTATCCTCTGAGATCCCAGATCATGAGTTTTTCACAGCAT | 1 | ND |
| 533 | NM_012236 | SCMH1 | CTGGCCTCTCACCAGGAGTTTAGGCTGAATGCCTTCCACGTGATGGAGGAAAAGGCCAAC | 2 | ND |
| 534 | NM_005768 | MBOAT5 | TTCCTGAGCCTACTATTCATATTGCCTTATATTCACAAAGCAATGGTGCCAAGGAAAGAG | 2 | ND |
| 535 | NM_004977 | KCNC3 | GGGGGTGCTGACCATCGCCATGCCTGTGCCCGTCATTGTCAACAACTTTGGCATGTACTA | 4 | ND |
| 536 | NM_001525 | HCRTR1 | TGAAAGGCTGTGGCTTCAGTCCTGGGTTTCTGCCTGTGTGACTCTGGATAAGTCACTTCC | 5 | ND |
| 537 | NM_005268 | GJB5 | GCTCTGGTGGACATATGTCTGCAGCCTAGTGTTCAAGGCGAGCGTGGACATCGCCTTTCT | 1 | ND |
| 538 | NM_003531 | HIST1H3C | TCTGTGCGCTATTCACGCTAAACGCGTCACCATCATGCCCAAAGATATCCAGCTGGCACG | 5 | ND |
| 539 | NM_017887 | C1orf123 | CAGCTACTCCCTCCACAAATAAGAATTCGTGCAGCAGCAGTTCACTCCTTAGGAAAATGA | 4 | ND |
| 540 | NM_003077 | SMARCD2 | TTTAGTTTTATAAATGTAGTGATAGGATTCCTTGTTGCTTGGTCCCCAAAGCCTTATACT | 4 | ND |
| 541 | NM_000475 | NR0B1 | TAAAGTCATGTGGGCCACACAAGTGCAGTAGTGCAGTTCACCATGAGGGAAGAATAAAGA | 2 | ND |
| 542 | NM_003310 | TSSC1 | GGGAGAGCCGCTGTTCCCTTCCTGTAGCAGCAGCATTTATGAATGGGGTGAATGGGGCTA | 5 | ND |
| 543 | NM_020999 | NEUROG3 | CATTCAAAGAATACTAGAATGGTAGCACTACCCGGCCGGAGCCGCCCACCGTCTTGGGTC | 5 | ND |
| 544 | NM_012454 | TIAM2 | GCTTCAGGGAATAACATTCTGAGCCCTCGATGGCAGTATTTCCTTCGGAACTGAAATACA | 1 | ND |
| 545 | NM_003049 | SLC10A1 | TGGATTCTGGTCCCAAAGCAATTCTGAAAGCCAGTGTGGTAAACTAGAGAGAGCAGCAAA | 1 | ND |
| 546 | NM_000112 | SLC26A2 | TCCTCCTTTGAAGCTAATGGCATTTGTATATACACACTGCAGCAGAGCTTGTAGCTGGAC | 1 | ND |
| 547 | NM_001499 | GLE1L | CAGAGTGGGGGACCTCATTCTTGCTCATCTACATAAGAAGTGTCCTTACTCTGTTCCTTT | 1 | ND |
| 548 | NM_018943 | TUBA8 | TATATACCTTCCCCTTGGCTGTGTCTCTTTATTTATGCTGTGCCATTCAAAGCACATGTT | 4 | ND |
| 549 | NM_014663 | JMJD2A | CTTCCCAAGAGAGTCAAATCTAGACTGTCAGTAGCCTCAGACATGCGCTTCAATGAGATT | 3 | ND |
| 550 | NM_004925 | AQP3 | AATTTGGGTCAATACATCCTTTTGTCTCCCAAGGGAAGAGAATGGGCAGCAGGTATGTGT | 3 | ND |
| 551 | NM_002139 | RBMX | ATAATGCTGGCCATTTTGCCTTTCTGACATTTCCTTGGGAATCTGCAAGAACCTCCCCTT | 5 | ND |
| 552 | NM_017943 | FBXO34 | TATAATGGTTGTGTTTTCATGGGGCTATGAAAGTGCACGTTAAACCTGAGCGCCTTTACC | 3 | ND |

(continued)

| | Genbank accession number | Gene Symbol | Probe Sequence | Intensity group | STD order |
|---|---|---|---|---|---|
| 553 | NM_003602 | NCF1 | AAACGTGTTTTCACAGGTGCTGTTTTCTGTTTTCCGTGTTCGTAACAGAAGGGAGGGGAA | 2 | ND |
| 554 | NM_005631 | SMO | GGTGGGTGAGGAGATTCCCACCTTCCATAGCCTCCAAACATGTTCCCAAGGCCCCACTTT | 4 | ND |
| 555 | NM_006067 | COX4NB | CTATAATCCTCCAAATCAAAGCTCTTTCTGCTTGTGCAAGATTGTTCCTATTAAACAGTT | 5 | ND |
| 556 | NM_006791 | MORF4L1 | AAAGAATTCTGCAACTTTGTTCAGTGCCAGCGATTATGAAGTGGCTCCTCCTGAGTACCA | 5 | ND |
| 557 | NM_005296 | GPR23 | GGTAAAATATGTTATGTGCATTTTGAAAACAGAAAACAAATTGCGTTGGCATGTACGTGG | 1 | ND |
| 558 | NM_014225 | PPP2R1A | CTCTGACTGTTCTGTCTCTCGCCTGATGCTGGAAGAGGAGCAAACACTGGCCTCTGGTGT | 4 | ND |
| 559 | NM_014433 | RTDR1 | CCATCAGTGTCATCGAGTTCAAACCCTGAGCCCTTCATTCACCTCTGTGAGTGAATAAAT | 2 | ND |
| 560 | NM_006640 | Sep-09 | AGGCTCTGTTCCTCAATGGCCTTTTGCTACGTGCCTCCCGAGAAATTTGTGTCTTTTTGTAT | 5 | ND |
| 561 | NM_003153 | STAT6 | CAAGGGCTGAGATTCTTCGTGTATAGCTGTGTGAACGTGTATGTACCTAGGATATGTTAA | 5 | ND |
| 562 | NM_014187 | HSPC171 | GCCAAAAGCAGTCTGAGGTATTGGGTATACTTATACTCTATAGGGTCGTTGAATAAATGG | 5 | ND |
| 563 | NM_003746 | DYNLL1 | AAATTTTCAGCCTTGCTAAGGGAACATCTCGATGTTTGAACCTTTGTTGTGTTTTGTACA | 5 | ND |
| 564 | NM_005283 | XCR1 | CTGTGTGGGTAGCCAGCATCCTGTCCTCCATCCTCGACACCATCTTCCACAAGGTGCTTT | 5 | ND |
| 565 | NM_007033 | RER1 | TTCTGGCCGATTCTGGTGATGTACTTCATCATGCTCTTCTGTATCACGATGAAGAGGCAA | 5 | ND |
| 566 | NM_015913 | TXNDC12 | CTACAAGTATTTTTATGTCAGTGCCGAGCAAGTTGTTCAGGGGATGAAGGAAGCTCAGGA | 2 | ND |
| 567 | NM_000333 | ATXN7 | TGGTGAACAGCAGTGATTCTACTCTTTCTCTTGGGCCATTCATTCACCAGTCCAATGAAC | 3 | ND |
| 568 | NM_018421 | TBC1D2 | CAAGCAAGAGCACTGCCTCTATAGGGTAACCTGGAACATTCTCTAGGTTATATCAATATA | 4 | ND |
| 569 | NM_015927 | TGFB1I1 | CCATCCTGGATAACTACATCTCGGCGCTCAGCGCGCTCTGGCACCCGGACTGTTTCGTCT | 2 | ND |
| 570 | NM_018562 | | TCTTCAAGCACATCAGAAGTTTCTCCCTAACTTAATTATGTTTGTTCTAGACCCTCTAGC | 1 | ND |
| 571 | NM_006874 | ELF2 | GAGGGGTTGGGATGGGTAATCTCATTGTTACATATAGCAATTTTTGATGCATTTTATATG | 3 | ND |
| 572 | NM_014280 | DNAJC8 | GTGATGGTTAGAAACTTCGTGGATAGTTTGTGGAAATCATCCAATTAAACATACTGCTTA | 5 | ND |
| 573 | NM_006501 | | TGGATCGGAAATACAGCATCTGTAAGAGCGGCTGCTTCTACCAGAAGAAAGAGGAGGACT | 2 | ND |
| 574 | NM_007123 | USH2A | AATATTATCAAAAGCTTAAATAAAGACAGATTGAACTCTGTACCAGCACAATCCTGCCTC | 1 | ND |
| 575 | NM_006594 | AP4B1 | TGCTGATTATTTTGAGAAAACTTGGCTTAGCCTTAAAGTTGCTCATCAGCAAGTGTTGCC | 2 | ND |
| 576 | NM_016226 | VPS29 | TTAGCCCTGTTGCAGAGGCAATTTGATGTGGACATTCTTATCTCGGGACACACACACAAA | 5 | ND |

| | Genbank accession number | Gene Symbol | Probe Sequence | Intensity group | STD order |
|---|---|---|---|---|---|
| 577 | NM_007373 | SHOC2 | ATGGAAAAGAGGCACATTGCATAGAAGCCATTGGGGAGTTCAGTGGAAGTTCTGTAAGAT | 4 | ND |
| 578 | NM_001269 | RCC1 | GCCAGAGACTAGTCCTGAGATGGAAACAGCAACTTGTACAGTGGCTGAGAAAATAGGATA | 4 | ND |
| 579 | NM_005260 | GDF9 | CCCAAAATGAGTGTGAGCTCCATGACTTTAGACTTAGCTTTAGTCAGCTGAAGTGGGACA | 1 | ND |
| 580 | NM_006311 | NCOR1 | AAACAAACAAAAAACTGCCTTTGATACAGGCAATTCAGTGGACTATAATAATAGTGGAGG | 3 | ND |
| 581 | NM_020230 | PPAN | TGGAGGGCATGAAGAAGGCACGGGTCGGGGGTAGTGATGAAGAGGCCTCTGGGATCCCTT | 4 | ND |
| 582 | NM_007245 | ATXN2L | GTGACCCCGACTGTCTCCTGACTTAGCCGAGGTAAGGTCAGTGCAGCAGACAGGGCCAGA | 1 | ND |
| 583 | NM_006367 | CAP1 | TTTTTTAACAATGAGCATGAAGGTAGCAGAAGCTGGTGTGTTCCAGATGGTTCTTCTAA | 5 | ND |
| 584 | NM_017567 | NAGK | CTGGGGCCATATGATGGGTGATGAGGGTTCAGCCTACTGGATCGCACACCAAGCAGTGAA | 5 | ND |
| 585 | NM_016611 | | AGACCCAAGCCTGACCCCATCCGAGTGGAATTTGAGTCCTAAAGAAATAAAAGAGTCGAT | 4 | ND |
| 586 | NM_014336 | AIPL1 | CAGTTTCTAGATTTTACCCCATGTCAATGACAAATGAGGATTTGATGCTCTGATCCTTTC | 1 | ND |
| 587 | NM_004510 | SP110 | AAGGAACGCAAAGAACTGGAAACGGAATATACGTTGTGAAGGAATGACCCTAGGAGAGCT | 4 | ND |
| 588 | NM_004592 | SFRS8 | TCTCTCAGGAAAAAGAAGCCCAGATCTCTTCAGCAATCGTTTCTTCCGTGCAGAGCAAAA | 4 | ND |
| 589 | NM_005364 | MAGEA8 | CTCAGTTCCTGTTCTATTGGGCGATTTGGAGGTTTATCTTTGTTTCCTTTTGGAATTGTT | 2 | ND |
| 590 | NM_000088 | COL1A1 | CCGTAGGGGTGGGAGGAAGCAAAAGACTCTGTACCTATTTTGTATGTGTATAATAATTTG | 5 | ND |
| 591 | NM_015478 | L3MBTL | CATTGCTTAACGATGGGGATACATTCTTAGAAATGTGTCACTAGGCAATTCTGTCATTGT | 1 | ND |
| 592 | NM_001841 | CNR2 | CATCACTGCCTGGCTCACTGGAAGAAGTGTGTGAGGGGCCTTGGGTCAGAGGCAAAAGAA | 3 | ND |
| 593 | NM_000460 | THPO | CCTGGCAGTTGAACAGAGGGAGAGACTAACCTTGAGTCAGAAAACAGAGAAAGGGTAATT | 2 | ND |
| 594 | NM_000455 | STK11 | **CCGTGGCCTCGTGCTCCGCAGGGCGCCCAGCGCCGTCCGGCGGCCCCGCCGCAGACCAGC** | 5 | ND |
| 595 | NM_003522 | HIST1H2BG | AAGCGCACCCGCAAGGAGAGTTACTCTGTGTACGTGTACAAGGTGCTGAAACAGGTCCAT | 4 | ND |
| 596 | NM_000616 | CD4 | TTCTCTCATTATTTCTCTCTGACCCTCTCCCCACTGCTCATTTGGATCCCAGGGGAGTGT | 5 | ND |
| 597 | NM_014359 | OPTC | GACTGCCTACCTGTATGCACGCTTCAACCGCATCAGCCGTATCAGGGCCGAAGACTTCAA | 1 | ND |
| 598 | NM_005014 | OMD | TTATGGTGAACAACGAAGCACTAATGGTCAAACAATACAACTAAAGACACAAGTTTTCAG | 2 | ND |
| 599 | NM_017503 | SURF2 | CCTTGGAAGCACGGAGGATGGGGATGGCACTGATGACTTTTTGACAGACAAAGAGGATGA | 4 | ND |

(continued)

| | Genbank accession number | Gene Symbol | Probe Sequence | Intensity group | STD order |
|---|---|---|---|---|---|
| 600 | NM_017451 | BAIAP2 | TTCCCGTAAGCACGTAATTCCTGCGAGGTCCGGCCAGCTACACCTGGAGTGTGGGGCCTGGT | 1 | ND |
| 601 | NM_018619 | | AGAGTTCAACACCAGCCTGGGCCATATTGTGAGAACACGTCTCTACAGACGATCAAAAAA | 5 | ND |
| 602 | NM_007265 | ECD | CACCGATCACAGACCAACAAGTAAGCCAACAAAAAATTAACCAGCACATTTAGCTTCTCT | 4 | ND |
| 603 | NM_003744 | NUMB | ATTGATGCCTACTGAAATAAAAGAGGAAAGGCTGGAAGCTGCAGACAGGATCCCTAGCT | 4 | ND |
| 604 | NM_006550 | | AAATTTTGGATTGTATGTTCAGGAGAAGAGAGGGATGGATTGAAAAGAAGGCAGCAGCTAGA | 3 | ND |
| 605 | NM_012304 | FBXL7 | ACAAAGCTGACTGTTCACACTGATTGCCCAGCACATACCGTCTTGCCAGTTTCTTCTTTT | 4 | ND |
| 606 | NM_006914 | RORB | AGAAGCTGCAGGTATTTAAGCAATCTCATCCAGAGATAGTGAATACACTGTTTCCTCCGT | 1 | ND |
| 607 | NM_002087 | GRN | ATCCCCTCCCCGTTTCAGTGGACCCTGTGGCCAGGTGCTTTTCCCTATCCACAGGGGTGT | 5 | ND |
| 608 | NM_021062 | HIST1H2BB | GGCTAAGCATGCTGTCCGAGGGCACTAAGGCAGTTACCAAGTACACTAGCTCTAAATA | 4 | ND |
| 609 | NM_002582 | PARN | GAGTGTCGGCTGTGAAATCTGCAAAAAGAGCTGACATTCCAGCTGCTGTGATCATGAATT | 3 | ND |
| 610 | NM_014748 | SNX17 | CAGGCTATCATGATGAGCATCTGCTTGCAGTCCATGGTTGATGAACTGATGGTGAAGAAA | 4 | ND |
| 611 | NM_016488 | PPHLN1 | ACAGTTTGCATTGAGGCAGAATTTACATGAAATAGGTGAGCGGTGTGTTGAAGAACTCAA | 5 | ND |
| 612 | NM_017859 | UCKL1 | TGCTCATGGCAGAGATGGGCGTGCACTCAGTGGCCTATGCATTTCCGCGAGTGAGAATCA | 4 | ND |
| 613 | NM_018278 | | ACAGTAAGACATTGTTGGTGGAGATGAGAGTTTGAGAGTCAGGGTGACAATAAGTTATTT | 2 | ND |
| 614 | NM_000394 | CRYAA | CGGAGGACCTCACCGTGAAGGTGCAGGACGACTTTGTGGAGATCCACGGAAAGCACAACG | 1 | ND |
| 615 | NM_021019 | MYL6 | ATGACAGAGGAAGAAGTAGAGATGCTGGTGGCAGGGCATGAGGACAGCAATGGTTGTATC | 5 | ND |
| 616 | NM_004198 | CHRNA6 | AAATTTACAGACACACCATATTTGTTCTGCATTCCCTGCCACAAGGAAAGGAAAGCAAAGGC | 2 | ND |
| 617 | NM_014907 | FRMPD1 | CTGAGGGCTTCATCCAACTCATGGAGAGCTTGCTGGAGCTACAAGACATTTTAGAAACTT | 1 | ND |
| 618 | NM_018207 | TRIM62 | AATCTGGGCCACCCCAGCAGTATTTTTATTTTAAAATGTTGCCCATTTTATGAGTTATGAT | 3 | ND |
| 619 | NM_021023 | CFHR1 | TCAGGAAGTTACTGGGATTACATTCATTGCACACAAAATGGGTGGTCACCAGCAGTACCA | 2 | ND |
| 620 | NM_014898 | ZFP30 | TAAAATTTTCCATGATGTTCTTTTAAAGATCAAATTATCCATGAAATTATAGCCAGTTTA | 1 | ND |
| 621 | NM_014565 | OR1A1 | AACTTCTACTGTGACATTACCCCCTTGCTGAAGTTATCCGTTCTGACATCCACTTTCAT | 2 | ND |
| 622 | NM_020389 | TRPC7 | CCAGAAAATCATGAAACGGCTCATAAAAAGATACGTCCTGAAAGCCCAGGTGGACAGAGA | 2 | ND |
| 623 | NM_017885 | HCFC1 R1 | TTCCCTCAACAGAGAGGACACTGAGCCCAACGGAGTTCTGGGGATGGGAGGGGTGGGAGCATG | 5 | ND |

| | Genbank accession number | Gene Symbol | Probe Sequence | Intensity group | STD order |
|---|---|---|---|---|---|
| 624 | NM_000066 | C8B | TTGGGGGCATTTATGAATACACCCTCGTTATGAACAAAGAGGCCATGGAGAGAGGAGATT | 2 | ND |
| 625 | NM_003080 | SMPD2 | GGCAGGTGCATTCTACCTCTTCCACGTACAGGAGGTCAATGGCTTATATAGGGCCCAGGC | 4 | ND |
| 626 | NM_018945 | PDE7B | ATGAGCAAGCAGTGGAGTGAAAGGGTCTGTGAAGAATTCTACAGGCAAGGTGAACTTGAA | 5 | ND |
| 627 | NM_005334 | HCFC1 | TTTGTTTCTGAGGAGAGTACACCGTTCACTATTGTAGAGTAACCCCTGTGACTCAATATT | 5 | ND |
| 628 | NM_001719 | BMP7 | TGGTGTCTGTGCGAAAGGAAAATTGACCCGGAAGTTCCTGTAATAAATGTCACAATAAAA | 5 | ND |
| 629 | NM_003577 | UTF1 | CGCGACGCGGAACCCCACCTGGAGCTCCGCTTCAGCCCGTCCCCACCGAAGTCTGCGGAC | 5 | ND |
| 630 | NM_003320 | TUB | TTCAACTTCAGAAGGCCTCACTCAAGCCTGAGAGAAGTTGGGAGGGTGGTGGGGACAGGT | 1 | ND |
| 631 | NM_013374 | PDCD6IP | CATAAGACATGGTTGGGACATCAGATACTTACAAAGATGGTTTAAGTATGGATACTAGAG | 5 | ND |
| 632 | NM_004942 | DEFB4 | ACAAATTGGCACCTGTGGTCTCCCTGGAACAAAATGCTGCAAAAGCCATGAGGAGGCCA | 4 | ND |
| 633 | NM_016518 | PIPOX | TGAATCCCCCATAAACACCAGATGATTGAGTCTACCTTCTTTCCTTGGCCCGCTCCCTTT | 4 | ND |
| 634 | NM_006220 | | TGATTGAACAAAATACCAAGTCTCCCCTCTTCATGGGAAAAGTGGTGAATCCCACCCAAA | 5 | ND |
| 635 | NM_006702 | PNPLA6 | ATTGAGCCCCCCACGAGCTATGTCTCTGATGGCTGTGCTGACGGAGAGGAGTCAGATTGT | 5 | ND |
| 636 | NM_018229 | C14orf108 | GTGTTGTTACAGAATACCAGAGACCATGTTAGAGACAACTACATCTCTTCAAAAAACAGC | 3 | ND |
| 637 | NM_003716 | CADPS | GGAGAATACATAGTCTAACCACTAGGCGTGTCCCTGTTATCAGCAAAGATCAATGATGCT | 2 | ND |
| 638 | NM_000079 | CHRNA1 | GGAGAAGATGACTCTGAGCATCTCTGTCTTACTGTCTTTGACTGTGTTCCTTCTGGTCAT | 1 | ND |
| 639 | NM_005884 | PAK4 | TGGAGAGAACACTAAGAGGTGAACATGTATGAGTGTGTGCACGCGTGTGAGTGTGCATGT | 3 | ND |
| 640 | NM_018117 | BRWD2 | GCATCTATGTTGAGAGTAAGTTTGTATCCTGCGTTGGTCTCAGAAAGAACGTGAATGCTT | 2 | ND |
| 641 | NM_014814 | PSMD6 | CACAGTCTTCCAGCAGTTCGGCAGTATCTGTTTTCACTCTATGAATGCCGTTACTCTGTT | 5 | ND |
| 642 | NM_018280 | C22orf26 | CCAGGTGTGACCTTGTAGATGGCCTCTATTCTGGATTCACAGCTGCTGAGGAGGAGGAAG | 1 | ND |
| 643 | NM_018312 | SAPS3 | TGATTATTCCTACAAGTGAAACACTAGACTATTTGGAGTGTATATGGCTTGTGTTTTGGG | 2 | ND |
| 644 | NM_004699 | FAM50A | GGTGGAGCAGCTCATGTACATCAAGGAGGACTTGATCATCCCTCACCATCACAGCTTCTA | 5 | ND |
| 645 | NM_005267 | GJA8 | CTTCTAGAAGAAGAGAAAATCGTTTCCCACTATTTCCCCTTGACCGAGGTTGGGATGGTG | 1 | ND |
| 646 | NM_001244 | TNFSF8 | TTCAGGAAGAAAGCGCCTCTCTACCATACAGTATTTCATCCCTCCAAACACTTGGGCAAA | 3 | ND |
| 647 | NM_004518 | KCNQ2 | ACATTTCATAATGCCTTCAGTACCGACGTACACTTCTGACCATTTTGTATGTGTCCTTGT | 1 | ND |

(continued)

| | Genbank accession number | Gene Symbol | Probe Sequence | Intensity group | STD order |
|---|---|---|---|---|---|
| 648 | NM_006222 | | TCGCAGACCCAGGGCAATGTGGTGGGAGGAGTGTTCCAAAGAGAAGAGATCTGGTCAGCAGA | 4 | ND |
| 649 | NM_004492 | GTF2A2 | AGAGGGTCAGGAACAGAGTCAATTTCAGGGGCTCTAAATACGTACAGATTCTGCGATA | 5 | ND |
| 650 | NM_005263 | GFI1 | CTGTGGACAAGATGTCATTCACTCAGCAAATGTTCATGGATCACCGGCTACCAAGT | 1 | ND |
| 651 | NM_001277 | CHKA | AGCCTGTATGTGTGTGGGGCGTGGATCGAGTGTAGCTGTGAAATCCATATATGAAAT | 4 | ND |
| 652 | NM_020989 | CRYGC | CTAAGGCAGGCTCTTTGCGGAGAGTGGTGGATTTGTATTAAAATAGCTTAACACTACCAA | 1 | ND |
| 653 | NM_017676 | FLJ20125 | CTCTTGAAAAGCAGACTTTCAGTCTGTTGGACTCTTCAAACCAGGTTCTTGAATACTTAA | 1 | ND |
| 654 | NM_018664 | SNFT | ATACCTGGGAGGAAGGCTTTCCTTCACAATTGTATACAGGGGGCACCTGTGGCCAGGCC | 2 | ND |
| 655 | NM_005294 | RABGAP1 | TTATTGTCTGCTTCACCTATTTCAACATCTTCCGCATCTGCCAACAGCACACAAAGGATA | 1 | ND |
| 656 | NM_007197 | FZD10 | CTTCACAGTGCCAGGAAAGAGTGGTTCTGCGTGTATATTTGTAATATATGATATTT | 2 | ND |
| 657 | NM_005865 | PRSS16 | CTGCTTAGGCTATGTTAGCTGTGACAGGAACCTGCCATAGATTTGCACTGTTCTTTCCTA | 2 | ND |
| 658 | NM_002236 | KCNF1 | AACTTTGTCAGGTACTACACAACAAGCAGCGCGTCCTGGAGACCGGCCAAGCACGAGCTG | 1 | ND |
| 659 | NM_004679 | VCY | ATCTACTCCCCTATCTCCCTGAGCAGCAACTAAGTTTAGGCCCAGCTGCCAGACCTCAGA | 4 | ND |
| 660 | NM_018671 | UNC45A | TTGGCCAGCACTGCCTGCGAGCCTCACTCAGAGGGGCCCTTTTCTGTACTACTGTAGTCA | 5 | ND |
| 661 | NM_001163 | APBA1 | TGTCCACCTGCCAGAGCATTATTAAGGGCTTAAAGAATCAGTCCGAGTCAAGCTGAATA | 2 | ND |
| 662 | NM_005998 | CCT3 | GTGAGACGGGTACTTTGGTGGACATGAAGGAACTGGGCATATGGGAGCCATTGGCTGTGA | 5 | ND |
| 663 | NM_003403 | YY1 | AGACAGGCCCTATGTGTGCCCCTTCGATGGTGTAATAAGAAGTTTGCTCAGTCAACTAA | 5 | ND |
| 664 | NM_016025 | METTL9 | TGTCAAGCAGAAGTGATGAGTGTTCAAGGAGGCAGGTTGCCCCAAAGTGTTTCTAACTT | 2 | ND |
| 665 | NM_004262 | TMPRSS11D | CTCAAGAATATGCTGGCCACACAGTTCCAGAGCTAAGGCAAGGACAGGTCAGAATAATAA | 1 | ND |
| 666 | NM_005604 | POU3F2 | AGAGAGACAGAGAGATGGCAAGCACTGAGATAAATACCTGCAAAACTAAATAAATTACC | 4 | ND |
| 667 | NM_014133 | | ACACACACAACTTCTCTGCTACGAAAAGTCTTGATTCTGGCCTCAGGATGTGAACA | 2 | ND |
| 668 | NM_014744 | TBC1D5 | TTTTTTAAATTCTTCATAGTTGAGTATTATTTGCAATTTATTAGTTACAGTGCTATTAA | 1 | ND |
| 669 | NM_016190 | CRNN | TTCTCAATGAGATAATTTCTGCAAGGAGCTTTCTATCCTGAACTCTTCTTTCTTACCTGC | 1 | ND |
| 670 | NM_007285 | GABARAPL2 | TGAGGTAGGTGCGGTATTAAAGTGAAAGGGAAGGTGATGCATTTATTCTGGGTTATGCTT | 5 | ND |
| 671 | NM_006257 | PRKCQ | ACTGATCAACAGCATGGACCAGAATATGTTCAGGAACTTTCCTTCATGAACCCCGGGAT | 1 | ND |

(continued)

| | Genbank accession number | Gene Symbol | Probe Sequence | Intensity group | STD order |
|---|---|---|---|---|---|
| 672 | NM_000122 | ERCC3 | GACACACAGGAAATGGCTTACTCAACCAAGCGGCAGAGATTCTTGGTAGATCAAGGTTAT | 4 | ND |
| 673 | NM_000533 | PLP1 | TTCATGGGGTATTATCCATTCAGTCATCGTAGGTGATTTGAAGGTCTTGATTTGTTTTAG | 1 | ND |
| 674 | NM_012432 | SETDB1 | TCAAGTGGCAGTAAAATCAACCCGAGGCTTTGCTCTTAAATCAACCCATGGGATTGCAAT | 2 | ND |
| 675 | NM_018298 | MCOLN3 | GAGTGCCTTTTCTCTCTGATAAATGGAGATGATATGTTTGCCACGTTTGCAAAAATGCAG | 3 | ND |
| 676 | NM_000219 | KCNE1 | TATGTCGTTGAAAACCATCTGGCCATAGAACAACCCAACACACACCTTCCTGAGACGAAG | 1 | ND |
| 677 | NM_005107 | ENDOGL1 | CCCAGATAAGAAAGCCATCCTAGTTTTTATCTCAAGATGTGTCATACCGTCTGTAATGAA | 1 | ND |
| 678 | NM_004551 | NDUFS3 | TGTGGATCCTAGACAGCGCCTTATCTATGATTGAGTGTCCGTGTAAATAAATTCCTACTT | 5 | ND |
| 679 | NM_017895 | DDX27 | CATGTCTGTCAATCTCCCTTCTTGCTGATTAGCTTTCATATGACTATATTAAATGGAAGT | 5 | ND |
| 680 | NM_001242 | TNFRSF7 | AGCCACAACTGCAGTCCCATCCTCTTGTCAGGGCCCTTTCCTGTGTACACGTGACAGAGT | 4 | ND |
| 681 | NM_005066 | SFPQ | GTAGGGGGTGAAAGTGGGTTTGATTAAATGGATCTTTTATGGCCCTATGATCTATCCTTT | 5 | ND |
| 682 | NM_012390 | SMR3A | CACCCTATGGTCCAGGGAGAATTCAATCACACTCTCTTCCTCCTCCTTATGGCCCAGGTT | 5 | ND |
| 683 | NM_004437 | EPB41 | AGAGCAAGAGCAGTATGAAAGTACCATCGGATTCAAACTTCCCAGTTACCGAGCAGCTAA | 2 | ND |
| 684 | NM_007065 | CDC37 | AGGAACTCCAGAAGTGCTTCGATGTGAAGGACGTGCAGATGCTGCAGGACGCCATCAGCA | 5 | ND |
| 685 | NM_013318 | | CGTCGTCTGCCCTTTCAAATATGCTGGTCTACATGGAAAGACAAAGAGGAAAGGCCAAAA | 5 | ND |
| 686 | NM_007354 | C3orf27 | TGCCCGGCCTGATACCCTAGCAGGTTGGGTATTCAGAGGTGCCTGGGGAGCTTGTTAAAA | 1 | ND |
| 687 | NM_018332 | DDX19A | ATAGGTGCAAATGATGGGGGGCAATAGAAGAAAAAATTTGCATTTTGGAAAATTGGGTCC | 2 | ND |
| 688 | NM_003058 | SLC22A2 | CAGATCCTGCCAAATTCTTCCAGCTCACTTTATTCTCAGCATTCCTAGGACATTGGACAT | 2 | ND |
| 689 | NM_007080 | LSM6 | GTGAAATGTTCTTTCACTTGTAAGTTTCAGTCATTTTCTTTTACCTCGTTGTCAGTGTAC | 4 | ND |
| 690 | NM_021174 | KIAA1967 | AGAAAAAGGCTTTTCGAGTGTGGGACAAGGTCTGATGTCAGTGAACGGAATTGAAGAGCA | 1 | ND |
| 691 | NM_014901 | RNF44 | ACCTTGCTATAGATGCCATGTTACCAATGATTTCCTGTGGTGGGGGCTTGCCATTGTTTA | 3 | ND |
| 692 | NM_000098 | CPT2 | ATCCATCAAAAGTTAACTTCTGGGCAGATGAAAAGCTACCATCACTTCCTCATCATGAAA | 4 | ND |
| 693 | NM_018352 | FLJ11184 | ATGGACAGTAACCTGTTTCCTGAAAGATTCCTGTGGGTACTTTTTGAGCTGTGATAATAG | 3 | ND |
| 694 | NM_016156 | MTMR2 | TGTTCACCTGCTACAAGTAAGAGTTTGGTGCTGGTAGAAACATTTGACTCTGATGTCTAT | 4 | ND |
| 695 | NM_015530 | GORASP2 | ACTCACACGCAACATTTCTTGTACTTTGTAAGTCGTTTGCGAGAATGCAGACCACCTCAC | 5 | ND |

EP 2 084 296 B1

(continued)

| Genbank accession number | Gene Symbol | Probe Sequence | Intensity group | STD order |
|---|---|---|---|---|
| 696 | NM_015369 | | CTTTCCTAAATTGCAATATGGCCTTGGTATGGTTTATTTGTACTTTGGTGGGGGATTCG | 1 | ND |
| 697 | NM_006694 | JTB | AAGCAAATCGAGTCCATATAGCTACATTCCACCCTTGTATCCTGGGTCTTAGAGACCCTA | 5 | ND |
| 698 | NM_002653 | PITX1 | CTTCTTCAACTCCATGAGCCCGCTGTCGTGCAGTCCATGTTCTCAGCACCCAGCTCCAT | 4 | ND |
| 699 | NM_014474 | SMPDL3B | AGGTGTCCCCATAAGGCCATGTTCATCACACCTGGAGTCACCCCATGGAAAACCACATT | 3 | ND |
| 700 | NM_004941 | DHX8 | GTTGGGAGCTCATATCTAACACAGAGACACATTGCATCAACTTCAAGAAAGGGACAATTT | 3 | ND |
| 701 | NM_007204 | DDX20 | GATACCTTTGGATATCCATCCTCCTCGACTTATAGTACAGTGGTGTATAGTGGCATTTCT | 2 | ND |
| 702 | NM_000620 | NOS1 | CTCCCCAATTCCCCAGGGAAGGAAACTGTTGTGTCCAATCCCCATTAAAGACAAATTGAT | 1 | ND |
| 703 | NM_001281 | CKAP1 | TGGTGGGCAGCCCTGCTTCCTGCATGGAACTGGGAGCTGTATGGAGTTGACGACAAGTTCT | 4 | ND |
| 704 | NM_006590 | USP39 | CTGGGAGTAGTTGAAGAACAGATAATTCCTTCCAAACATCAAGCCTTGGGATTCTTGGAG | 5 | ND |
| 705 | NM_006249 | PRB3 | CAAGGTCCCCACCTGTCGTCCAGGAAAGCCAGAAGGATCACCTTCACAAGGAGGCAACAAA | 5 | ND |
| 706 | NM_002218 | ITIH4 | GCCGCTTCTGGGGCCTGGACCACCATGGGGAGGAAGAGTCCCACTCATTACAAAATAAAGA | 3 | ND |
| 707 | NM_005969 | NAP1L4 | GCAGCACAAATCCGTGGACAGAGCTTACTCCATCTAACTCGTTTTCAAGTGCATGATTTT | 4 | ND |
| 708 | NM_018317 | TBC1D19 | TTGCTTTTATGGGATAGAATCCTAGGATACAACTCTCTGGAAATTCTTGCTGTGCTGGCA | 1 | ND |
| 709 | NM_006938 | SNRPD1 | GCAGAGGAAGAGGGGGTCTAGGCGATAATGTCTCTCAAAGATTCAAAGTCATATGAGAT | 5 | ND |
| 710 | NM_001375 | DNASE2 | CCACTGAAGCCAGAGGATCGATTGAACCAGGGAGATCATGGTCACAGTGAACTATGATTA | 5 | ND |
| 711 | NM_012434 | SLC17A5 | TCAAACTTTCCCTTCCCAGCACACTCAGTGAAGCTTTGTTAAAGGAATGAGGAGTTTAGGCCCC | 4 | ND |
| 712 | NM_018610 | | ATTTTGAGAACTGCAGCTGAGCAGTCAGTGAAGCTTTGTTAAAGGAATGAGGAGTTTAGGCCCC | 1 | ND |
| 713 | NM_002807 | PSMD1 | GATTAAGGGCCAGAGGATCTCACTTGCTTATCTGAAGAAGATTGTCCAGGCTCATATTGG | 5 | ND |
| 714 | NM_014275 | MGAT4B | CGTTTTAGAAGAGCTTTACTTGGGCGCCGTCTCTGGGCGCGAACACTGGAATGCAT | 5 | ND |
| 715 | NM_016305 | SS18L2 | AACCTGAAGAGAGAAACTCAGTAGGTGTTGGGCAGGAATTGGTGAGATTCCTGACTTGATA | 5 | ND |
| 716 | NM_005381 | NCL | CCTTGGAAATCCGTCTAGTTAACATTTCAAGGGCAATACCGTGTTGGTTTTGACTGGATA | 5 | ND |
| 717 | NM_018067 | RPRC1 | TGGAAGTGGCTGGCCCTGGGCCCCTGGGTGGGTCCTCTCTGTTGTTTTAATCTGCACCTTAT | 5 | ND |
| 718 | NM_014498 | GOLPH4 | CCAGCAGATGACCCTAATAATCAAGGTGAGGATGAATTTGAAGAAGCCGAGCAAGTGAGA | 3 | ND |
| 719 | NM_014044 | UNC50 | GTGTCCACTATAGGATTTGGCTTTGTGCTGGACATGGGATTCTTTGAGACAATAAAGCTT | 4 | ND |

| | Genbank accession number | Gene Symbol | Probe Sequence | Intensity group | STD order |
|---|---|---|---|---|---|
| 720 | NM_006362 | STX5 | TAAACTACCCGAAGGACTTAGGTGCTTTGTGTACTTAACCCCAGGACCTCCTTACTTTTT | 5 | ND |
| 721 | NM_019071 | ING3 | TCAACAGGTATATTCTGCTGCATGTACTGTACTCCAGAGCTGTTATGTAACACTGTATAT | 3 | ND |
| 722 | NM_013444 | UBQLN2 | TACAGTGTAACATTGTGTCAACATTTGCAGATTGACTGTATATGACCTTAATCTTTGTGC | 4 | ND |
| 723 | NM_015669 | PCDHB5 | GATCTAGAATTCGAGAGTGTCATGGACAAAAATTTCACCTTGAGATTGAGCTTTTATTTC | 1 | ND |
| 724 | NM_001883 | CRHR2 | TATTTCAACTCCTTCCTGCAGTCGTTCCAGGGTTTCTTCGTGTCTGTCTTCTACTGCTTC | 3 | ND |
| 725 | NM_015322 | FEM1B | CCCAGAACTCTTGAAGAGTTTGTTGGATTTCATTAAGTGACTGGATATGTAAAGTCGTTT | 2 | ND |
| 726 | NM_003317 | TITF1 | CTGCTCCACGCGCTTCGACTTTTCTTAACAACCTGGCCGCGTTTAGACCAAGGAACAAAA | 2 | ND |
| 727 | NM_016157 | TRO | CCCCATGTTTACAGATACCGCTAATAAATTGCAGTAGTCCTTCCCATGGAGCCAAAGTAC | 3 | ND |
| 728 | NM_006570 | RRAGA | GGTTCGTGTGTGTTCTCATTACCTGGTTATGATAGATATGCACATCAAAGCCTTTACCAG | 5 | ND |
| 729 | NM_005023 | PGGT1B | TGAATGTAAGCACACGGACTTCTGAACGCCTTCTAGATCTCCATCAAAGCTGGAAAACCA | 2 | ND |
| 730 | NM_020482 | FHL5 | AATCACAAAGACAACTCTCCTTGCAAAATACTGCACTCTGCTGTTAGAAGCAGAGGAAAA | 1 | ND |
| 731 | NM_000889 | ITGB7 | CACCCTACTTCATTTTCAGAGTGACACCCAAGAGGGCTGCTTCCCATGCCTGCAACCTTG | 5 | ND |
| 732 | NM_013292 | MYLPF | TGAGGATGTGATCACCGGAGCCTTCAAGGTCTTGGACCCTGAGGGAAAGGGCACCATCAA | 1 | ND |
| 733 | NM_012460 | TIMM9 | TTGAGGCCTTATGATTCAGCAGCTTGGTCACTTGATTAGAAAAATAAACCATTGTTTCTT | 5 | ND |
| 734 | NM_005481 | THRAP5 | CATGCTCAAGTCGCCCAACAGAACCACGGCGGTGAAGCAGTGGGAGCAGCGCTGGATCAA | 3 | ND |
| 735 | NM_007374 | SIX6 | CTGCCCAGATTCTCCCATGGGTATTTCACGTCGAAAGGACGCTGTTACATATGTATAACT | 1 | ND |
| 736 | NM_018273 | TMEM143 | AGACAGAGAAAGCTGTAGTCCTAAAAAGAGGCTGGGTTCATGGTCGGTGAAACAAAGACA | 2 | ND |
| 737 | NM_006869 | CENTA1 | TTCCAGGATGCTTCTCTGGAACCTCAAGGCAGGCAGCCCAGGCCCTGGGCCTGATCTCTA | 4 | ND |
| 738 | NM_000857 | GUCY1B3 | AAAGTTTGGCTTTTGATGTGGATGATGTGAGCTTCATGTGTCTTAAAATCTACTACAAGC | 1 | ND |
| 739 | NM_013449 | BAZ2A | GCAATTTTCCCTTGGTATAAGATGTGCTAGATTAATTTCATTGTGAGGTGGATGGGGGAG | 2 | ND |
| 740 | NM_006992 | LRRC23 | TGCAGATTGCTAGTTTTGCTTATAACCAGATTACTGACACTGAAGGCATCTCTCATCCTC | 2 | ND |
| 741 | NM_005188 | CBL | TCCATGAGTATGAATAGCAGCCCATTAGTAGGTCCAGAGTGTGACCACCCCAAAATCAAA | 3 | ND |
| 742 | NM_005452 | WDR46 | GCCATCTGCCCTGGACAGATTTGTGCGCTGAGCCAGACTCCAGGGTTGCCTGGGAACAGT | 5 | ND |
| 743 | NM_020689 | SLC24A3 | TTGATTCTGTTGCACATTTTGCACTGGTTTATGGCGATTGTTTTCTTGGACGGATAGTGT | 4 | ND |

| | Genbank accession number | Gene Symbol | Probe Sequence | Intensity group | STD order |
|---|---|---|---|---|---|
| 744 | NM_004810 | GRAP2 | CTTTCTGTGGACTGAGAAGTTTCCATCCCTAAATAAGCTGGTAGACTACTACAGGACAAA | 1 | ND |
| 745 | NM_007223 | GPR176 | TTTGGCTTTGGGCCTTTTGAGTTGCCTCCTCAGTGGCTCTCAGAGACCCGAAACAGCAAG | 2 | ND |
| 746 | NM_000032 | ALAS2 | CAGGCCTACTCCTGTCTTCTGCTTTGTTGTGTGCCTCTAGCTGAATTGAGCCTAAAAATA | 4 | ND |
| 747 | NM_003976 | ARTN | AGCCTAAAAGACACCAGAGACCTCAGCTATGGAGCCCTTCGGACCCACTTCTCACAGACT | 4 | ND |
| 748 | NM_003446 | ZNF157 | GTTGTACAATGAAGAAAGCCTCTCACTGAAGACTTCCCTCACCATTGGATCAAGCTCCTT | 3 | ND |
| 749 | NM_004238 | TRIP12 | TTTGCTGTGTGAAATTTAAAAAAGGGATGTTTTTCCAGGCTGGAACAATAAATGTGGCTG | 5 | ND |
| 750 | NM_004706 | ARHGEF1 | CAGGAAGGCCTTTTGCAAGAAGGAGAGGAATGGGGGAGAGGACGTGAGGGACCACCCCCA | 1 | ND |
| 751 | NM_005286 | NPBWR2 | CCTCCCCACGATGGGTGCCAACGTCTCTCAGGACAATGGCACTGGCCACAATGCCACCTT | 4 | ND |
| 752 | NM_020249 | | TTAATCATAAAGCTATACTAGCTCACATCTGAAGTCAACATGAAGTTTCCTACTTCCTTG | 1 | ND |
| 753 | NM_004429 | EFNB1 | GAGAGGGAGCAGAACAGCCAGCCCCTTCCAGGTGGCAGTCGGAAGGG TTTTTGTTTTTGT | 4 | ND |
| 754 | NM_018011 | FLJ10154 | GCCAAAACTGTCCTTCTCATTAAAAACCCAGGATTAAATTGCAAACTCTGAACTTTTTAC | 3 | ND |
| 755 | NM_001454 | FOXJ1 | TTCGGTGGAGCAGGCTGCCGACAGCCTGGACTTCGATGAGACCTTCCTGGCCACATCCTT | 2 | ND |
| 756 | NM_006660 | CLPX | GGACGGTTGCCTGTGGTGGTTCCATTGCATAGCCTAGATGAGAAACACTTGTACAAATA | 4 | ND |
| 757 | NM_007037 | ADAMTS8 | CACCAATTATGGCTACAATGACATTGTCACCATCCCAGCTGGTGCCACTAATATTGACGT | 1 | ND |
| 758 | NM_012409 | PRND | CTGCATCAATGCCACCCAGGCGGCGAACCAGGGGGAGTTCCAGAAGCCAGACAACAAGCT | 1 | ND |
| 759 | NM_014424 | HSPB7 | TATATAGATGGGGTTTTTCCAATACAGCTGGTTCGTGATAAACTGCATGAAACTCCTGCC | 1 | ND |
| 760 | NM_021018 | HIST1H3F | AAGCCCCACCGCTACAGGCCTGGTACTGTCGCCCTCCGTGAAATCCGCCGCTATCAGAAA | 5 | ND |
| 761 | NM_000542 | SFTPB | GGCTCCTGGCTGGACAGGGAAAAGTGCAAGCAATTTGTGGAGCAGCACACGCCCCAGCTG | 1 | ND |
| 762 | NM_003048 | SLC9A2 | TGTGTTTCCTCGGAAAAAATTGTTTATTACGGCTGCCATTGTTGTCATATTCTTTACTGT | 1 | ND |
| 763 | NM_001234 | CAV3 | GGAATGCTGCATTTTGTTCGTGCCTGTAAGATTGGTTTGTGTCCTGACCAGCTCCAAAAA | 1 | ND |
| 764 | NM_018029 | PPP4R2 | ATCTTGAGGGGGGAGTGTTGGGTGGAATCATAGATCCATGCACTCCTAACATGAACTAAT | 3 | ND |
| 765 | NM_003562 | SLC25A11 | TCCATGCCTGTGGACATTGCCAAGACCCGAATCCAGAACATGCGGATGATTGATGGGAAG | 3 | ND |
| 766 | NM_014466 | TEKT2 | CTTCACACGTACCACAAATAGCACCCTGAGTCCACTCAAAAGCTGCCAGCTGGAGCTGGC | 2 | ND |
| 767 | NM_000823 | GHRHR | ACAGTGAAGATTATCTACACCGTGGGCCATAGCATCTCTATTGTAGCCCTCTTCGTGGCC | 1 | ND |

| | Genbank accession number | Gene Symbol | Probe Sequence | Intensity group | STD order |
|---|---|---|---|---|---|
| 768 | NM_017590 | ZC3H7B | AATTTAATGGTCCTTTAAAATGTCTGTGTATTAAAAATTTAAGAATACCACACTTTAATA | 4 | ND |
| 769 | NM_001805 | CEBPE | GCAAGAGCCGAGACAAGGCCAAGAGGCGCATTCTGGAGACGCAGCAGAAGGTGCTGGAGT | 4 | ND |
| 770 | NM_003016 | SFRS2 | TATTTTTGTGCACTAGGCGCAGTTGTGTAGCAGTTGAGTAATGCTGGTTAGCTGTTAAGG | 5 | ND |
| 771 | NM_018572 | | GGTGTATGTGTCCAGGAATTTATCCATTTCTTTTGGATTTTATAGTTCCTGTCGCTTGCC | 4 | ND |
| 772 | NM_002032 | FTH1 | TGCATGCATGTTGGGGTTTCCTTTACCTTTTCTATAAGTTGTACCAAAACATCCACTTAA | 5 | ND |
| 773 | NM_006678 | CD300C | ACCAAAGGGTCAGCAGGGAAAAGGAATGGCCGAGTGTCCATCAGGGACAGTCCTGCAAAC | 2 | ND |
| 774 | NM_018023 | YEATS2 | AGCAGTCTCCACGTGTGTTAATGTTTCAAACGTGTATCATAATGTGTATAATTGTGTAAC | 4 | ND |
| 775 | NM_000174 | GP9 | GTGGCCGCGCTGGGCCTGGCTCTTCTGGCTGGCCTGCTGTGTGCCACCACAGAGGCCCTG | 5 | ND |
| 776 | NM_001292 | CLK3 | GGGATGAGAACAGCTCTGACGGCCGGTATGTGAAGGAGAACTGCAAACCTCTGAAGAGTT | 3 | ND |
| 777 | NM_002429 | MMP19 | ACCAGTACTGCAGGATTGTTGCACTAAATGAAATACTGTATGTGAAGTGCCTGGCACAGT | 2 | ND |
| 778 | NM_018038 | | CTAGTCTTGATAGATGTATAATTATTACATATCCACCATCACTTCATTTAGCTGGGCAAC | 1 | ND |
| 779 | NM_000015 | NAT2 | AGACGTCTCCAACATCTTCATTTATAACCACATCATTTTGTTCCTTGCAGACCCCAGAAG | 2 | ND |
| 780 | NM_005850 | SF3B4 | CTTGGACCAATCAGAGATGCTGTAGCTCCTTGGGGCAAAGGTACTAATCCCTTTCAGCAC | 3 | ND |
| 781 | NM_004656 | BAP1 | CATGTTGACATAAGTTCCTACCTGACTATGCTTTCTCTCCTAGGAGCTGTCCTGGTGGGC | 3 | ND |
| 782 | NM_012244 | SLC7A8 | GGATGGAGTTAGAACCTTAATGATAATTTCTTTCGTTTGGTGTAGGTTTTAGAGATTTGT | 5 | ND |
| 783 | NM_002140 | HNRPK | TGGGGAGCAGTACTACAAGTTGAGTAATGGTATGAGTATATACCAGAATTCTGATTGGCA | 5 | ND |
| 784 | NM_000452 | SLC10A2 | GGTACAGACTTCCTTCTTAGAGAGTGTCAGAGAATATGCTCCCAATGGTGGAAAGGAAGA | 1 | ND |
| 785 | NM_021098 | CACNA1H | GCGGCCCAATGTCACCTTCACTCACAGTCTGAGTTCTTGTCCGCCTGTCACGCCCTCACC | 5 | ND |
| 786 | NM_006559 | KHDRBS1 | ATTTGAGATTCTGCACTCCATGAAAAGTTCACTTGGACGCTGGGGCCAAAAGCTGTTGAT | 5 | ND |
| 787 | NM_014761 | KIAA0174 | CACCTCAGCATCTGAAGACATTGACTTTGATGATCTTTCCCGGAGGTTTGAAGAGCTGAA | 2 | ND |
| 788 | NM_020905 | RDH14 | CAATGTTTGGTGTTTGTGTGGAAATTATCTGCCTGGTGTGTGCACACAAGTCTTACTTGG | 5 | ND |
| 789 | NM_001817 | CEACAM4 | TGTTCCAAAACATCACCCTGGAGGACGCAGGATCCTACACCCTACGAACCATAAATGCCA | 1 | ND |
| 790 | NM_017803 | DUS2L | CCAGGGGTGGATCCTGGCCCCTTTGGTGGATCTGAGTGACAGGGTCAAGTTCTCTTTGAA | 4 | ND |
| 791 | NM_014046 | HLA-E | GGGCTGGGCTAAACATTGTTGCCGTTTCATACTTCTACCAACTCAGCTTTTACACAATAA | 5 | ND |

| | Genbank accession number | Gene Symbol | Probe Sequence | Intensity group | STD order |
|---|---|---|---|---|---|
| 792 | NM_017907 | C11orf59 | CAGCCTCACTGCGGCTTATACAGTACCCTAACCTGCTACTAATCACAGAGAAAAATGTGA | 4 | ND |
| 793 | NM_014952 | BAHD1 | AGGAGGTAGGGTCTTGGCTGCCCCGAACTTAAATGCTTTTGAAATCTCTTAGATGTGGAA | 5 | ND |
| 794 | NM_007169 | PEMT | CTGGGGTTCGCTGGAACTTTCCTAGGTGATTACTTCGGGATCCTCAAGGAGGCGAGAGTG | 3 | ND |
| 795 | NM_020393 | PGLYRP4 | GAGTTTCTCCAGGGGAGGAACTGTGTTTTATTCATCTCTATGTCCTCTGTTTCTCAGCAG | 1 | ND |
| 796 | NM_002042 | GABRR1 | CCATGTTTTCACTATCCCTTCTGCAGCTTTCCAAAGCTACATTGACGAGACACTTACTGG | 1 | ND |
| 797 | NM_001065 | TNFRSF1A | AGGGGCGAGCACGGAACAATGGGGCCTTCAGCTGGAGCTGTGGACTTTTGTACATACACT | 5 | ND |
| 798 | NM_018026 | PACS1 | TTCCTCATCATCCCCCTCGGTTCTCACCCTGTGGCCAAATACTTGGGGTCAGTCGACAGT | 5 | ND |
| 799 | NM_001507 | MLNR | AACTTTTCTATCTGAGCGCATCTATCAACCCAATCCTCTACAACCTCATTTCAAAGAAGT | 1 | ND |
| 800 | NM_001862 | COX5B | CATCTGTGAAGAGGACAATACCAGCGTCGTCTGGTTTTGGCTGCACAAAGGCGAGGCCCA | 5 | ND |
| 801 | NM_016037 | UTP11L | GAGTCGTCGAAAACGTTGACGTGTTATAGATAAGCCTTGTCATTCTGTATCAAAAATCTG | 4 | ND |
| 802 | NM_005801 | EIF1 | CCTCACAGCTTGTATAATGTAACCATTTGGGGTCCGCTTTTAACTTGGACTAGTGTAACT | 5 | ND |
| 803 | NM_014080 | DUOX2 | GTCCTACTAAATATACTCTTTTGAGACTGGCCTCTTTTACTCACCATAATGCCTTTGTAA | 2 | ND |
| 804 | NM_012368 | OR2C1 | CTCAGCCAGCTATGGGTATCTGCTTCCGGCCAAGAACAGCAAACAGGACCAGGGCAAGTT | 1 | ND |
| 805 | NM_005119 | THRAP3 | TTCATTTCATTTGGAGCTAAGATGACTAATTTGATGATTTTCGATCTCTTTTCCCCTGTC | 2 | ND |
| 806 | NM_000486 | AQP2 | GTGTTCATCCCCAAGTTCTCTTTTGTCCTCACATGCAGAGTTGTGCATGCCCCTGAGTGT | 3 | ND |
| 807 | NM_021080 | DAB1 | CTCCCTCACCTGTACCTCAGAGGCCTTCTCCAGTTACTTCAACAAAGTCGGGGTGGCACA | 4 | ND |
| 808 | NM_021114 | SPINK2 | TTTACATGAGATTTGTTAACACACATTTTCTGAGAGCAGGTATGGAAGACAGCCATGTGT | 1 | ND |
| 809 | NM_006600 | NUDC | ATGGGGCTGCCAACTTCAGACGAACAGAAGAAACAGGAGATTCTGAAGAAGTTCATGGAT | 5 | ND |
| 810 | NM_007363 | NONO | TGGGGAAACCATGGCAAAGTGGATCCAGTTAGAGCCCATTAATCTTGATCATTCCGGTTT | 3 | ND |
| 811 | NM_006183 | NTS | AAATGTTTGCAGTCTTGTAAATAATTTGAACAGCCCAGCTGAGGAAACAGGAGAAGTTCA | 2 | ND |
| 812 | NM_017660 | GATAD2A | AAAGGATCAGGTCTGCTTTTAGTTTCATTTTTTGTTTCTTTCCCGTCCCACTCTTTAAAAA | 4 | ND |
| 813 | NM_003922 | HERC1 | TTTGGTCACTTTTGATAAGTTTGCATGAAACCATTTTGGTGCATTTTTTAGTTGGGAATGG | 3 | ND |
| 814 | NM_002872 | RAC2 | CACTTACCTGTGAGAGTCTTCAAACTTTTAAACCTTGCCAGTCAGGACTTTTGCTATTGC | 5 | ND |
| 815 | NM_006833 | COPS6 | TACCTCGGCACCATCACCAAAACGTGCAACACCATGAACCAGTTTGTGAACAAGTTCAAT | 5 | ND |

| | Genbank accession number | Gene Symbol | Probe Sequence | Intensity group | STD order |
|---|---|---|---|---|---|
| 816 | NM_014052 | | TATTCTGCCTAACGTTTGCTTCTGTGATGGTTATATTGCCTAGCAAGCACACCCGTGGTT | 5 | ND |
| 817 | NM_012191 | HYAL1 | CATCCAGGGCAAGGAACTGTCTTTCTGGTTCAATAGACTGCCCCGACAGTCTACAAGCCT | 1 | ND |
| 818 | NM_001997 | FAU | CCCGTGCTGGAAAAGTGAGAGGTCAGACTCCTAAGGTGGCCAAACAGGAGAAGAAGAAGA | 5 | ND |
| 819 | NM_002169 | IFNA5 | ACTTTCAAAGAATCACCCTCTATCTGACAGAGAAGAAATACAGCCCTTGTGCATGGGAGG | 1 | ND |
| 820 | NM_014021 | SSX2IP | CAGATGATTGTTTGCTAGATTTTGTTTTCTACAATCAAAATGTTGACCTGCAAAGCAGTG | 1 | ND |
| 821 | NM_005030 | PLK1 | CGGCAGCGTGCAGATCAACTTCTTCCAGGATCACACCAAGCTCATCTTGTGCCCACTGAT | 1 | ND |
| 822 | NM_001414 | EIF2B1 | ACATTCCTCAGCAGGATCAAGCCAAACAGTAAAAACTACCAAGAGAACACGAGGAAGGCA | 4 | ND |
| 823 | NM_018428 | UTP6 | AAGTGGTGGCTACAAAAAGGCCAGAGCTGTGTTTAAAAGTTTACAGGAGAGCCGACCATT | 4 | ND |
| 824 | NM_000588 | IL3 | AATGAATTCCGGAGGGAAACTGACGTTCTATCTGAAAACCCTTGAGAATGCGCAGGCTCAA | 1 | ND |
| 825 | NM_005430 | WNT1 | GGCACAGCAGGCACGGCAGGGCGCGCCTGTAACAGCTCGTCGCCCGCGCTGGACGGCTGC | 1 | ND |
| 826 | NM_016340 | RAPGEF6 | CTTTTCCAGCCTCATGGGTATGGCACTCTTAATTAAAATTTCAGTGACTGTTTACTGGAT | 4 | ND |
| 827 | NM_002517 | NPAS1 | GGGCCCGCGGGCACCAGGCTGCCGCGGAAGGGGGACTGAGGACTGGCAGAGCTGCCGGCG | 4 | ND |
| 828 | NM_004590 | CCL16 | AGTGATGACCAGGCTTTAGTGGAAGCCCTTGTTTACAGAAGAGAGGGGTAAACCTATGAA | 1 | ND |
| 829 | NM_016014 | C9orf77 | AGGTTGAAACAGTTTGTGTCACAGGAACTGGTGCAGAAACATAAAGAAGGGAAGTGATTT | 1 | ND |
| 830 | NM_002695 | POLR2E | AGGAGGAGGTGACAGAGCTGCTGGCCCGATATAAGCTCCGAGAGAACCAGCTGCCCAGGA | 5 | ND |
| 831 | NM_016093 | RPL26L1 | TACAAAGGTCAGCAAATTGGCAAAGTAGTCCAGGTTTACAGGAAGAAATATGTTATCTAC | 5 | ND |
| 832 | NM_017506 | OR7A5 | TCCTCCTGATAATGTAGACCTGAACATACTTGTGTATTTTGCAGCTGGTCATGAGATTGT | 1 | ND |
| 833 | NM_014231 | VAMP1 | CAGGAGCATCACAATTTGAGAGCAGTGCTGCCAAGCTAAAGAGGAAGTATTGGTGGAAAA | 1 | ND |
| 834 | NM_000481 | AMT | GGAATGATTGCTGACTCACAGTAGGGCTGCTATGCCTGTGTGTAAACTTGGGGATGGCTG | 2 | ND |
| 835 | NM_005182 | CA7 | TAGCCGGCCACTAGGGCACCATCTTCTCAAGGGCTTCCATGTCAGCAGACACCAAACCAT | 1 | ND |
| 836 | NM_004501 | HNRPU | GAACCTCAGCATTGTGCACGATAAGAGAATGTGTCAGTATTTCAGGGTTCTACATTTTAT | 5 | ND |
| 837 | NM_000942 | PPIB | CAAAAACAGCAAATTCCATCGTGTAATCAAGGACTTCATGATCCAGGGCGGAGACTTCAC | 5 | ND |
| 838 | NM_013399 | C16orf5 | CATGGCCTCCTGTCACTGTGAATCGTGGCCCAGTCTCAGTTTGTAGTTTCTCATTAAATT | 4 | ND |

| | Genbank accession number | Gene Symbol | Probe Sequence | Intensity group | STD order |
|---|---|---|---|---|---|
| 839 | NM_013391 | DMGDH | TGGTATTGACCGAACCAACCAGAAACCGGCTTCAGAAAAAAGGTGGAAAGGACAAAACTT | 2 | ND |
| 840 | NM_002981 | CCL1 | GAGAGGCAAAGAGGCCTGCGCCTTGGACACAGTTGGATGGGTTCAGAGGCACAGAAAAAT | 1 | ND |
| 841 | NM_001384 | DPH2 | TAATTAAGATTAAAAGCTCAGTTTCTCAGTCACATTAGTCATTCAAGTGTTCAGACAGCC | 4 | ND |
| 842 | NM_004389 | CTNNA2 | GTGAAAAATCTGGAAGTGTAATGGTAGAACATAAAACTTGTATTGCTTCTGTTTCAGTGC | 1 | ND |
| 843 | NM_016552 | ANKMY1 | CAGGCTGTCTTTGCCAAGGAGAGCCAGTGGGACCCCACGTGGCTGTACCTGTGCAAGAGA | 1 | ND |
| 844 | NM_006188 | OCM | CAGAGGAATTCCAGGAAATGGTGCATTCTTAAAi4GCCCCAGTCTCTGGAGAAAAGAGAGA | 1 | ND |
| 845 | NM_003085 | SNCB | AATCACGAGATCTTCCTTCCGCTCTGAGGCAACCCCCTCGGAGCCTGTGTTAGTGTCTGT | 4 | ND |
| 846 | NM_002733 | PRKAG1 | ACTAAAGCCTTGCAACATCGATCACATTACTTTGAGGGTGTTCTCAAGTGCTACCTGCAT | 2 | ND |
| 847 | NM_005830 | MRPS31 | ATATATTTCTGGAGAAACACCTGGAGAGCTTTCCAAAACAAGGACCAATTCGCCACTTCA | 4 | ND |
| 848 | NM_002713 | PPP1R8 | CAAGCTTTGTACAGAGATTTGTACATTTGTGTAATAGGCCTTTTCATGCTTTATGTGTAG | 4 | ND |
| 849 | NM_006285 | TESK1 | TGGGGATCACTGAACCAGACACAGCATTGCTGACACATGAGACTAACACGTGCAATTATT | 5 | ND |
| 850 | NM_004875 | POLR1C | ATGCTGCTAAAGATTCCTCTGACCCCAACGAACTGTACGTGAACCACAAAGTGTATACCA | 3 | ND |
| 851 | NM_016333 | SRRM2 | TCCTCTTCATCATCGTCGTCGTCGTCCTCCTCCTCCTCTGGCTCCAGTTCTAGTGACTCA | 5 | ND |
| 852 | NM_017409 | HOXC10 | GCACTCACACACAGCATTCTGTTCTCCATGCAAAGTTAAGATCGAATCCATCCGCTTGTA | 4 | ND |
| 853 | NM_006402 | HBXIP | AGTGCACAAAATGGCCTCTTGATGCTCATATCTGTTCTTCAGCAGCCTGTCATAGGAACT | 5 | ND |
| 854 | NM_014892 | RBM16 | ATAGATATATTTCAGAATAGCAAGTGGTGGTATATCTTATCCATATCTTTAGGCTGCTGC | 4 | ND |
| 855 | NM_020904 | PLEKHA4 | AAGGAACACCACCCCTTACTTGCCGACTTCCGAAGGTCACCGGGAGCGGGTTCTCAGCCT | 5 | ND |
| 856 | NM_006777 | ZBTB33 | TCAACTATCAGTTTATGTCTTCACATATAAAGTCAGTTCATAGTCAAGATCCTTCTGGGG | 1 | ND |
| 857 | NM_004565 | PEX14 | GTTCATTTGTGTGATCATGTATAGACCTCAGAACGGAAGATAGGACTGTATATAATTGTA | 5 | ND |
| 858 | NM_003656 | CAMK1 | TGAACAGATTTTGAAGGCCGAGTACGAGTTTGACTCTCCTTACTGGGACGACATCTCTGA | 2 | ND |
| 859 | NM_013400 | KCNH2 | ATAATGAATGAATTCTGCTTTCCTATAATTTCTACCTATTGGGCCTTGTTCTGTTCTCTG | 5 | ND |
| 860 | NM_006058 | TNIP1 | ACACCCCTACCCCTCTGTGAGGAGCTGTGGGAAGTGTGGGTTTGTCTCCAGAACAGAAGA | 5 | ND |
| 861 | NM_020344 | SLC24A2 | AAACCTTCATCGAGGAAGTTTTTTTCCCATCACGTTCTTTGGCTCCATTACCTGGATTGCA | 5 | ND |
| 862 | NM_012198 | GCA | TGGTGGTGTTTGAGGGTTGGCTAGAAATGAAAGCCTGGATTTTGTGCCATGTTTGTAATA | 4 | ND |

| | Genbank accession number | Gene Symbol | Probe Sequence | Intensity group | STD order |
|---|---|---|---|---|---|
| 863 | NM_018527 | NARG1L | TGCTCTTGCTATTGTTTTTTAACCATCCCCTGCCAGAAACATATACACAGATACACAACC | 2 | ND |
| 864 | NM_012088 | PGLS | AGCACGAACTGTCATCTTTGTGGCAACTGGAGAAGGCAAGGCAGCTGTTCTGAAGCGCAT | 5 | ND |
| 865 | NM 006561 | CUGBP2 | TTTTTCTGATTCTTCTGTCCTCATTGTGAACATAACCGTGTAGTTGAAACAGTCAAACTT | 3 | ND |
| 866 | NM_016481 | C9orf156 | CCGAGATGGACCTTGGGCAGCTCAGTTCACAAGATGTTGATCAGGCGTCATTTAAATATT | 2 | ND |
| 867 | NM_006937 | SUMO2 | AGACGGGAGGTGTCTACTGAAAAGGGAACCTGCTTCTTTACTCCAGAACTCTGTTCTTTA | 5 | ND |
| 868 | NM_005826 | HNRPR | ATCGCTCAGCAGCCGCTTCAGCAAGGTGGTGACTATTCTGGTAACTATGGTTACAATAAT | 5 | ND |
| 869 | NM_000322 | RDS | GTTGGTCCCAAGTTTAATTAGATTTCTGAATCTCGTTGAGGCCAAGGAATGATCCATACT | 1 | ND |
| 870 | NM_005500 | SAE1 | TCAAGTCACTCAGAGGCTGTTGCATTTCAGGGCTATGTTGGTCCTTTGTTTACCTCCTAA | 5 | ND |
| 871 | NM_002768 | PCOLN3 | GGCTCACTGCATTTGGTTTTCTTTTCAGAACTTGGGAGCCCCCAGGGAGGGGCTAGTGTT | 5 | ND |
| 872 | NM_015884 | MBTPS2 | ACTCTGGATGGTTACAGCACGGTAATGTTTGCACTCATCTGACAGAATCCCTGAGTTACA | 1 | ND |
| 873 | NM_006092 | CARD4 | TGGCAAAGATAGAGAATGCCCTCAGCTCTTAGCTGGTCTAAGAATGACGATGCCTTCAAA | 2 | ND |
| 874 | NM_003103 | | GGCATTGAGTTGTGATATAGTTTTACTTTGATGTGCATTTTGAATTTCAGCTACACCTAG | 5 | ND |
| 875 | NM_001425 | EMP3 | TGTTCATGTTCCAGCTCTACACCATGCGACGAGGAGGTCTCTTCTATGCCACCGGCCTCT | 4 | ND |
| 876 | NM_005549 | KCNA10 | GACTTCTTCAGGAACATCATGAACATCATTGACATCATCTCCATTATCCCCTACTTTGCA | 1 | ND |
| 877 | NM_006917 | RXRG | GGTTCTTCAAGAGGACGATAAGGAAGGACCTCATCTACACGTGTCGGGATAATAAAGACT | 1 | ND |
| 878 | NM_006272 | S100B | ATGGTTACTACTGCCTGCCACGAGTTCTTTGAACATGAGTGAGATTAGAAAGCAGCCAAA | 3 | ND |
| 879 | NM_004083 | DDIT3 | GGAAGTGTATCTTCATACATCACCACACCTGAAAGCAGATGTGCTTTTCCAGACTGATCC | 1 | ND |
| 880 | NM_016039 | C14orf166 | TCACATTCTCGGGGGAGGAAGCCCAGAAAATTGGGTATGTTCTAGAGATTTACCACCATT | 5 | ND |
| 881 | NM_004212 | SLC28A2 | TCTACAACAATACCGTCTGTGCCTAAGGCTGCTTGATCTATTTCTATAACAGTTTTGATC | 1 | ND |
| 882 | NM_017435 | SLCO1C1 | ACAATTAACTCATACCTTGGGTTCCTTCAAGTATTACTCCTATAGTATTTTCTCCCATAG | 1 | ND |
| 883 | NM_003355 | UCP2 | GGTTCCTGGAACGTGGTGATGTTCGTCACCTATGAGCAGCTGAAACGAGCCCTCATGGCT | 3 | ND |
| 884 | NM_015711 | GLTSCR1 | TCCAGCCGCCCGCGCCAGATTTTGAAATCTCGGAGACAAAACTAGTACTGTAAGATAAAT | 5 | ND |
| 885 | NM_006253 | PRKAB1 | TGAGGTTTTACAATTGTTTCTTACAGTCATGTGCACTAAGTACTCTTTTTGTAAGCAGAG | 2 | ND |
| 886 | NM_003408 | ZFP37 | ATGTCACACCTTGTTGTACATGAGAAACTTTGAAAATGGATCTTCATATAGAATCAGTGG | 1 | ND |

EP 2 084 296 B1

(continued)

| | Genbank accession number | Gene Symbol | Probe Sequence | Intensity group | STD order |
|---|---|---|---|---|---|
| 887 | NM_014223 | NFYC | ACTTGCCACCAATGCTCAACAGATTACACAGACAGAGGTCCAGCAAGGACAGCAGCAGTT | 2 | ND |
| 888 | NM_019062 | RNF186 | TTGGGATAGCATAGCTAAAACTGTTGGTGGCTAGGGGAGCAGACACAAACTAGTTGAACA | 1 | ND |
| 889 | NM_000671 | ADH5 | GCCAAATTGCTCTTCAAAGTAAATGTGAGTTTTTGTGAATTACATGAGTATGGAATGGTG | 3 | ND |
| 890 | NM_006011 | ST8SIA2 | ATCAAAAGACCCACCACCGGCCTCTTGATGTATACCCTGCCACACGTTTCTGCAAACAA | 5 | ND |
| 891 | NM_004040 | RHOB | GCGCTGCAGAAGCGCTACGGCTCCCAGAACGGCTGCATCAACTGCTGCAAGGTGCTATGA | 2 | ND |
| 892 | NM_006773 | DDX18 | TTGTAGACTTTAGAATTTGGACTTACCTAACAAGAGTATAAATTGACTGGGTTGCAAGC | 3 | ND |
| 893 | NM_000605 | IFNA2 | AGCCTAAGGTTTAGGCTCACCCCATTTCAACCAGTCTAGCAGCATCTGCAACATCTACAAT | 1 | ND |
| 894 | NM_000203 | IDUA | ACATACGAGATCCAGTTCTCTCAGGACGGTAAGGCGTACACCCGGTCAGCAGGAAGCCA | 2 | ND |
| 895 | NM_003492 | CXorf12 | AACTTACTTTCAAAGACATAAAGCACAGATCTCCGCACAGGGGATGTGTGTTCCTGAT | 4 | ND |
| 896 | NM_004805 | POLR2D | CAGCCCATTTGCTGTATGAACTGTGGTTGTGTGCCCAATGACAAGGCTACTAAGAAA | 3 | ND |
| 897 | NM_003948 | CDKL2 | GATGGGATTTGCTGAGAGGTTTTCCCAAGAACTACAGTTAAAAGTACAGAAAGATGCCAGA | 1 | ND |
| 898 | NM_000777 | CYP3A5 | AGGATTTCTACTTTGGTCTTCAAGAAAGCTGTGCCCCAGAi4CACCAGAGATTTCAACTTA | 1 | ND |
| 899 | NM_002167 | ID3 | AGCCAGGTGGAAATCCTACAGCGCGGTCATCGACTACATTCTCGACCTGCAGGTAGTCCTG | 4 | ND |
| 900 | NM_004413 | CDK10 | GGCAGGATGCCTGGGGACAGTTCAGGACACACACAGTAGGCCCGCAATAAAAGCAACA | 2 | ND |
| 901 | NM_016041 | DERL2 | GAAGGACTCGGTGATACCCACTGGGATCTTTTATCCTTTGTTGCAAAAGTGTGGACACTT | 4 | ND |
| 902 | NM_015962 | C14orf111 | GGATCCAAGATTTGAACGATTACCATGTACACACAAAGGAACCTATGCAGATGACTGCTT | 1 | ND |
| 903 | NM_006934 | SLC6A9 | GCCATCATGTACATCTACGGGCACCGGAACTACTTCCAGGACATCCAGATGATGCTGGGA | 1 | ND |
| 904 | NM_000559 | HBG1 | ACTGAGCTCACTGCCCATGATGCAGAGCTTTCAAGGATAGGCTTTATTCTGCAAGCAATA | 1 | ND |
| 905 | NM_001305 | NCF1 | GGAAGTCCTGGGGTTTTCCTCTCTTTGTGGTTTTCTGTTTTGTAATTTAAGAAGA | 5 | ND |
| 906 | NM_007039 | PTPN21 | AGAGGGGTTTCTAACCTGGGAAAGGTGCTCAAGGAGGACTTGGTTTCAAGGGCCTTGCCC | 2 | ND |
| 907 | NM_005423 | TFF2 | TCCAACTTCATCTTTGAAGTGCCCTGGTGCTTCTTCCCGAAGTCTGTGGAAGACTGCCAT | 3 | ND |
| 908 | NM_021221 | LY6G5B | TCAAGGTTCGCTTCATCGTTCGAGGTGTGGACGTACATTTCCTACCGCTGCCAAGAAA | 1 | ND |
| 909 | NM_016063 | HDDC2 | CAGACTGCTTCAGACTTCTAATCATAGGCTTGTAAACCTACTAATAGGCTCTGCCCCTCT | 1 | ND |
| 910 | NM_020406 | | CTTGGACACCAGATTCTTTCCCATTCTGTCCATGAATCATCTTCCCCACACAATCATT | 2 | ND |

(continued)

| | Genbank accession number | Gene Symbol | Probe Sequence | Intensity group | STD order |
|---|---|---|---|---|---|
| 911 | NM_003089 | SNRP70 | GAATACACATGGTCTACAGTAAGCGGTCAGGAAAGCCCCGTGGCTATGCCTTCATCGAGT | 3 | ND |
| 912 | NM_003470 | USP7 | GACCACTTCAACAAAGCCCCAAAGAGGAGTCGCTACACTTACCTTGAAAAGGCCATTAAA | 4 | ND |
| 913 | NM_001685 | ATPSJ | TACAAATCTAAGCGACAGACATCTGGAGGACCTGTTGATGCTAGTTCAGAGTATCAGCAA | 5 | ND |
| 914 | NM_003411 | ZFY | GCTTCCGAAGAGACCTTCAGAAAAGAACCAGCACATAATGAGACACCATAAAGAAGTTGGTC | 1 | ND |
| 915 | NM_000118 | ENG | GACTGTCTTCATGCGCTTGAACATCATCAGCCCTGACCTGTCTGGTTGCACAAGCAAAGG | 3 | ND |

Table 2

| | | GenBankAccession# | Symbol | Description |
|---|---|---|---|---|
| | 1 | AL080059 | TSPYL5 | TSPY-like 5 |
| | 2 | AW014921 | | CDNA FLJ41489 fis, clone BRTHA2004582 |
| | 3 | AI813331 | DIAPH3 | Diaphanous homolog 3 (Drosophila) |
| | 4 | NM_016359 | NUSAP1 | Nucleolar and spindle associated protein 1 |
| | 5 | AA555029 | LOC286052 | Hypothetical protein LOC286052 |
| | 6 | NM_003748 | ALDH4A1 | Aldehyde dehydrogenase 4 family, member A1 |
| | 7 | AI554061 | QSCN6L1 | Quiescin Q6-like 1 |
| | 8 | NM_003862 | FGF18 | Fibroblast growth factor 18 |
| | 9 | AA992378 | DIAPH3 | Diaphanous homolog 3 (Drosophila) |
| | 10 | AA404325 | PQLC2 | PQ loop repeat containing 2 |
| | 11 | U82987 | BBC3 | BCL2 binding component 3 |
| | 12 | AL137718 | DIAPH3 | Diaphanous homolog 3 (Drosophila) |
| | 13 | AB037863 | RP5-860F19.3 | KIAA1442 protein |
| | 14 | NM_020188 | C16orf61 | Chromosome 16 open reading frame 61 |
| | 15 | NM_020974 | SCUBE2 | Signal peptide, CUB domain, EGF-like 2 |
| | 16 | NM_000127 | EXT1 | Exostoses (multiple) 1 |
| | 17 | NM_002019 | FLT1 | Fms-related tyrosine kinase 1 (vascular endothelial growth factor/vascular permeability factor receptor) |
| | 18 | NM_002073 | GNAZ | Guanine nucleotide binding protein (G protein), alpha z polypeptide |
| | 19 | NM_000436 | OXCT1 | 3-oxoacid CoA transferase 1 |
| | 20 | NM_004994 | MMP9 | Matrix metallopeptidase 9 (gelatinase B, 92kDa gelatinase, 92kDa type IV collagenase) |
| | 21 | AI918032 | RUNDC1 | RUN domain containing 1 |
| | 22 | AI283268 | | CDNA: FLJ22719 fis, clone HSI14307 |
| | 23 | AI738508 | ECT2 | Epithelial cell transforming sequence 2 oncogene |
| | 24 | NM_003875 | GMPS | Guanine monphosphate synthetase |
| | 25 | NM_006101 | KNTC2 | Kinetochore associated 2 |
| | 26 | NM_003882 | WISP1 | WNT1 inducible signaling pathway protein 1 |
| | 27 | NM_003607 | CDC42BPA | CDC42 binding protein kinase alpha (DMPK-like) |
| | 28 | AF073519 | DKFZP686P18101 | Similar to TFIIH basal transcription factor complex p44 subunit (Basic transcription factor 2 44 kDa subunit) (BTF2-p44) (General transcription factor IIH polypeptide 2) |
| | 29 | AF052162 | AYTL2 | Acyltransferase like 2 |
| | 30 | NM_000849 | GSTM3 | Glutathione S-transferase M3 (brain) |
| | 31 | AI377418 | GPR180 | G protein-coupled receptor 180 |
| | 32 | NM_016577 | RAB6A | RAB6A, member RAS oncogene family |
| | 33 | AI694320 | ZNF533 | Zinc finger protein 533 |
| | 34 | AA528243 | RTN4RL1 | Reticulon 4 receptor-like 1 |

(continued)

| | | GenBankAccession# | Symbol | Description |
|---|---|---|---|---|
| | 35 | NM_015984 | UCHL5 | Ubiquitin carboxyl-terminal hydrolase L5 |
| | 36 | NM_006117 | PECI | Peroxisomal D3,D2-enoyl-CoA isomerase |
| | 37 | AK000745 | MTDH | Metadherin |
| | 38 | AI224578 | | Full-length cDNA clone CS0DI029YM01 of Placenta Cot 25-normalized of Homo sapiens (human) |
| | 39 | NM_003239 | TGFB3 | Transforming growth factor, beta 3 |
| | 40 | NM_014791 | MELK | Maternal embryonic leucine zipper kinase |
| | 41 | X05610 | COL4A2 | Collagen, type IV, alpha 2 |
| | 42 | NM_016448 | DTL | Denticleless homolog (Drosophila) |
| | 43 | NM_018401 | STK32B | Serine/threonine kinase 32B |
| | 44 | NM_000788 | DCK | Deoxycytidine kinase |
| | 45 | AI817737 | FBXO31 | F-box protein 31 |
| | 46 | AL080079 | GPR126 | G protein-coupled receptor 126 |
| | 47 | NM_006931 | SLC2A14 | Solute carrier family 2 (facilitated glucose transporter), member 14 |
| | 48 | AF257175 | PECI | Peroxisomal D3,D2-enoyl-CoA isomerase |
| | 49 | NM_014321 | ORC6L | Origin recognition complex, subunit 6 like (yeast) |
| | 50 | NM_002916 | RFC4 | Replication factor C (activator 1) 4, 37kDa |
| | 51 | AI992158 | CDCA7 | Cell division cycle associated 7 |
| | | AW024884 | LOC643008 | PP12104 |
| | 52 53 | AF201951 | MS4A7 | Membrane-spanning 4-domains, subfamily A, member 7 |
| | 54 | NM_005915 | MCM6 | MCM6 minichromosome maintenance deficient 6 (MIS5 homolog, S. pombe) (S. cerevisiae) |
| | 55 | NM_001282 | AP2B1 | Adaptor-related protein complex 2, beta 1 subunit |
| | 56 | AI741117 | C9orf30 | Chromosome 9 open reading frame 30 |
| | 57 | NM_000599 | IGFBP5 | Insulin-like growth factor binding protein 5 |
| | 58 | NM_020386 | HRASLS | HRAS-like suppressor |
| | 59 | NM_014889 | PITRM1 | Pitrilysin metallopeptidase 1 |
| | 60 | AF055033 | IGFBP5 | Insulin-like growth factor binding protein 5 |
| | 61 | NM_006681 | NMU | Neuromedin U |
| | 62 | NM_007203 | PALM2-AKAP2 | PALM2-AKAP2 protein |
| | 63 | AI583960 | LGP2 | Likely ortholog of mouse D11lgp2 |
| | 64 | NM_003981 | PRC1 | Protein regulator of cytokinesis 1 |
| | 65 | AA834945 | LOC441921 | Transcribed locus |
| | 66 | NM_001809 | CENPA | Centromere protein A |
| | 67 | W90004 | EGLN1 | Egl nine homolog 1 (C. elegans) |
| | 68 | NM_004702 | | Data not found |
| | 69 | NM_007036 | ESM1 | Endothelial cell-specific molecule 1 |

(continued)

|  | | GenBankAccession# | Symbol | Description |
|---|---|---|---|---|
| | 70 | NM_018354 | C20orf46 | Chromosome 20 open reading frame 46 |
| | 71 | NM_000286 | PEX12 | Peroxisomal biogenesis factor 12 |
| | 72 | NM_014968 | | Data not found |
| | 73 | NM_001216 | CA9 | Carbonic anhydrase IX |
| | 74 | NM_001673 | ASNS | Asparagine synthetase |
| | 75 | NM_000096 | CP | Ceruloplasmin (ferroxidase) |
| | 76 | NM_000291 | PGK1 | Phosphoglycerate kinase 1 |
| | 77 | NM_006265 | RAD21 | RAD21 homolog (S. pombe) |
| | 78 | NM_003600 | AURKA | Aurora kinase A |
| | 79 | N38891 | HIPK2 | Homeodomain interacting protein kinase 2 |
| | 80 | NM_000320 | QDPR | Quinoid dihydropteridine reductase |
| | 81 | AB033007 | ERGIC1 | Endoplasmic reticulum-golgi intermediate compartment (ERGIC) 1 |
| | 82 | AA748494 | ASPM | Asp (abnormal spindle)-like, microcephaly associated (Drosophila) |
| | 83 | NM_004336 | BUB1 | BUB1 budding uninhibited by benzimidazoles 1 homolog (yeast) |
| | 84 | AL355708 | NEO1 | Neogenin homolog 1 (chicken) |
| | 85 | NM_000017 | ACADS | Acyl-Coenzyme A dehydrogenase, C-2 to C-3 short chain |
| | 86 | N69403 | | Transcribed locus |
| | 87 | NM_006281 | STK3 | Serine/threonine kinase 3 (STE20 homolog, yeast) |
| | 88 | NM_004701 | CCNB2 | Cyclin B2 |
| | 89 | NM_006763 | BTG2 | BTG family, member 2 |
| | 90 | AF148505 | ALDH6A1 | Aldehyde dehydrogenase 6 family, member A1 |
| | 91 | AF155117 | KIF21A | Kinesin family member 21 A |
| | 92 | NM_018265 | C1orf106 | Chromosome 1 open reading frame 106 |
| | 93 | H15286 | LYPD6 | Hypothetical protein MGC52057 |
| | 94 | NM_017779 | DEPDC1 | DEP domain containing 1 |
| | 95 | NM_020166 | MCCC1 | Methylcrotonoyl-Coenzyme A carboxylase 1 (alpha) |
| | 96 | NM_001280 | CIRBP | Cold inducible RNA binding protein |
| | 97 | AF161553 | IVNS1ABP | Influenza virus NS1A binding protein |
| | 98 | NM_018098 | ECT2 | Epithelial cell transforming sequence 2 oncogene |
| | 99 | NM_003376 | VEGF | Vascular endothelial growth factor |
| | 100 | NM_001168 | BIRC5 | Baculoviral IAP repeat-containing 5 (survivin) |
| | 101 | AJ224741 | MATN3 | Matrilin 3 |
| | 102 | NM_014875 | KIF14 | Kinesin family member 14 |
| | 103 | M21551 | NMB | Neuromedin B |
| | 104 | U45975 | PIB5PA | Phosphatidylinositol (4,5) bisphosphate 5-phosphatase, A |
| | 105 | AI300570 | | CDNA: FLJ23228 fis, clone CAE06654 |

(continued)

|  | GenBankAccession# | Symbol | Description |
|---|---|---|---|
| 106 | AI248998 | CACNA1D | Calcium channel, voltage-dependent, L type, alpha 1 D subunit |
| 107 | NM_004504 | HRB | HIV-1 Rev binding protein |
| 108 | AI917602 |  | Transcribed locus, strongly similar to NP_061886.1 hypothetical protein FLJ10156 [Homo sapiens] |
| 109 | AI924323 | NDUFA4L2 | NADH dehydrogenase (ubiquinone) 1 alpha subcomplex, 4-like 2 |
| 110 | NM_002808 | PSMD2 | Proteasome (prosome, macropain) 26S subunit, non-ATPase, 2 |
| 111 | AI741818 | SDSL | Serine dehydratase-like |
| 112 | AI125487 |  | Transcribed locus |
| 113 | NM_014109 | ATAD2 | ATPase family, AAA domain containing 2 |
| 114 | AI743843 | C1orf96 | Chromosome 1 open reading frame 96 |
| 115 | NM_005196 |  | Data not found |
| 116 | AI741080 | B3GALNT2 | Beta-1,3-N-acetylgalactosaminyltransferase 2 |
| 117 | NM_014750 | DLG7 | Discs, large homolog 7 (Drosophila) |
| 118 | AI912791 | ZNF395 | Zinc finger protein 395 |
| 119 | NM_003158 |  | Data not found |
| 120 | NM_004456 | EZH2 | Enhancer of zeste homolog 2 (Drosophila) |
| 121 | NM_003258 | TK1 | Thymidine kinase 1, soluble |
| 122 | AI694966 | RAI2 | Retinoic acid induced 2 |
| 123 | AI247495 | MYO10 | Myosin X |
| 124 | AL137514 | IHPK2 | Inositol hexaphosphate kinase 2 |
| 125 | NM_018455 | CENPN | Centromere protein N |
| 126 | NM_004911 | PDIA4 | Protein disulfide isomerase family A, member 4 |
| 127 | NM_005563 | STMN1 | Stathmin 1/oncoprotein 18 |
| 128 | U96131 | TRIP13 | Thyroid hormone receptor interactor 13 |
| 129 | NM_003878 | GGH | Gamma-glutamyl hydrolase (conjugase, folylpolygammaglutamyl hydrolase) |
| 130 | NM_000224 | KRT18 | Keratin 18 |
| 131 | AI374873 |  | CDNA FLJ32438 fis, clone SKMUS2001402 |
| 132 | AW276078 | LOC387763 | Hypothetical LOC387763 |
| 133 | NM_004052 | BNIP3 | BCL2/adenovirus E1B 19kDa interacting protein 3 |
| 134 | AI149869 | QSER1 | Glutamine and serine rich 1 |
| 135 | NM_018410 | DKFZp762E1312 | Hypothetical protein DKFZp762E1312 |
| 136 | NM_000158 | GBE1 | Glucan (1,4-alpha-), branching enzyme 1 (glycogen branching enzyme, Andersen disease, glycogen storage disease type IV) |
| 137 | NM_013262 | MYLIP | Myosin regulatory light chain interacting protein |
| 138 | AI076929 | LOC124220 | Similar to common salivary protein 1 |

(continued)

| | GenBankAccession# | Symbol | Description |
|---|---|---|---|
| 139 | NM_004163 | RAB27B | RAB27B, member RAS oncogene family |
| 140 | AB020689 | TBC1D9 | TBC1 domain family, member 9 |
| 141 | NM_018136 | ASPM | Asp (abnormal spindle)-like, microcephaly associated (Drosophila) |
| 142 | NM_003662 | PIR | Pirin (iron-binding nuclear protein) |
| 143 | NM_015416 | LETMD1 | LETM1 domain containing 1 |
| 144 | AI810244 | MGC7036 | Hypothetical protein MGC7036 |
| 145 | R70506 | | CDNA FLJ26120 fis, clone SYN00419 |
| 146 | NM_012177 | FBXO5 | F-box protein 5 |
| 147 | NM_012429 | SEC14L2 | SEC14-like 2 (S. cerevisiae) |
| 148 | AL133603 | PLEKHA1 | Pleckstrin homology domain containing, family A (phosphoinositide binding specific) member 1 |
| 149 | AI669860 | ZDHHC20 | Zinc finger, DHHC-type containing 20 |
| 150 | NM_013437 | LRP12 | Low density lipoprotein-related protein 12 |
| 151 | NM_003676 | DEGS1 | Degenerative spermatocyte homolog 1, lipid desaturase (Drosophila) |
| 152 | AA828380 | HIF1A | Hypoxia-inducible factor 1, alpha subunit (basic helix-loop-helix transcription factor) |
| 153 | AL137502 | RRAGD | Ras-related GTP binding D |
| 154 | AB033043 | KIAA1217 | KIAA1217 |
| 155 | AA553367 | HIF1A | Hypoxia-inducible factor 1, alpha subunit (basic helix-loop-helix transcription factor) |
| 156 | AL133619 | CCDC74B | Coiled-coil domain containing 74B |
| 157 | NM_006372 | SYNCRIP | Synaptotagmin binding, cytoplasmic RNA interacting protein |
| 158 | NM_006201 | PCTK1 | PCTAIRE protein kinase 1 |
| 159 | NM_016569 | TBX3 | T-box 3 (ulnar mammary syndrome) |
| 160 | NM_012214 | MGAT4A | Mannosyl (alpha-1,3-)-glycoprotein beta-1,4-N-acetylglucosaminyltransferase, isozyme A |
| 161 | AA524005 | TMEM64 | Transmembrane protein 64 |
| 162 | N33100 | | Data not found |
| 163 | AI820045 | RHBDF2 | Rhomboid 5 homolog 2 (Drosophila) |
| 164 | AI222165 | PABPC1 | Poly(A) binding protein, cytoplasmic 1 |
| 165 | NM_018004 | TMEM45A | Transmembrane protein 45A |
| 166 | X94232 | MAPRE2 | Microtubule-associated protein, RP/EB family, member 2 |
| 167 | AI095706 | CD163L1 | CD163 molecule-like 1 |
| 168 | D25328 | PFKP | Phosphofructokinase, platelet |
| 169 | NM_004480 | FUT8 | Fucosyltransferase 8 (alpha (1,6) fucosyltransferase) |
| 170 | NM_006096 | NDRG1 | N-myc downstream regulated gene 1 |
| 171 | AB032973 | MULK | Multiple substrate lipid kinase |

(continued)

|  |  | GenBankAccession# | Symbol | Description |
|---|---|---|---|---|
|  | 172 | NM_004798 | KIF3B | Kinesin family member 3B |
|  | 173 | NM_004603 | NCF1 | Neutrophil cytosolic factor 1, (chronic granulomatous disease, autosomal 1) |
|  | 174 | NM_002811 | PSMD7 | Proteasome (prosome, macropain) 26S subunit, non-ATPase, 7 (Mov34 homolog) |
|  | 175 | NM_018454 | NUSAP1 | Nucleolar and spindle associated protein 1 |
|  | 176 | NM_001905 | CTPS | CTP synthase |
|  | 177 | NM_014363 | SACS | Spastic ataxia of Charlevoix-Saguenay (sacsin) |
|  | 178 | AA579843 | C11orf48 | Chromosome 11 open reading frame 48 |
|  | 179 | NM_000507 | FBP1 | Fructose-1,6-bisphosphatase 1 |
|  | 180 | AB037745 | KIAA1324 | KIAA1324 |
|  | 181 | AA703254 | TBX19 | T-box 19 |
|  | 182 | AI310524 | NIPA1 | Non imprinted in Prader-Willi/Angelman syndrome 1 |
|  | 183 | NM_005496 | SMC4 | SMC4 structural maintenance of chromosomes 4-like 1 (yeast) |
|  | 184 | AI657153 | TMEM25 | Transmembrane protein 25 |
|  | 185 | NM_001333 | CTSL2 | Cathepsin L2 |
|  | 186 | NM_001007 | RPS4X | Ribosomal protein S4, X-linked |
|  | 187 | NM_006115 | PRAME | Preferentially expressed antigen in melanoma |
|  | 188 | AL050021 | SLC7A1 | Solute carrier family 7 (cationic amino acid transporter, y+ system), member 1 |
|  | 189 | AA632295 | STON2 | Stonin 2 |
|  | 190 | AI313222 | C13orf3 | Chromosome 13 open reading frame 3 |
|  | 191 | L27560 | IGFBP5 | Insulin-like growth factor binding protein 5 |
|  | 192 | NM_018104 |  | Data not found |
|  | 193 | AI151442 | RASL11B | RAS-like, family 11, member B |
|  | 194 | NM_001124 | ADM | Adrenomedullin |
|  | 195 | AI925240 | GSDMDC1 | Gasdermin domain containing 1 |
|  | 196 | NM_012261 | C20orf103 | Chromosome 20 open reading frame 103 |
|  | 197 | NM_018120 | ARMC1 | Armadillo repeat containing 1 |
|  | 198 | NM_020244 | CHPT1 | Choline phosphotransferase 1 |
|  | 199 | NM_014078 | MRPL13 | Mitochondrial ribosomal protein L13 |
|  | 200 | NM_015434 | INTS7 | Integrator complex subunit 7 |
|  | 201 | AI080735 | RRM2 | Ribonucleotide reductase M2 polypeptide |
|  | 202 | AI261191 | ZNF627 | Zinc finger protein 627 |
|  | 203 | N38757 | C1orf198 | Chromosome 1 open reading frame 198 |
|  | 204 | NM_001827 | CKS2 | CDC28 protein kinase regulatory subunit 2 |
|  | 205 | AA994026 | COL23A1 | Collagen, type XXIII, alpha 1 |

(continued)

| | | GenBankAccession# | Symbol | Description |
|---|---|---|---|---|
| | 206 | AI765936 | | Transcribed locus, strongly similar to XP_510292.1 PREDICTED: hypothetical protein XP_510292 [Pan troglodytes] |
| | 207 | NM_002570 | PCSK6 | Proprotein convertase subtilisin/kexin type 6 |
| | 208 | AA921830 | MLF1IP | MLF1 interacting protein |
| | 209 | U58033 | MTMR2 | Myotubularin related protein 2 |
| | 210 | NM_014754 | PTDSS1 | Phosphatidylserine synthase 1 |
| | 211 | NM_002900 | RBP3 | Retinol binding protein 3, interstitial |
| | 212 | AL050090 | MYRIP | Myosin VIIA and Rab interacting protein |
| | 213 | NM_015417 | C20orf28 | Chromosome 20 open reading frame 28 |
| | 214 | H30384 | | Transcribed locus |
| | 215 | NM_005342 | HMGB3 | High-mobility group box 3 |
| | 216 | AI857856 | KIF21A | Kinesin family member 21A |
| | 217 | NM_016337 | EVL | Enah/Vasp-like |
| | 218 | AI479658 | RBED1 | RNA binding motif and ELMO/CED-12 domain 1 |
| | 219 | NM_004358 | CDC25B | Cell division cycle 25B |
| | 220 | AW083338 | ALDH6A1 | Aldehyde dehydrogenase 6 family, member A1 |
| | 221 | NM_002358 | MAD2L1 | MAD2 mitotic arrest deficient-like 1 (yeast) |
| | 222 | AA932206 | ACE | Angiotensin I Converting enzyme (peptidyl-dipeptidase A) 1 |
| | 223 | AF052159 | PTPLB | Protein tyrosine phosphatase-like (proline instead of catalytic arginine), member b |
| | 224 | NM_019013 | FAM64A | Family with sequence similarity 64, member A |
| | 225 | NM_013296 | GPSM2 | G-protein signalling modulator 2 (AGS3-like, C. elegans) |
| | 226 | AI951530 | MGC16385 | Hypothetical protein MGC16385 |
| | 227 | AL137295 | MLLT10 | Myeloid/lymphoid or mixed-lineage leukemia (trithorax homolog, Drosophila); translocated to, 10 |
| | 228 | AI479633 | KIAA1683 | KIAA1683 |
| | 229 | AL080110 | PAQR3 | Progestin and adipoQ receptor family member III |
| | 230 | NM_003234 | TFRC | Transferrin receptor (p90, CD71) |
| | 231 | NM_020675 | SPBC25 | Spindle pole body component 25 homolog (S. cerevisiae) |

Glas et al. 2006

Table 3

| | GenBankAccession# | GeneSymbol | Description |
|---|---|---|---|
| 1 | Y10659 | IL13RA1 | Interleukin 13 receptor, alpha 1 |
| 2 | XM_376516 | | |
| 3 | X98834 | SALL2 | Sal-like 2 (Drosophila) |
| 4 | X69433 | IDH2 | Isocitrate dehydrogenase 2 (NADP+), mitochondrial |
| 5 | X16468 | COL2A1 | Collagen, type II, alpha 1 (primary osteoarthritis, spondyloepiphyseal dysplasia, congenital) |
| 6 | W70343 | LOX | Lysyl oxidase |

(continued)

| | GenBankAccession# | GeneSymbol | Description |
|---|---|---|---|
| 7 | W68711 | C9orf91 | Chromosome 9 open reading frame 91 |
| 8 | W37375 | DNAJC8 | DnaJ (Hsp40) homolog, subfamily C, member 8 |
| 9 | U90030 | BICD1 | Bicaudal D homolog 1 (Drosophila) |
| 10 | U67206 | CASP7 | Caspase 7, apoptosis-related cysteine peptidase |
| 11 | U65410 | MAD2L1 | MAD2 mitotic arrest deficient-like 1 (yeast) |
| 12 | U58515 | CHI3L2 | Chitinase 3-like 2 |
| 13 | U57059 | TNFSF10 | Tumor necrosis factor (ligand) superfamily, member 10 |
| 14 | U41654 | RRAGA | Ras-related GTP binding A |
| 15 | U38847 | TARBP1 | Tar (HIV-1) RNA binding protein 1 |
| 16 | U07802 | | |
| 17 | U03100 | CTNNA1 | Catenin (cadherin-associated protein), alpha 1, 102kDa |
| 18 | T60160 | GABARAPL1 | GABA(A) receptor-associated protein like 1 |
| 19 | T57841 | UFD1L | Ubiquitin fusion degradation 1 like (yeast) |
| 20 | T55569 | C12orf44 | Chromosome 12 open reading frame 44 |
| 21 | T47815 | PSME1 | Proteasome (prosome, macropain) activator subunit 1 (PA28 alpha) |
| 22 | S79267 | CD4 | CD4 molecule |
| 23 | R92446 | | |
| 24 | R62612 | FN1 | Fibronectin 1 |
| 25 | R48844 | COL1A1 | Collagen, type I, alpha 1 |
| 26 | R39094 | ANKHD1 | Ankyrin repeat and KH domain containing 1 |
| 27 | NM_203330 | CD59 | CD59 molecule, complement regulatory protein |
| 28 | NM_199075 | | |
| 29 | NM_198433 | AURKA | Aurora kinase A |
| 30 | NM_198129 | LAMA3 | Laminin, alpha 3 |
| 31 | NM_156036 | | |
| 32 | NM_153490 | KRT17 | Keratin 17 |
| 33 | NM_152866 | MS4A1 | Membrane-spanning 4-domains, subfamily A, member 1 |
| 34 | NM_130439 | MXI1 | MAX interactor 1 |
| 35 | NM_057158 | DUSP4 | Dual specificity phosphatase 4 |
| 36 | NM_033292 | CASP1 | Caspase 1, apoptosis-related cysteine peptidase (interleukin 1, beta, convertase) |
| 37 | NM_032853 | MUM1 | Melanoma associated antigen (mutated) 1 |
| 38 | NM_031966 | CCNB1 | Cyclin B1 |
| 39 | NM_030819 | GFOD2 | Glucose-fructose oxidoreductase domain containing 2 |
| 40 | NM_030766 | BCL2L14 | BCL2-like 14 (apoptosis facilitator) |
| 41 | NM_024629 | MLF1IP | MLF1 interacting protein |
| 42 | NM_022916 | VPS33A | Vacuolar protein sorting 33 homolog A (S. cerevisiae) |
| 43 | NM_022841 | RFXDC2 | Regulatory factor X domain containing 2 |
| 44 | NM_022829 | SLC13A3 | Solute carrier family 13 (sodium-dependent dicarboxylate transporter), member 3 |
| 45 | NM_021800 | DNAJC12 | DnaJ (Hsp40) homolog, subfamily C, member 12 |
| 46 | NM_020974 | SCUBE2 | Signal peptide, CUB domain, EGF-like 2 |
| 47 | NM_020675 | SPBC25 | Spindle pole body component 25 homolog (S. cerevisiae) |
| 48 | NM_020470 | YIF1A | Yip1 interacting factor homolog A (S. cerevisiae) |
| 49 | NM_020153 | C11orf60 | Chromosome 11 open reading frame 60 |
| 50 | NM_020038 | | |
| 51 | NM_018944 | C21orf45 | Chromosome 21 open reading frame 45 |
| 52 | NM_018558 | GABRQ | Gamma-aminobutyric acid (GABA) receptor, theta |
| 53 | NM_017859 | UCKL1 | Uridine-cytidine kinase 1-like 1 |

(continued)

| | GenBankAccession# | GeneSymbol | Description |
|---|---|---|---|
| 54 | NM_017760 | LUZP5 | Leucine zipper protein 5 |
| 55 | NM_017612 | ZCCHC8 | Zinc finger, CCHC domain containing 8 |
| 56 | NM_017534 | MYH2 | Myosin, heavy polypeptide 2, skeletal muscle, adult |
| 57 | NM_016730 | FOLR1 | Folate receptor 1 (adult) |
| 58 | NM_016548 | GOLPH2 | Golgi phosphoprotein 2 |
| 59 | NM_016524 | SYT17 | Synaptotagmin XVII |
| 60 | NM_016343 | CENPF | Centromere protein F, 350/400ka (mitosin) |
| 61 | NM_015997 | C1orf66 | Chromosome 1 open reading frame 66 |
| 62 | NM_015905 | TRIM24 | Tripartite motif-containing 24 |
| 63 | NM_015254 | KIF13B | Kinesin family member 13B |
| 64 | NM_014845 | KIAA0274 | KIAA0274 |
| 65 | NM_014796 | | |
| 66 | NM_014615 | KIAA0182 | KIAA0182 |
| 67 | NM_014612 | FAM120A | Chromosome 9 open reading frame 10 |
| 68 | NM_014338 | PISD | Phosphatidylserine decarboxylase |
| 69 | NM_014109 | ATAD2 | ATPase family, AAA domain containing 2 |
| 70 | NM_014042 | C11orf51 | Chromosome 11 open reading frame 51 |
| 71 | NM_013936 | OR12D2 | Olfactory receptor, family 12, subfamily D, member 2 |
| 72 | NM_013411 | AK2 | Adenylate kinase 2 |
| 73 | NM_013296 | GPSM2 | G-protein signalling modulator 2 (AGS3-like, C. elegans) |
| 74 | NM_013279 | C11orf9 | Chromosome 11 open reading frame 9 |
| 75 | NM_012324 | MAPK8IP2 | Mitogen-activated protein kinase 8 interacting protein 2 |
| 76 | NM_012319 | SLC39A6 | Solute carrier family 39 (zinc transporter), member 6 |
| 77 | NM_007315 | STAT1 | Signal transducer and activator of transcription 1, 91 kDa |
| 78 | NM_007295 | BRCA1 | Breast cancer 1, early onset |
| 79 | NM_007203 | PALM2-AKAP2 | PALM2-AKAP2 protein |
| 80 | NM_007192 | SUPT16H | Suppressor of Ty 16 homolog (S. cerevisiae) |
| 81 | NM_007014 | WWP2 | WW domain containing E3 ubiquitin protein ligase 2 |
| 82 | NM_006965 | ZNF24 | Zinc finger protein 24 |
| 83 | NM_006930 | SKP1A | S-phase kinase-associated protein 1A (p19A) |
| 84 | NM_006766 | MYST3 | MYST histone acetyltransferase (monocytic leukemia) 3 |
| 85 | NM_006720 | ABLIM1 | Actin binding LIM protein 1 |
| 86 | NM_006596 | | |
| 87 | NM_006534 | NCOA3 | Nuclear receptor coactivator 3 |
| 88 | NM_006437 | PARP4 | Poly (ADP-ribose) polymerase family, member 4 |
| 89 | NM_006416 | SLC35A1 | Solute carrier family 35 (CMP-sialic acid transporter), member A1 |
| 90 | NM_006410 | HTATIP2 | HIV-1 Tat interactive protein 2, 30kDa |
| 91 | NM_006379 | SEMA3C | Sema domain, immunoglobulin domain (Ig), short basic domain, secreted, (semaphorin) 3C |
| 92 | NM_006314 | CNKSR1 | Connector enhancer of kinase suppressor of Ras 1 |
| 93 | NM_006243 | PPP2R5A | Protein phosphatase 2, regulatory subunit B (B56), alpha isoform |
| 94 | NM_006197 | PCM1 | Pericentriolar material 1 |
| 95 | NM_005940 | MMP11 | Matrix metallopeptidase 11 (stromelysin 3) |
| 96 | NM_005901 | SMAD2 | SMAD, mothers against DPP homolog 2 (Drosophila) |
| 97 | NM 005729 | PPIF | Peptidylprolyl isomerase F (cyclophilin F) |
| 98 | NM_005496 | SMC4 | SMC4 structural maintenance of chromosomes 4-like 1 (yeast) |
| 99 | NM_005441 | CHAF1B | Chromatin assembly factor 1, subunit B (p60) |
| 100 | NM_005426 | TP53BP2 | Tumor protein p53 binding protein, 2 |

|  | GenBankAccession# | GeneSymbol | Description |
|---|---|---|---|
| 101 | NM_005318 | H1F0 | H1 histone family, member 0 |
| 102 | NM_005310 | GRB7 | Growth factor receptor-bound protein 7 |
| 103 | NM_005256 | FANCF | Fanconi anemia, complementation group F |
| 104 | NM_005056 | JARID1A | Jumonji, AT rich interactive domain 1A (RBBP2-like) |
| 105 | NM_005030 | PLK1 | Polo-like kinase 1 (Drosophila) |
| 106 | NM_004911 | PDIA4 | Protein disulfide isomerase family A, member 4 |
| 107 | NM_004859 | CLTC | Clathrin, heavy polypeptide (Hc) |
| 108 | NM_004711 | SYNGR1 | Synaptogyrin 1 |
| 109 | NM_004703 | RABEP1 | Rabaptin, RAB GTPase binding effector protein 1 |
| 110 | NM_004702 |  |  |
| 111 | NM_004670 | PAPSS2 | 3'-phosphoadenosine 5'-phosphosulfate synthase 2 |
| 112 | NM_004659 |  |  |
| 113 | NM_004631 | LRP8 | Low density lipoprotein receptor-related protein 8, apolipoprotein e receptor |
| 114 | NM_004470 | FKBP2 | FK506 binding protein 2, 3kDa |
| 115 | NM_004457 | ACSL3 | Acyl-CoA synthetase long-chain family member 3 |
| 116 | NM_004448 | ERBB2 | V-erb-b2 erythroblastic leukemia viral oncogene homolog 2, neuro/glioblastoma derived oncogene homolog (avian) |
| 117 | NM_004360 | CDH1 | Cadherin 1, type 1, E-cadherin (epithelial) |
| 118 | NM_004346 | CASP3 | Caspase 3, apoptosis-related cysteine peptidase |
| 119 | NM_004323 | BAG1 | BCL2-associated athanogene |
| 120 | NM_004111 | FEN1 | Flap structure-specific endonuclease 1 |
| 121 | NM_003902 | FUBP1 | Far upstream element (FUSE) binding protein 1 |
| 122 | NM_003869 | CES2 | Carboxylesterase 2 (intestine, liver) |
| 123 | NM_003845 | DYRK4 | Dual-specificity tyrosine-(Y)-phosphorylation regulated kinase 4 |
| 124 | NM_003824 | FADD | Fas (TNFRSF6)-associated via death domain |
| 125 | NM_003600 | AURKA | Aurora kinase A |
| 126 | NM_003543 | HIST1H4H | Histone 1, H4h |
| 127 | NM_003489 | NRIP1 | Nuclear receptor interacting protein 1 |
| 128 | NM_003243 | TGFBR3 | Transforming growth factor, beta receptor III (betaglycan, 300kDa) |
| 129 | NM_003234 | TFRC | Transferrin receptor (p90, CD71) |
| 130 | NM_003200 | TCF3 | Transcription factor 3 (E2A immunoglobulin enhancer binding factors E1.2/E47) |
| 131 | NM_003107 | SOX4 | SRY (sex determining region Y)-box 4 |
| 132 | NM_002996 | CX3CL1 | Chemokine (C-X3-C motif) ligand 1 |
| 133 | NM_002916 | RFC4 | Replication factor C (activator 1) 4, 37kDa |
| 134 | NM_002894 | RBBP8 | Retinoblastoma binding protein 8 |
| 135 | NM_002840 | PTPRF | Protein tyrosine phosphatase, receptor type, F |
| 136 | NM_002803 | PSMC2 | Proteasome (prosome, macropain) 26S subunit, ATPase, 2 |
| 137 | NM_002740 | PRKCI | Protein kinase C, iota |
| 138 | NM_002710 | PPP1CC | Protein phosphatase 1, catalytic subunit, gamma isoform |
| 139 | NM_002690 | POLB | Polymerase (DNA directed), beta |
| 140 | NM_002633 | PGM1 | Phosphoglucomutase 1 |
| 141 | NM_002466 | MYBL2 | myeloblastosis viral oncogene homolog (avian)-like 2 |
| 142 | NM_002439 | MSH3 | MutS homolog 3 (E. coli) |
| 143 | NM_002417 | MKI67 | Antigen identified by monoclonal antibody Ki-67 |
| 144 | NM_002388 | MCM3 | MCM3 minichromosome maintenance deficient 3 (S. cerevisiae) |
| 145 | NM_002196 | INSM1 | Insulinoma-associated 1 |

(continued)

| | GenBankAccession# | GeneSymbol | Description |
|---|---|---|---|
| 146 | NM_002047 | GARS | Glycyl-tRNA synthetase |
| 147 | NM_002046 | GAPDH | Glyceraldehyde-3-phosphate dehydrogenase |
| 148 | NM_001958 | EEF1A2 | Eukaryotic translation elongation factor 1 alpha 2 |
| 149 | NM_001903 | CTNNA1 | Catenin (cadherin-associated protein), alpha 1, 102kDa |
| 150 | NM_001813 | CENPE | Centromere protein E, 312kDa |
| 151 | NM_001806 | CEBPG | CCAAT/enhancer binding protein (C/EBP), gamma |
| 152 | NM_001800 | CDKN2D | Cyclin-dependent kinase inhibitor 2D (p19, inhibits CDK4) |
| 153 | NM_001710 | CFB | Complement factor B |
| 154 | NM_001615 | ACTG2 | Actin, gamma 2, smooth muscle, enteric |
| 155 | NM_001562 | IL18 | Interleukin 18 (interferon-gamma-inducing factor) |
| 156 | NM_001453 | FOXC1 | Forkhead box C1 |
| 157 | NM_001394 | DUSP4 | Dual specificity phosphatase 4 |
| 158 | NM_001379 | DNMT1 | DNA (cytosine-5-)-methyltransferase 1 |
| 159 | NM_001333 | CTSL2 | Cathepsin L2 |
| 160 | NM_001316 | CSE1L | CSE1 chromosome segregation 1-like (yeast) |
| 161 | NM_001251 | EIF4A1 | Eukaryotic translation initiation factor 4A, isoform 1 |
| 162 | NM_001175 | ARHGDIB | Rho GDP dissociation inhibitor (GDI) beta |
| 163 | NM_001168 | BIRC5 | Baculoviral IAP repeat-containing 5 (survivin) |
| 164 | NM_001144 | AMFR | Autocrine motility factor receptor |
| 165 | NM_001101 | ACTB | Actin, beta |
| 166 | NM_001068 | TOP2B | Topoisomerase (DNA) II beta 180kDa |
| 167 | NM_001067 | TOP2A | Topoisomerase (DNA) II alpha 170kDa |
| 168 | NM_001002 | RPLPO | Ribosomal protein, large, P0 |
| 169 | NM_000947 | PRIM2A | Primase, polypeptide 2A, 58kDa |
| 170 | NM_000926 | PGR | Progesterone receptor |
| 171 | NM_000849 | GSTM3 | Glutathione S-transferase M3 (brain) |
| 172 | NM_000633 | BCL2 | B-cell CLL/lymphoma 2 |
| 173 | NM_000561 | GSTM1 | Glutathione S-transferase M1 |
| 174 | NM_000401 | EXT2 | Exostoses (multiple) 2 |
| 175 | NM_000346 | SOX9 | (sex determining region Y)-box 9 (campomelic dysplasia, autosomal sex-reversal) |
| 176 | NM_000337 | SGCD | Sarcoglycan, delta (35kDa dystrophin-associated glycoprotein) |
| 177 | NM_000311 | PRNP | Prion protein (p27-30) (Creutzfeldt-Jakob disease, Gerstmann-Strausler-Scheinker syndrome, fatal familial insomnia) |
| 178 | NM_000302 | PLOD1 | Procollagen-lysine 1, 2-oxoglutarate 5-dioxygenase 1 |
| 179 | NM_000213 | ITGB4 | Integrin, beta 4 |
| 180 | NM_000181 | GUSB | Glucuronidase, beta |
| 181 | NM_000135 | FANCA | Fanconi anemia, complementation group A |
| 182 | NM_000125 | ESR1 | Estrogen receptor 1 |
| 183 | NM_000096 | CP | Ceruloplasmin (ferroxidase) |
| 184 | NM_000064 | C3 | Complement component 3 |
| 185 | N95358 | MYO1B | Myosin IB |
| 186 | N72215 | PSAP | Prosaposin (variant Gaucher disease and variant metachromatic leukodystrophy) |
| 187 | N68825 | KIAA0133 | KIAA0133 |
| 188 | N67797 | DBR1 | Debranching enzyme homolog 1 (S. cerevisiae) |
| 189 | N51614 | FMNL1 | Formin-like 1 |
| 190 | N22323 | RP6-213H19.1 | Serine/threonine protein kinase MST4 |
| 191 | M55580 | SAT | Spermidine/spermine N1-acetyltransferase |
| 192 | M23254 | CAPN2 | Calpain 2, (m/ll) large subunit |

(continued)

| | GenBankAccession# | GeneSymbol | Description |
|---|---|---|---|
| 193 | J05581 | MUC1 | Mucin 1, cell surface associated |
| 194 | J03798 | SNRPD1 | Small nuclear ribonucleoprotein D1 polypeptide 16kDa |
| 195 | H96654 | WBP5 | WW domain binding protein 5 |
| 196 | H95960 | SPARC | Secreted protein, acidic, cysteine-rich (osteonectin) |
| 197 | H85475 | FLJ34306 | FLJ34306 protein |
| 198 | H85107 | | |
| 199 | H72683 | C10orf9 | Chromosome 10 open reading frame 9 |
| 200 | H71881 | CAMTA1 | Calmodulin binding transcription activator 1 |
| 201 | H63760 | TSPAN5 | Tetraspanin 5 |
| 202 | H59048 | NFATC3 | Nuclear factor of activated T-cells, cytoplasmic, calcineurin-deppndent 3 |
| 203 | H29308 | WDR51B | WD repeat domain 51B |
| 204 | H01495 | TRAM1 | Translocation associated membrane protein 1 |
| 205 | D89324 | | |
| 206 | D87448 | TOPBP1 | Topoisomerase (DNA) II binding protein 1 |
| 207 | D79995 | TTLL4 | Tubulin tyrosine ligase-like family, member 4 |
| 208 | D63487 | TTLL12 | Tubulin tyrosine ligase-like family, member 12 |
| 209 | BX649106 | EXOSC9 | Exosome component 9 |
| 210 | BX649090 | INPP4B | Inositol polyphosphate-4-phosphatase, type II, 105kDa |
| 211 | BX648387 | LOC285086 | Hypothetical protein LOC285086 |
| 212 | BX648364 | TCF7L2 | Transcription factor 7-like 2 (T-cell specific, HMG-box) |
| 213 | BX648308 | CHI3L | Chitinase 3-like 1 (cartilage glycoprotein-39) |
| 214 | BX648303 | SLC9A3R1 | Solute carrier family 9 (sodium/hydrogen exchanger), member 3 regulator 1 |
| 215 | BX648034 | NDUFB5 | NADH dehydrogenase (ubiquinone) 1 beta subcomplex, 5, 16kDa |
| 216 | BX647539 | BLVRA | Biliverdin reductase A |
| 217 | BX647392 | | |
| 218 | BX647212 | ARNT2 | Aryl-hydrocarbon receptor nuclear translocator 2 |
| 219 | BX647151 | MYBL2 | V-myb myeloblastosis viral oncogene homolog (avian)- |
| 220 | BX640616 | | |
| 221 | BX538158 | STX3 | Syntaxin 3 |
| 222 | BX538009 | HIPK2 | Homeodomain interacting protein kinase 2 |
| 223 | BX537826 | | |
| 224 | BX537705 | PRKACB | Protein kinase, cAMP-dependent, catalytic, beta |
| 225 | BX537698 | NFIB | Nuclear factor I/B |
| 226 | BX537500 | PDCD4 | Programmed cell death 4 (neoplastic transformation inhibitor) |
| 227 | BX537379 | H3F3B | H3 histone, family 3B (H3.3B) |
| 228 | B739088 | PSMA3 | Proteasome (prosome, macropain) subunit, alpha type, 3 |
| 229 | BU739068 | LSM1 | LSM1 homolog, U6 small nuclear RNA associated (S. cerevisiae) |
| 230 | BU730089 | PARD6A | Par-6 partitioning defective 6 homolog alpha (C.elegans) |
| 231 | BU729963 | RBX1 | Ring-box 1 |
| 232 | BU729319 | RAB3A | member RAS oncogene family |
| 233 | BU536516 | TFF3 | Trefoil factor 3 (intestinal) |
| 234 | BQ898943 | CKS2 | CDC28 protein kinase regulatory subunit 2 |
| 235 | BQ894155 | IGFBP2 | Insulin-like growth factor binding protein 2, 36kDa |
| 236 | BQ688566 | MMP7 | Matrix metallopeptidase 7 (matrilysin, uterine) |
| 237 | BQ278502 | PTTG1 | Pituitary tumor-transforming 1 |
| 238 | BQ278454 | CKS1B | CDC28 protein kinase regulatory subunit 1B |

(continued)

| | GenBankAccession# | GeneSymbol | Description |
|---|---|---|---|
| 239 | BQ073581 | NINJ1 | Ninjurin 1 |
| 240 | BQ056428 | TYMS | Thymidylate synthetase |
| 241 | BQ056337 | CDKN3 | Cyclin-dependent kinase inhibitor 3 (CDK2-associated dual specificity phosphatase) |
| 242 | BM994509 | SOD2 | Superoxide dismutase 2, mitochondrial |
| 243 | BM926728 | GSTP1 | Glutathione S-transferase pi |
| 244 | BM924855 | IMPA2 | Inositol(myo)-1(or4)-monophosphatase 2 |
| 245 | BM920905 | C10orf116 | Chromosome 10 open reading frame 116 |
| 246 | BM916335 | ISG15 | ISG15 ubiquitin-like modifier |
| 247 | BM911641 | COX5A | Cytochrome c oxidase subunit Va |
| 248 | BM909357 | BIRC5 | Baculoviral IAP repeat-containing 5 (survivin) |
| 249 | BM907902 | ETFA | Electron-transfer-flavoprotein, alpha polypeptide (glutaric aciduria II) |
| 250 | BM907771 | ARPC1B | Actin related protein 2/3 complex, subunit 1B, 41kDa |
| 251 | BM809638 | NME1 | Non-metastatic cells 1, protein (NM23A) expressed in |
| 252 | BM701438 | | Transcribed locus |
| 253 | BM701226 | NCF1 | Neutrophil cytosolic factor 1, (chronic granulomatous disease, autosomal 1) |
| 254 | BM701128 | TMEM126A | Transmembrane protein 126A |
| 255 | BM690957 | TCEAL1 | Transcription elongation factor A (SII)-like 1 |
| 256 | BM545518 | NDUFA7 | NADH dehydrogenase (ubiquinone) 1 alpha subcomplex, 7, 14.5kDa |
| 257 | BM542397 | TSG101 | Tumor susceptibility gene 101 |
| 258 | BM470905 | CSRP2 | Cysteine and glycine-rich protein 2 |
| 259 | BM462434 | YIF1A | Yip1 interacting factor homolog A (S. cerevisiae) |
| 260 | BM458012 | IFI6 | Interferon, alpha-inducible protein 6 |
| 261 | BG827359 | SEC61G | Sec61 gamma subunit |
| 262 | BG324446 | FBL | Fibrillarin |
| 263 | BG114681 | NME1 | Non-metastatic cells 1, protein (NM23A) expressed in |
| 264 | BF972232 | GSTK1 | Glutathione S-transferase kappa 1 |
| 265 | BF790785 | CD38 | CD38 molecule |
| 266 | BF572330 | RPL11 | Ribosomal protein L11 |
| 267 | BF569100 | KRT18 | Keratin 18 |
| 268 | BF569085 | CYC1 | Cytochrome c-1 |
| 269 | BF217712 | | |
| 270 | BF210063 | IFITM1 | Interferon induced transmembrane protein 1 (9-27) |
| 271 | BF204697 | PHB2 | Prohibitin 2 |
| 272 | BF055474 | PHF11 | PHD finger protein 11 |
| 273 | BF055311 | NEFL | Neurofilament, light polypeptide 68kDa |
| 274 | BE896331 | PCNA | Proliferating cell nuclear antigen |
| 275 | BE748755 | CBX3 | Chromobox homolog 3 (HP1 gamma homolog, Drosophila) |
| 276 | BC071593 | KIT | V-kit Hardy-Zuckerman 4 feline sarcoma viral oncogene homolog |
| 277 | BC071577 | | |
| 278 | BC068438 | GART | Phosphoribosylglycinamide formyltransferase, phosphoribosylglycinamide synthetase, phosphoribosylaminoimidazole synthetase |
| 279 | BC063817 | WDR26 | WD repeat domain 26 |
| 280 | BC063289 | C4A | Complement component 4A (Rodgers blood group) |
| 281 | BC063281 | SREBF1 | Sterol regulatory element binding transcription factor 1 |

(continued)

| | GenBankAccession# | GeneSymbol | Description |
|---|---|---|---|
| 282 | BC059394 | LYN | V-yes-1 Yamaguchi sarcoma viral related oncogene homolog |
| 283 | BC054491 | EIF2C2 | Eukaryotic translation initiation factor 2C, 2 |
| 284 | BC050420 | MTHFD1 | Methylenetetrahydrofolate dehydrogenase (NADP+ dependent) 1, methenyltetrahydrofolate cyclohydrolase, formyltetrahydrofolate synthetase |
| 285 | BC048292 | ARF3 | ADP-ribosylation factor 3 |
| 286 | BC046477 | C11orf54 | Chromosome 11 open reading frame 54 |
| 287 | BC044929 | COX17 | COX17 cytochrome c oxidase assembly homolog (S. cerevisiae) |
| 288 | BC043352 | ZBTB4 | Zinc finger and BTB domain containing 4 |
| 289 | BC042178 | CXCL9 | Chemokine (C-X-C motif) ligand 9 |
| 290 | BC041846 | CDH3 | Cadherin 3, type 1, P-cadherin (placental) |
| 291 | BC039269 | NALP2 | NACHT, leucine rich repeat and PYD containing 2 |
| 292 | BC038505 | BAG4 | BCL2-associated athanogene 4 |
| 293 | BC038114 | ST6GALNAC2 | ST6 (alpha-N-acetyl-neuraminyl-2,3-beta-galactosyl-1,3)-N-acetylgalactosaminide alpha-2,6-sialyltransferase 2 |
| 294 | BC037236 | DUSP6 | Dual specificity phosphatase 6 |
| 295 | BC036702 | | CDNA clone IMAGE:5266735 |
| 296 | BC036520 | KIAA0251 | KIAA0251 protein |
| 297 | BC036503 | SFRP1 | Secreted frizzled-related protein 1 |
| 298 | BC036314 | FAM3B | Family with sequence similarity 3, member B |
| 299 | BC035854 | DSCR1 L2 | Down syndrome critical region gene 1-like 2 |
| 300 | BC035716 | ISGF3G | Interferon-stimulated transcription factor 3, gamma 48kDa |
| 301 | BC035578 | STK24 | Serine/threonine kinase 24 (STE20 homolog, yeast) |
| 302 | BC033517 | ACOX2 | Acyl-Coenzyme A oxidase 2, branched chain |
| 303 | BC031055 | HDAC2 | Histone deacetylase 2 |
| 304 | BC029512 | PHYH | Phytanoyl-CoA 2-hydroxylase |
| 305 | BC028578 | BIRC2 | Baculoviral IAP repeat-containing 2 |
| 306 | BC028033 | MFAP2 | Microfibrillar-associated protein 2 |
| 307 | BC026289 | PLDN | Pallidin homolog (mouse) |
| 308 | BC025232 | CDC6 | CDC6 cell division cycle 6 homolog) (S. cerevisiae) |
| 309 | BC024200 | FAM3C | Family with sequence similarity 3, member C |
| 310 | BC022008 | PRAME | Preferentially expressed antigen in melanoma |
| 311 | BC021714 | PPFIBP2 | PTPRF interacting protein, binding protein 2 (liprin beta 2) |
| 312 | BC019092 | EPOR | Erythropoietin receptor |
| 313 | BC017338 | FUCA1 | Fucosidase, alpha-L- 1, tissue |
| 314 | BC016341 | ISG20 | Interferon stimulated exonuclease gene 20kDa |
| 315 | BC014553 | RAB3IP | RAB3A interacting protein (rabin3) |
| 316 | BC013875 | MMP1 | Matrix metallopeptidase 1 (interstitial collagenase) |
| 317 | BC011050 | C5orf13 | Chromosome 5 open reading frame 13 |
| 318 | BC010281 | ARL6IP | ADP-ribosylation factor-like 6 interacting protein |
| 319 | BC006793 | GATA3 | GATA binding protein 3 |
| 320 | BC006325 | GTSE1 | G-2 and S-phase expressed 1 |
| 321 | BC006155 | AP2A2 | Adaptor-related protein complex 2, alpha 2 subunit |
| 322 | BC005978 | KPNA2 | Karyopherin alpha 2 (RAG cohort 1, importin alpha 1) |
| 323 | BC004372 | CD44 | CD44 molecule (Indian blood group) |
| 324 | BC002671 | DUSP4 | Dual specificity phosphatase 4 |
| 325 | BC002506 | PDCD10 | Programmed cell death 10 |
| 326 | BC001769 | TRAF4 | TNF receptor-associated factor 4 |

(continued)

| | GenBankAccession# | GeneSymbol | Description |
|---|---|---|---|
| 327 | BC001535 | UTP18 | UTP18, small subunit (SSU) processome component, homolog (yeast) |
| 328 | BC001233 | CEP57 | Centrosomal protein 57kDa |
| 329 | BC001188 | TFRC | Transferrin receptor (p90, CD71) |
| 330 | BC001185 | KCTD15 | Potassium channel tetramerisation domain containing 15 |
| 331 | BC000596 | RPL23AP7 | Ribosomal protein L23a pseudogene 7 |
| 332 | AY358648 | CSNK1G1 | kinase 1, gamma 1 |
| 333 | AY325903 | DSCR1 | Down syndrome critical region gene 1 |
| 334 | AY260762 | ZFHX4 | Zinc finger homeodomain 4 |
| 335 | AV713720 | | |
| 336 | AV693985 | | |
| 337 | AL834469 | ECHDC1 | Enoyl Coenzyme A hydratase domain containing 1 |
| 338 | AL833264 | FEM1B | Fem-1 homolog b (C. elegans) |
| 339 | AL832862 | LOC155060 | Hypothetical protein LOC155060 |
| 340 | AL832245 | KIAA0020 | KIAA0020 |
| 341 | AL523310 | MAP4 | Microtubule-associated protein 4 |
| 342 | AL512688 | FABP7 | Fatty acid binding protein 7, brain |
| 343 | AL442089 | KCNH2 | Potassium voltage-gated channel, subfamily H (eag-related), member 2 |
| 344 | AL359937 | GSTT1 | Glutathione S-transferase theta 1 |
| 345 | AL137162 | | |
| 346 | AL136877 | SMC4 | SMC4 structural maintenance of chromosomes 4-like 1 (yeast) |
| 347 | AL133102 | HDGFRP3 | Hepatoma-derived growth factor, related protein 3 |
| 348 | AL117652 | | MRNA; cDNA DKFZp586B1324 (from clone DKFZp586B1324) |
| 349 | AL117478 | GPSM1 | G-protein signalling modulator 1 (AGS3-like, C. elegans) |
| 350 | AL110212 | H2AFV | H2A histone family, member V |
| 351 | AL050227 | PTGER3 | Prostaglandin E receptor 3 (subtype EP3) |
| 352 | AL049783 | PFAAP5 | Phosphonoformate immuno-associated protein 5 |
| 353 | AK131568 | | |
| 354 | AK131563 | AHCYL1 | S-adenosylhomocysteine hydrolase-like 1 |
| 355 | AK130583 | PCBP2 | Poly(rC) binding protein 2 |
| 356 | AK127672 | CSK | C-src tyrosine kinase |
| 357 | AK127456 | PSMC6 | Proteasome (prosome, macropain) 26S subunit, ATPase, 6 |
| 358 | AK126315 | NCF1 | Neutrophil cytosolic factor 1, (chronic granulomatous disease, autosomal 1) |
| 359 | AK125855 | DAZAP2 | DAZ associated protein 2 |
| 360 | AK125710 | CD58 | CD58 molecule |
| 361 | AK125625 | ARHGDIB | Rho GDP dissociation inhibitor (GDI) beta |
| 362 | AK125307 | FGF12 | Fibroblast growth factor 12 |
| 363 | AK124774 | PDCD5 | Programmed cell death 5 |
| 364 | AK124323 | NMI | N-myc (and STAT) interactor |
| 365 | AK123590 | PIGT | Phosphatidylinositol glycan, class T |
| 366 | AK123477 | IFI30 | Interferon, gamma-inducible protein 30 |
| 367 | AK122841 | GDI2 | GDP dissociation inhibitor 2 |
| 368 | AK098329 | | |
| 369 | A098055 | GATM | Glycine amidinotransferase (L-arginine:glycine amidinotransferase) |
| 370 | AK096865 | CDV3 | CDV3 homolog (mouse) |
| 371 | AK096284 | LFNG | Lunatic fringe-homolog (Drosophila) |
| 372 | AK096006 | CRABP1 | Cellular retinoic acid binding protein 1 |

(continued)

| | GenBankAccession# | GeneSymbol | Description |
|---|---|---|---|
| 373 | AK095669 | | |
| 374 | AK095316 | SNRPB2 | Small nuclear ribonucleoprotein polypeptide B" |
| 375 | AK094899 | SHFM1 | Split hand/foot malformation (ectrodactyly) type 1 |
| 376 | AK094855 | PPOX | Protoporphyrinogen oxidase |
| 377 | AK094784 | BMP7 | Bone morphogenetic protein 7 (osteogenic protein 1) |
| 378 | AK094534 | CCNH | Cyclin H |
| 379 | AK094393 | | |
| 380 | AK093917 | KDELR2 | KDEL (Lys-Asp-Glu-Leu) endoplasmic reticulum protein retention receptor 2 |
| 381 | AK093842 | XBP1 | X-box binding protein 1 |
| 382 | AK093775 | CD3D | CD3d molecule, delta (CD3-TCR complex) |
| 383 | AK093096 | CLGN | Calmegin |
| 384 | AK092726 | RCC1 | Regulator of chromosome condensation 1 |
| 385 | AK092638 | CCNG2 | Cyclin G2 |
| 386 | AK092391 | CST6 | Cystatin E/M |
| 387 | AK092210 | MAP3K15 | Mitogen-activated protein kinase kinase kinase 15 |
| 388 | AK092094 | | CDNA FLJ34775 fis, clone NT2NE2003315 |
| 389 | AK091644 | UBE2Z | Ubiquitin-conjugating enzyme E2Z (putative) |
| 390 | AK091579 | STARD3NL | STARD3 N-terminal like |
| 391 | AK091034 | | CDNA FLJ33715 fis, clone BRAWH2008577 |
| 392 | AK090462 | | |
| 393 | AK057302 | GALE | UDP-galactose-4-epimerase |
| 394 | AK057117 | SSR4 | Signal sequence receptor, delta (translocon-associated protein delta) |
| 395 | AK056821 | SAR1B | SAR1 gene homolog B (S. cerevisiae) |
| 396 | AK055699 | LYPD6 | Hypothetical protein MGC52057 |
| 397 | AK055249 | UCHL1 | Ubiquitin carboxyl-terminal esterase L1 (ubiquitin thiolesterase) |
| 398 | AK054976 | HINT1 | Histidine triad nucleotide binding protein 1 |
| 399 | AK054596 | IGBP1 | Immunoglobulin (CD79A) binding protein 1 |
| 400 | AK027271 | CDC2 | Cell division cycle 2, G1 to S and G2 to M |
| 401 | AK027032 | GLG1 | Golgi apparatus protein 1 |
| 402 | AK025738 | ASF1A | ASF1 anti-silencing function 1 homolog A (S. cerevisiae) |
| 403 | AK023925 | C10orf7 | Chromosome 10 open reading frame 7 |
| 404 | AK023803 | ARF1 | ADP-ribosylation factor 1 |
| 405 | AK021842 | FLJ25476 | FLJ25476 protein |
| 406 | AK001562 | C17orf63 | Chromosome 17 open reading frame 63 |
| 407 | AK001548 | GTPBP4 | GTP binding protein 4 |
| 408 | AK001280 | HDGFRP3 | Hepatoma-derived growth factor, related protein 3 |
| 409 | AJ606319 | MYB | V-myb myeloblastosis viral oncogene homolog (avian) |
| 410 | AJ551176 | SDC1 | Syndecan 1 |
| 411 | AJ536056 | FUT8 | Fucosyltransferase 8 (alpha (1,6) fucosyltransferase) |
| 412 | AJ318054 | SRPK1 | SFRS protein kinase 1 |
| 413 | AJ296290 | WNK1 | WNK lysine deficient protein kinase 1 |
| 414 | AI733259 | | Transcribed locus, strongly similar to NP_004356.2 centrin 3; homolog of S. cerevisiae CDC31; CDC31 yeast homolog; EF-hand superfamily member; centrin, EF-hand protein, 3 (CDC31 yeast homolog) [Homo sapiens] |
| 415 | AI676033 | ISG20L2 | Interferon stimulated exonuclease gene 20kDa-like 2 |
| 416 | AI669875 | FAM8A1 | Family with sequence similarity 8, member A1 |
| 417 | AI636233 | TMEM8 | Transmembrane protein 8 (five membrane-spanning domains) |

(continued)

| | GenBankAccession# | GeneSymbol | Description |
|---|---|---|---|
| 418 | AI493245 | CD44 | CD44 molecule (Indian blood group) |
| 419 | AI364298 | PRPSAP2 | Phosphoribosyl pyrophosphate synthetase-associated protein 2 |
| 420 | AI359120 | CHD6 | Chromodomain helicase DNA binding protein 6 |
| 421 | AI340932 | GENX-3414 | Genethonin 1 |
| 422 | AI266693 | NAT9 | N-acetyltransferase 9 |
| 423 | AI083527 | | |
| 424 | AI057637 | ACACB | Acetyl-Coenzyme A carboxylase beta |
| 425 | AF519769 | ENAH | Enabled homolog (Drosophila) |
| 426 | AF467287 | LRBA | LPS-responsive vesicle trafficking, beach and anchor containing |
| 427 | AF220152 | TACC2 | Transforming, acidic coiled-coil containing protein 2 |
| 428 | AF125507 | ORC3L | Origin recognition complex, subunit 3-like (yeast) |
| 429 | AF123759 | CLN8 | Ceroid-lipofuscinosis, neuronal 8 (epilepsy, progressive with mental retardation) |
| 430 | AF114013 | TNFSF13 | Tumor necrosis factor (ligand) superfamily, member 13 |
| 431 | AF114012 | TNFSF13 | Tumor necrosis factor (ligand) superfamily, member 13 |
| 432 | AF053305 | BUB1 | BUB1 budding uninhibited by benzimidazoles 1 homolog (yeast) |
| 433 | AF051151 | TLR5 | Toll-like receptor 5 |
| 434 | AF041410 | MAG | Malignancy-associated protein |
| 435 | AB049150 | PEG10 | Paternally expressed 10 |
| 436 | AB014607 | ACOT11 | Acyl-CoA thioesterase 11 |
| 437 | AB008112 | PEX1 | Peroxisome biogenesis factor 1 |
| 438 | AB002385 | PTPRN2 | Protein tyrosine phosphatase, receptor type, N polypeptide 2 |
| 439 | AA975556 | FLJ22709 | Hypothetical protein FLJ22709 |
| 440 | AA933888 | CXADR | Coxsackie virus and adenovirus receptor |
| 441 | AA912071 | DLX2 | Distal-less homeobox 2 |
| 442 | AA886199 | C1orf34 | Chromosome 1 open reading frame 34 |
| 443 | AA775791 | ZDHHC16 | Zinc finger, DHHC-type containing 16 |
| 444 | AA772093 | NEURL | Neuralized-like (Drosophila) |
| 445 | AA703184 | EZH1 | Enhancer of zeste homolog 1 (Drosophila) |
| 446 | AA679940 | TXN2 | Thioredoxin 2 |
| 447 | AA677388 | ITIH1 | Inter-alpha (globulin) inhibitor H1 |
| 448 | AA669758 | NPM1 | Nucleophosmin (nucleolar phosphoprotein B23, numatrin) |
| 449 | AA668425 | AGL | Amylo-1,6-glucosidase, 4-alpha-glucanotransferase (glycogen debranching enzyme, glycogen storage disease type III) |
| 450 | AA663983 | TPI1 | Triosephosphate isomerase 1 |
| 451 | AA644587 | | Transcribed locus |
| 452 | AA626362 | WFDC6 | WAP four-disulfide core domain 6 |
| 453 | AA625960 | MCFP | Mitochondrial carrier family protein |
| 454 | AA598955 | TNC | Tenascin C (hexabrachion) |
| 455 | AA504120 | | CDNA FLJ35270 fis, clone PROST2005630 |
| 456 | AA497029 | LDHA | Lactate dehydrogenase A |
| 457 | AA496000 | | |
| 458 | AA489638 | SERB1 | SERPINE1 mRNA binding protein 1 |
| 459 | AA489015 | ALG2 | Asparagine-linked glycosylation 2 homolog (S. cerevisiae, alpha-1,3-mannosyltransferase) |
| 460 | AA486275 | SERPINB1 | Serpin peptidase inhibitor, clade B (ovalbumin), member 1 |
| 461 | AA481283 | CASP2 | Caspase 2, apoptosis-related cysteine peptidase (neural precursor cell expressed, developmentally downregulated 2) |

(continued)

| | GenBankAccession# | GeneSymbol | Description |
|---|---|---|---|
| 462 | AA479888 | RABEP1 | Rabaptin, RAB GTPase binding effector protein 1 |
| 463 | AA479202 | TIMP3 | TIMP metallopeptidase inhibitor 3 (Sorsby fundus dystrophy, pseudoinflammatory) |
| 464 | AA464246 | HLA-B | Major histocompatibility complex, class I, B |
| 465 | AA456931 | COX6C | Cytochrome c oxidase subunit VIc |
| 466 | AA454540 | GNAQ | Guanine nucleotide binding protein (G protein), q polypeptide |
| 467 | AA449773 | CDC42EP4 | CDC42 effector protein (Rho GTPase binding) 4 |
| 468 | AA446839 | | |
| 469 | AA446193 | KIAA0999 | KIAA0999 protein |
| 470 | AA431187 | | MRNA; cDNA DKFZp686H1233 (from clone DKFZp686H1233) |
| 471 | A430668 AI732703 | | |
| 472 | AA427899 | TUBB | Tubulin, beta |
| 473 | AA418826 | LOC400721 | Similar to Zinc finger protein 418 |
| 474 | AA398237 | TSC22D1 | TSC22 domain family, member 1 |
| 475 | AA293365 | MAP2K4 | Mitogen-activated protein kinase kinase 4 |
| 476 | AA293306 | IL4R | Interleukin 4 receptor |
| 477 | AA279072 | INPPL1 | Inositol polyphosphate phosphatase-like 1 |
| 478 | AA206591 | | Transcribed locus, weakly similar to NP_055301.1 neuronal thread protein AD7c-NTP [Homo sapiens] |
| 479 | AA176957 | NEB | Nebulin |
| 480 | AA165628 | | Transcribed locus |
| 481 | AA137228 | MKLN1 | Muskelin 1, intracellular mediator containing kelch motifs |
| 482 | AA137072 | PTPRS | Protein tyrosine phosphatase, receptor type, S |
| 483 | AA054643 | PARD6B | Par-6 partitioning defective 6 homolog beta (C. elegans) |
| 484 | AA046411 | APPBP2 | Amyloid beta precursor protein (cytoplasmic tail) binding protein 2 |
| 485 | AA035436 | API5 | Apoptosis inhibitor 5 |
| 486 | AA029042 | SIAH2 | Seven in absentia homolog 2 (Drosophila) |
| 487 | AA002091 | ACOT12 | Acyl-CoA thioesterase 12 |
| 488 | XM_087225 | | |
| 489 | XM_058945 | | |
| 490 | XM_047080 | | |
| 491 | X55150 | | |
| 492 | U86046 | | |
| 493 | S78535 | | |
| 494 | NM_058216 | | |
| 495 | NM_057749 | | |
| 496 | NM_053025 | | |
| 497 | NM_032989 | | |
| 498 | NM_031966 | | |
| 499 | NM_023109 | | |
| 500 | NM_021975 | | |
| 501 | NM_021953 | | |
| 502 | NM_021874 | | |
| 503 | NM_020974 | | |
| 504 | NM_020686 | | |
| 505 | NM_019887 | | |
| 506 | NM_018463 | | |
| 507 | NM_017779 | | |
| 508 | NM_017460 | | |

(continued)

| | GenBankAccession# | GeneSymbol | Description |
|---|---|---|---|
| 509 | NM_017458 | | |
| 510 | NM_016434 | | |
| 511 | NM_015460 | | |
| 512 | NM_014791 | | |
| 513 | NM_014417 | | |
| 514 | NM_014109 | | |
| 515 | NM_013407 | | |
| 516 | NM_012261 | | |
| 517 | NM_012231 | | |
| 518 | NM_012177 | | |
| 519 | NM_012112 | | |
| 520 | NM_007295 | | |
| 521 | NM_007194 | | |
| 522 | NM_007182 | | |
| 523 | NM_006824 | | |
| 524 | NM_006744 | | |
| 525 | NM_006681 | | |
| 526 | NM_006669 | | |
| 527 | NM_006526 | | |
| 528 | NM_006347 | | |
| 529 | NM_006221 | | |
| 530 | NM_006206 | | |
| 531 | NM_006115 | | |
| 532 | NM_005975 | | |
| 533 | NM_005954 | | |
| 534 | NM_005940 | | |
| 535 | NM_005915 | | |
| 536 | NM_005778 | | |
| 537 | NM_005657 | | |
| 538 | NM_005573 | | |
| 539 | NM_005562 | | |
| 540 | NM_005544 | | |
| 541 | NM_005465 | | |
| 542 | NM_005441 | | |
| 543 | NM_005429 | | |
| 544 | NM_005426 | | |
| 545 | NM_005378 | | |
| 546 | NM_005310 | | |
| 547 | NM_005231 | | |
| 548 | NM_005228 | | |
| 549 | NM_005167 | | |
| 550 | NM_005163 | | |
| 551 | NM_004994 | | |
| 552 | NM_004955 | | |
| 553 | NM_004938 | | |
| 554 | NM_004846 | | |
| 555 | NM_004689 | | |
| 556 | NM_004559 | | |
| 557 | NM_004530 | | |
| 558 | NM_004526 | | |

| | GenBankAccession# | GeneSymbol | Description |
|---|---|---|---|

(continued)

| | GenBankAccession# | GeneSymbol | Description |
|---|---|---|---|
| 559 | NM_004499 | | |
| 560 | NM_004496 | | |
| 561 | NM_004484 | | |
| 562 | NM_004448 | | |
| 563 | NM_004383 | | |
| 564 | NM_004360 | | |
| 565 | NM_004336 | | |
| 566 | NM_004324 | | |
| 567 | NM_004323 | | |
| 568 | NM_004305 | | |
| 569 | NM_004219 | | |
| 570 | NM_004163 | | |
| 571 | NM_004064 | | |
| 572 | NM_004060 | | |
| 573 | NM_003882 | | |
| 574 | NM_003862 | | |
| 575 | NM_003844 | | |
| 576 | NM_003842 | | |
| 577 | NM_003810 | | |
| 578 | NM_003766 | | |
| 579 | NM_003620 | | |
| 580 | NM_003600 | | |
| 581 | NM_003579 | | |
| 582 | NM_003380 | | |
| 583 | NM_003377 | | |
| 584 | NM_003376 | | |
| 585 | NM_003324 | | |
| 586 | NM_003258 | | |
| 587 | NM_003257 | | |
| 588 | NM_003255 | | |
| 589 | NM_003254 | | |
| 590 | NM_003242 | | |
| 591 | NM_003239 | | |
| 592 | NM_003236 | | |
| 593 | NM_003234 | | |
| 594 | NM_003225 | | |
| 595 | NM_003219 | | |
| 596 | NM_003194 | | |
| 597 | NM_003161 | | |
| 598 | NM_003095 | | |
| 599 | NM_003056 | | |
| 600 | NM_003012 | | |
| 601 | NM_002982 | | |
| 602 | NM_002899 | | |
| 603 | NM_002776 | | |
| 604 | NM_002726 | | |
| 605 | NM_002659 | | |
| 606 | NM_002658 | | |
| 607 | NM_002656 | | |
| 608 | NM_002645 | | |

(continued)

| | GenBankAccession# | GeneSymbol | Description |
|---|---|---|---|
| 609 | NM_002639 | | |
| 610 | NM_002609 | | |
| 611 | NM_002608 | | |
| 612 | NM_002607 | | |
| 613 | NM_002592 | | |
| 614 | NM_002539 | | |
| 615 | NM_002497 | | |
| 616 | NM_002474 | | |
| 617 | NM_002467 | | |
| 618 | NM_002466 | | |
| 619 | NM_002426 | | |
| 620 | NM_002417 | | |
| 621 | NM_002392 | | |
| 622 | NM_002388 | | |
| 623 | NM_002354 | | |
| 624 | NM_002276 | | |
| 625 | NM_002273 | | |
| 626 | NM_002253 | | |
| 627 | NM_002230 | | |
| 628 | NM_002206 | | |
| 629 | NM_002166 | | |
| 630 | NM_002165 | | |
| 631 | NM_002135 | | |
| 632 | NM_002127 | | |
| 633 | NM_002121 | | |
| 634 | NM_002051 | | |
| 635 | NM_002046 | | |
| 636 | NM_002019 | | |
| 637 | NM_002014 | | |
| 638 | NM_002012 | | |
| 639 | NM_002006 | | |
| 640 | NM_001982 | | |
| 641 | NM_001968 | | |
| 642 | NM_001953 | | |
| 643 | NM_001945 | | |
| 644 | NM_001912 | | |
| 645 | NM_001908 | | |
| 646 | NM_001904 | | |
| 647 | NM_001769 | | |
| 648 | NM_001758 | | |
| 649 | NM_001754 | | |
| 650 | NM_001657 | | |
| 651 | NM_001626 | | |
| 652 | NM_001530 | | |
| 653 | NM_001511 | | |
| 654 | NM_001498 | | |
| 655 | NM_001432 | | |
| 656 | NM_001333 | | |
| 657 | NM_001324 | | |
| 658 | NM_001299 | | |

(continued)

| | GenBankAccession# | GeneSymbol | Description |
|---|---|---|---|
| 659 | NM_001274 | | |
| 660 | NM_001255 | | |
| 661 | NM_001251 | | |
| 662 | NM_001238 | | |
| 663 | NM_001216 | | |
| 664 | NM_001191 | | |
| 665 | NM_001188 | | |
| 666 | NM_001168 | | |
| 667 | NM_001167 | | |
| 668 | NM_001166 | | |
| 669 | NM_001165 | | |
| 670 | NM_001101 | | |
| 671 | NM_001071 | | |
| 672 | NM_001068 | | |
| 673 | NM_001067 | | |
| 674 | NM_001002 | | |
| 675 | NM_000964 | | |
| 676 | NM_000963 | | |
| 677 | NM_000926 | | |
| 678 | NM_000875 | | |
| 679 | NM_000852 | | |
| 680 | NM_000849 | | |
| 681 | NM_000770 | | |
| 682 | NM_000734 | | |
| 683 | NM_000693 | | |
| 684 | NM_000689 | | |
| 685 | NM_000639 | | |
| 686 | NM_000633 | | |
| 687 | NM_000618 | | |
| 688 | NM_000602 | | |
| 689 | NM_000600 | | |
| 690 | NM_000599 | | |
| 691 | NM_000598 | | |
| 692 | NM_000597 | | |
| 693 | NM_000561 | | |
| 694 | NM_000546 | | |
| 695 | NM_000526 | | |
| 696 | NM_000442 | | |
| 697 | NM_000424 | | |
| 698 | NM_000422 | | |
| 699 | NM_000389 | | |
| 700 | NM_000376 | | |
| 701 | NM_000362 | | |
| 702 | NM_000321 | | |
| 703 | NM_000314 | | |
| 704 | NM_000269 | | |
| 705 | NM_000245 | | |
| 706 | NM_000237 | | |
| 707 | NM_000224 | | |
| 708 | NM_000211 | | |

(continued)

(continued)

| | GenBankAccession# | GeneSymbol | Description |
|---|---|---|---|
| 709 | NM_000181 | | |
| 710 | NM_000177 | | |
| 711 | NM_000125 | | |
| 712 | NM_000120 | | |
| 713 | NM_000118 | | |
| 714 | NM_000110 | | |
| 715 | NM_000089 | | |
| 716 | NM_000088 | | |
| 717 | NM_000077 | | |
| 718 | NM_000059 | | |
| 719 | NM_000043 | | |
| 720 | NM_000038 | | |
| 721 | M29145 | | |
| 722 | EGFRd27 | | |
| 723 | Contig47405 | | |
| 724 | Contig46653 | | |
| 725 | Contig44799 | | |
| 726 | BG675392 | | |
| 727 | B000712 | | |
| 728 | AL050227 | | |
| 729 | AK000618 | | |
| 730 | AJ420468 | | |
| 731 | AF159141 | | |

**Claims**

1. A method for normalizing data of a breast cancer sample, the method comprising
   providing a first and a second array comprising between 10 and 12.000 nucleic acid molecules comprising a first and second set of nucleic acid molecules wherein each nucleic acid molecule of said first set comprises a nucleotide sequence that is able to hybridize to a different gene selected from the genes listed in Table 1,
   wherein said first and said second array comprise an identical first set of nucleic acid molecules comprising at least 10 molecules comprising a nucleotide sequence that is able to hybridize to a different gene selected from the genes listed in Table 1;
   dividing a sample in at least two sub-samples and labeling nucleic acid expression products in a first sub-sample with a first label, and labeling a second sub-sample with a second label different from said first label;
   contacting said first array with said first sub-sample and determining for each of said nucleic acid molecule of said first set the amount of said first label that is associated therewith;
   contacting said second array with said second sub-sample and determining for each of said nucleic acid molecule of said first set the amount of second label that is associated therewith;
   determining from the difference in associated first and second label for each of said first set of nucleic acid molecules a systematic error in the measurements of detected label depending on the amount of said first and said second label that is detected; and
   correcting at least one value for the detected amount of label associated with at least one nucleic acid on said array for the systematic error.

2. A method according to claim 1, wherein said nucleic acid for which said value is corrected is a member of said second set of nucleic acid molecules.

3. The method according to claim 1, further comprising
   determining a transformation function for transforming the log10 ratios of the intensities of the detected hybridization signals to zero or approximately zero for expression products of genes listed in Table 1;
   using the determined transformation function to transform the intensity of the log10 ratios of hybridization signals obtained from nucleic acid molecules belonging to said second set of molecules.

4. Use of an array, comprising between 10 and 12.000 nucleic acid molecules comprising a first set of nucleic acid molecules wherein each nucleic acid molecule of said first set comprises a nucleotide sequence that is able to hybridize to a different gene selected from the genes listed in Table 1, wherein the first set of molecules comprises at least ten nucleic acid molecules that are able to hybridize to different genes listed in Table 1, in the method of claim 1.

5. Use of the array according to claim 4, wherein said at least ten nucleic acid molecules are able to hybridize to genes listed in Table 1 which have the lowest standard deviation and are rank-ordered 1-10.

6. Use of the array according to claim 4 or 5, wherein the first set of molecules comprises nucleic acid molecules selected from at least two different intensity groups as depicted in Table 1.

7. Use of the array according to any of claims 4-6, wherein the first set of molecules comprises nucleic acid molecules selected from all five intensity groups as depicted in Table 1.

8. Use of the array according to any of claims 4-7, further comprising a second set of nucleic acid molecules comprising nucleic acid molecules capable of hybridizing to nucleic acid molecules that are expressed in samples of breast tissue.

9. Use of the array according to claim 8, wherein the second set of nucleic acid molecules comprises at least five nucleotide sequences capable of hybridizing to nucleic acid molecules that are expressed in breast samples and that are selected from Table 2.

10. Use of the array according to claim 8, wherein the second set of nucleic acid molecules comprises at least five nucleotide sequences capable of hybridizing to nucleic acid molecules selected from Table 3.

11. Use of an array comprising between 10 and 12.000 nucleic acid molecules comprising a first and second set of nucleic acid molecules wherein
said first set of nucleic acid molecules comprises at least 10 molecules, each nucleic acid molecule of said first set comprising a nucleotide sequence that is able to hybridize to a different gene selected from the genes listed in Table 1, said array further comprising a second set of nucleic acid molecules comprising nucleic acid molecules capable of hybridizing to nucleic acid molecules that are expressed in samples of breast tissue in a method for detecting and/or staging of breast cancer, the method comprising
contacting said array with a sample comprising expression products from breast cancer cells of a patient;
detecting hybridization of said expression products to nucleic acid molecules of said array to provide an expression profile;
normalizing hybridization data obtained with at least one nucleic acid molecule of the second set of nucleic acid molecules with hybridization data obtained with the first set of nucleic acid molecules; and
comparing said hybridization with the hybridization of at least one reference sample to a similar array.

12. The use according to claim 11, wherein the second set of nucleic acid molecules comprises at least five nucleotide sequences capable of hybridizing to nucleic acid molecules that are expressed in breast samples and that are selected from Table 2.

13. The use according to claim 11 or 12, whereby said reference sample is a sample from a patient with known breast cancer, or with known outcome of breast cancer.

**Patentansprüche**

1. Verfahren zur Normalisierung von Daten einer Brustkrebsprobe, wobei das Verfahren umfasst
Bereitstellen eines ersten und eines zweiten Arrays, umfassend zwischen 10 und 12.000 Nukleinsäuremoleküle umfassend einen ersten und zweiten Satz Nukleinsäuremoleküle, wobei jedes Nukleinsäuremolekül des ersten Satzes eine Nukleotidsequenz umfasst, die in der Lage ist, mit einem anderen Gen, ausgewählt aus den in Tabelle 1 aufgeführten Genen, zu hybridisieren,
wobei das erste und das zweite Array einen identischen ersten Satz Nukleinsäuremoleküle umfasst, umfassend mindestens 10 Moleküle umfassend eine Nukleotidsequenz, die in der Lage ist, mit einem anderen Gen, ausgewählt aus den in Tabelle 1 aufgeführten Genen, zu hybridisieren;

Aufteilen einer Probe in mindestens zwei Teilproben und Markieren von Nukleinsäure-Expressionsprodukten in einer ersten Teilprobe mit einer ersten Markierung und Markieren einer zweiten Teilprobe mit einer zweiten Markierung, die sich von der ersten Markierung unterscheidet;

Kontaktieren des ersten Arrays mit der ersten Teilprobe und, für jedes der Nukleinsäuremoleküle des ersten Satzes, Bestimmen der Menge der ersten Markierung, die damit assoziiert ist;

Kontaktieren des zweiten Arrays mit der zweiten Teilprobe und, für jedes der Nukleinsäuremoleküle des ersten Satzes, Bestimmen der Menge der zweiten Markierung, die damit assoziiert ist;

Bestimmen anhand des Unterschieds in der assoziierten ersten und zweiten Markierung, für jedes Nukleinsäuremoleküle des ersten Satzes, eines systematischen Fehlers in den Messungen detektierter Markierung, abhängig von der Menge der ersten und der zweiten Markierung, die detektiert wird; und

Korrigieren von mindestens einem Wert für die detektierte Menge der Markierung, assoziiert mit mindestens einer Nukleinsäure auf dem Array, für den systematischen Fehler.

2. Verfahren nach Anspruch 1, wobei die Nukleinsäure, für die der Wert korrigiert wird, ein Mitglied des zweiten Satzes Nukleinsäuremoleküle ist.

3. Verfahren nach Anspruch 1, ferner umfassend das Bestimmen einer Transformationsfunktion zum Transformieren der log10-Verhältnisse der Intensitäten der detektierten Hybridisierungssignale zu Null oder annähernd Null für Expressionsprodukte von Genen, aufgeführt in Tabelle 1;

Verwenden der bestimmten Transformationsfunktion zum Transformieren der Intensität der log10-Verhältnisse von Hybridisierungssignalen, erhalten von Nukleinsäuremolekülen, die zu dem zweiten Satz Moleküle gehören.

4. Verwendung eines Arrays, umfassend zwischen 10 und 12.000 Nukleinsäuremoleküle, umfassend einen ersten Satz Nukleinsäuremoleküle, wobei jedes Nukleinsäuremolekül des ersten Satzes eine Nukleotidsequenz umfasst, die in der Lage ist, mit einem anderen Gen, ausgewählt aus den in Tabelle 1 aufgeführten Genen, zu hybridisieren, wobei der erste Satz Moleküle mindestens zehn Nukleinsäuremoleküle umfasst, die in der Lage sind, in dem Verfahren nach Anspruch 1 mit anderen in Tabelle 1 aufgeführten Genen zu hybridisieren.

5. Verwendung des Arrays nach Anspruch 4, wobei die mindestens zehn Nukleinsäuremoleküle in der Lage sind, mit Genen, aufgeführt in Tabelle 1, zu hybridisieren, welche die niedrigste Standardabweichung haben und in der Reihenfolge 1-10 geordnet sind.

6. Verwendung des Arrays nach Anspruch 4 oder 5, wobei der erste Satz Moleküle Nukleinsäuremoleküle umfasst, ausgewählt aus mindestens zwei verschiedenen Intensitätsgruppen, wie in Tabelle 1 dargestellt.

7. Verwendung des Arrays nach einem der Ansprüche 4-6, wobei der erste Satz Moleküle Nukleinsäuremoleküle umfasst, ausgewählt aus allen fünf Intensitätsgruppen, wie in Tabelle 1 dargestellt.

8. Verwendung des Arrays nach einem der Ansprüche 4-7, ferner umfassend einen zweiten Satz Nukleinsäuremoleküle, umfassend Nukleinsäuremoleküle, die in der Lage sind, mit Nukleinsäuremolekülen zu hybridisieren, die in Proben von Brustgewebe exprimiert sind.

9. Verwendung des Arrays nach Anspruch 8, wobei der zweite Satz Nukleinsäuremoleküle mindestens fünf Nukleotidsequenzen umfasst, die in der Lage sind, mit Nukleinsäuremolekülen, die in Brustgewebeproben exprimiert sind und die aus Tabelle 2 ausgewählt sind, zu hybridisieren.

10. Verwendung des Arrays nach Anspruch 8, wobei der zweite Satz Nukleinsäuremoleküle mindestens fünf Nukleotidsequenzen umfasst, die in der Lage sind, mit Nukleinsäuremolekülen, ausgewählt aus Tabelle 3, zu hybridisieren.

11. Verwendung eines Arrays, umfassend zwischen 10 und 12.000 Nukleinsäuremoleküle, umfassend einen ersten und zweiten Satz Nukleinsäuremoleküle, wobei der erste Satz Nukleinsäuremoleküle mindestens 10 Moleküle umfasst, wobei jedes Nukleinsäuremolekül des ersten Satzes eine Nukleotidsequenz umfasst, die in der Lage ist, mit einem anderen Gen, ausgewählt aus den in Tabelle 1 aufgeführten Genen, zu hybridisieren, wobei das Array ferner einen zweiten Satz Nukleinsäuremoleküle umfasst, umfassend Nukleinsäuremoleküle, die in der Lage sind, mit Nukleinsäuremolekülen zu hybridisieren, die in Proben von Brustgewebe exprimiert sind, in einem Verfahren zum Detektieren und/oder Einstufen von Brustkrebs, wobei das Verfahren umfasst,

Kontaktieren des Arrays mit einer Probe, umfassend Expressionsprodukte von Brustkrebszellen eines Patienten;

Detektieren der Hybridisierung der Expressionsprodukte zu Nukleinsäuremolekülen des Arrays, um ein Expressionsprofil bereitzustellen;

Normalisieren der Hybridisierungsdaten, erhalten mit mindestens einem Nukleinsäuremolekül des zweiten Satzes Nukleinsäuremoleküle, mit Hybridisierungsdaten, erhalten mit dem ersten Satz Nukleinsäuremoleküle, ; und

Vergleichen der Hybridisierung mit der Hybridisierung von mindestens einer Referenzprobe von einem ähnlichen Array.

12. Verwendung nach Anspruch 11, wobei der zweite Satz Nukleinsäuremoleküle mindestens fünf Nukleotidsequenzen umfasst, die in der Lage sind, mit Nukleinsäuremolekülen, die in Brustgewebeproben exprimiert sind und die aus Tabelle 2 ausgewählt sind, zu hybridisieren.

13. Verwendung nach Anspruch 11 oder 12, wobei die Referenzprobe eine Probe von einem Patienten mit bekanntem Brustkrebs oder mit bekanntem Ausgang von Brustkrebs ist.

**Revendications**

1. Méthode de normalisation de données d'un échantillon de cancer du sein, la méthode comprenant

la fourniture d'une première et d'une deuxième matrice comprenant entre 10 et 12 000 molécules d'acide nucléique comprenant un premier et un deuxième jeu de molécules d'acide nucléique, dans laquelle chaque molécule d'acide nucléique dudit premier jeu comprend une séquence nucléotidique qui peut s'hybrider à un gène différent sélectionné parmi les gènes figurant dans le tableau 1,

dans laquelle ladite première et ladite deuxième matrice comprennent un premier jeu identique de molécules d'acide nucléique comprenant au moins 10 molécules comprenant une séquence nucléotidique qui peut s'hybrider à un gène différent sélectionné parmi les gènes figurant dans le tableau 1 ;

la division d'un échantillon en au moins deux sous-échantillons et le marquage des produits d'expression d'acide nucléique en un premier sous-échantillon avec un premier marqueur, et le marquage d'un deuxième sous-échantillon avec un deuxième marqueur différent dudit premier marqueur ;

la mise en contact de ladite première matrice avec ledit premier sous-échantillon et la détermination pour chacune desdites molécules d'acide nucléique dudit premier jeu de la quantité dudit premier marqueur qui est associée à celui-ci ;

la mise en contact de ladite deuxième matrice avec ledit deuxième sous-échantillon et la détermination pour chacune desdites molécules d'acide nucléique dudit premier jeu de la quantité du deuxième marqueur qui est associée à celui-ci ;

la détermination à partir de la différence dans les premier et deuxième marqueurs associés pour chacun desdits premiers jeux de molécules d'acide nucléique d'une erreur systématique dans les mesures du marqueur détecté en fonction de la quantité dudit premier et dudit deuxième marqueur qui est détectée ; et

la correction d'au moins une valeur pour la quantité de marqueur détectée associée à au moins un acide nucléique sur ladite matrice par l'erreur systématique.

2. Méthode selon la revendication 1, dans laquelle ledit acide nucléique pour lequel ladite valeur est corrigée est un membre dudit deuxième jeu de molécules d'acide nucléique.

3. Méthode selon la revendication 1, comprenant en outre

la détermination d'une fonction de transformation pour transformer les rapports log10 des intensités des signaux d'hybridation détectés à zéro ou environ zéro pour les produits d'expression des gènes figurant dans le tableau 1 ;

l'utilisation de la fonction de transformation déterminée pour transformer l'intensité des rapports log10 de signaux d'hybridation obtenus des molécules d'acide nucléique appartenant audit deuxième jeu de molécules.

4. Utilisation d'une matrice, comprenant entre 10 et 12 000 molécules d'acide nucléique comprenant un premier jeu de molécules d'acide nucléique, dans laquelle chaque molécule d'acide nucléique dudit premier jeu comprend une séquence nucléotidique qui peut s'hybrider à un gène différent sélectionné parmi les gènes figurant dans le tableau 1, dans laquelle ledit premier jeu de molécules comprend au moins dix molécules d'acide nucléique qui peuvent s'hybrider à des gènes différents figurant dans le tableau 1, dans la méthode selon la revendication 1.

5. Utilisation de la matrice selon la revendication 4, dans laquelle lesdites au moins dix molécules d'acide nucléique peuvent s'hybrider à des gènes figurant dans le tableau 1, qui ont l'écart type le plus faible et sont classées par ordre de 1 à 10.

**6.** Utilisation de la matrice selon la revendication 4 ou 5, dans laquelle le premier jeu de molécules comprend des molécules d'acide nucléique sélectionnées parmi au moins deux groupes d'intensité différents représentés dans le tableau 1.

**7.** Utilisation de la matrice selon l'une quelconque des revendications 4 à 6, dans laquelle le premier jeu de molécules comprend des molécules d'acide nucléique sélectionnées parmi tous les cinq groupes d'intensité représentés dans le tableau 1.

**8.** Utilisation de la matrice selon l'une quelconque des revendications 4 à 7, comprenant en outre un deuxième jeu de molécules d'acide nucléique comprenant des molécules d'acide nucléique qui peuvent s'hybrider à des molécules d'acide nucléique qui sont exprimées dans des échantillons de tissu mammaire.

**9.** Utilisation de la matrice selon la revendication 8, dans laquelle le deuxième jeu de molécules d'acide nucléique comprend au moins cinq séquences nucléotidiques qui peuvent s'hybrider à des molécules d'acide nucléique qui sont exprimées dans des échantillons mammaires et qui sont sélectionnées dans le tableau 2.

**10.** Utilisation de la matrice selon la revendication 8, dans laquelle le deuxième jeu de molécules d'acide nucléique comprend au moins cinq séquences nucléotidiques qui peuvent s'hybrider à des molécules d'acide nucléique sélectionnées dans le tableau 3.

**11.** Utilisation d'une matrice comprenant entre 10 et 12 000 molécules d'acide nucléique comprenant un premier et un deuxième jeu de molécules d'acide nucléique, dans laquelle ledit premier jeu de molécules d'acide nucléique comprend au moins 10 molécules, chaque molécule d'acide nucléique dudit premier jeu comprenant une séquence nucléotidique qui peut s'hybrider à un gène différent sélectionné parmi les gènes figurant dans le tableau 1 ;
ladite matrice comprenant en outre un deuxième jeu de molécules d'acide nucléique comprenant des molécules d'acide nucléique qui peuvent s'hybrider aux molécules d'acide nucléique qui sont exprimées dans des échantillons de tissu mammaire, dans une méthode de détection et/ou d'évaluation du stade du cancer du sein, la méthode comprenant
la mise en contact de ladite matrice avec un échantillon comprenant des produits d'expression de cellules de cancer du sein d'un patient ;
la détection de l'hybridation desdits produits d'expression à des molécules d'acide nucléique de ladite matrice pour fournir un profil d'expression ;
la normalisation des données d'hybridation obtenues avec au moins une molécule d'acide nucléique du deuxième jeu de molécules d'acide nucléique avec des données d'hybridation obtenues avec le premier jeu de molécules d'acide nucléique ; et
la comparaison de ladite hybridation à l'hybridation d'au moins un échantillon de référence à une matrice similaire.

**12.** Utilisation selon la revendication 11, dans laquelle le deuxième jeu de molécules d'acide nucléique comprend au moins cinq séquences nucléotidiques qui peuvent s'hybrider à des molécules d'acide nucléique qui sont exprimées dans des échantillons mammaires et qui sont sélectionnées dans le tableau 2.

**13.** Utilisation selon la revendication 11 ou 12, dans laquelle ledit échantillon de référence est un échantillon d'un patient présentant un cancer du sein connu, ou un résultat connu de cancer du sein.

Fig. 1

Fig. 2

Fig. 3

Fig. 4

EP 2 084 296 B1

Fig. 5A

Fig. 5B

Fig. 6

Fig. 7

Fig. 8

Fig. 9

EP 2 084 296 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005158772 A
- US 2006134668 A

- WO 02103320 A
- WO 2002103320 A

**Non-patent literature cited in the description**

- **FALK et al.** *J Mol Med,* vol. 83, 1014-1024
- **HUANG et al.** *Statistical Applications Gen Mol Biol,* 2006, 5
- **LEE et al.** *Genome Res,* 2002, vol. 12, 292-297
- **POHJANVIRTA et al.** *Chemico-biological interactions,* 2006, vol. 160, 134-149
- *Array Design for the GeneChip® - HUMAN GENOME U133 SET,* 1-8, www.affymetrix.com
- **GOLUB et al.** *Science,* 1999, vol. 286, 531
- **ALIZADEH et al.** *Nature,* 2000, vol. 403, 503
- **PEROU et al.** *Nature,* 2000, vol. 406, 747
- **SORLIE et al.** *Proc Natl Acad Sci U S A,* 2001, vol. 98, 10869
- **POMEROY et al.** *Nature,* 2002, vol. 415, 436
- **VAN 'T VEER et al.** *Nature,* 2002, vol. 415, 530
- **BITTNER et al.** *Nature,* 2000, vol. 406, 536
- **HEDENFALK et al.** *N Engl J Med,* 2001, vol. 344, 539
- **RAMASWAMY et al.** *Proc Natl Acad Sci U S A,* 2001, vol. 98, 15149
- **GLAS et al.** *Blood,* 2005, vol. 105, 301

- **KHAN et al.** *Nat Med,* 2001, vol. 7, 673
- **HUANG et al.** *Lancet,* 2003, vol. 361, 1590
- **VAN DE VIJVER et al.** *N Engl J Med,* 2002, vol. 347, 1999
- **WANG et al.** *Lancet,* 2005, vol. 365, 671
- **JANSEN et al.** *J. Clin. Oncol.,* 2005, vol. 23, 732
- **CHANG et al.** *The Lancet,* 2003, vol. 362, 362
- **AYERS et al.** *J. Clin. Oncol.,* 2004, vol. 22, 2284
- **HANNEMANN et al.** *J. Clin. Oncol.,* 2005, vol. 23, 3331
- **YANG et al.** *Nucl. Acids Res.,* 2002, vol. 30, 15
- **SCHMITTGEN ; ZAKRAJSEK.** *J. Biochem. Biophys. Methods,* 2000, vol. 46, 69
- **WENG et al.** *Bioinformatics,* 2006, vol. 22, 1111
- **QUACKENBUSH.** *Nat Genet,* 2002, vol. 32, 496
- **CLEVELAND.** *J. Am. Stat. Assoc.,* 1979, vol. 74, 829
- **CLEVELAND ; DEVLIN.** *J Am Stat Assoc,* 1988, vol. 83, 596